# EUROPEAN PATENT SPECIFICATION

(11) **EP 1 368 020 B1**
(45) Date of publication and mention of the grant of the patent: **05.03.2008**
(21) Application number: 02705934.4
(22) Date of filing: 25.01.2002
(51) Int. Cl.: G01N 33/50, G01N 33/68, C07C 69/00, C07C 233/65

(54) **METHOD FOR IDENTIFYING MAXI-K CHANNEL BLOCKERS**
VERFAHREN ZUR IDENTIFIZIERUNG VON MAXI-K-KANAL-BLOCKERN
PROCEDE RELATIF A L'IDENTIFICATION D'INHIBITEURS DE CANAUX MAXI-K

(30) Priority: 30.01.2001 US 264956 P
(43) Date of publication of application: 10.12.2003
(73) Proprietor: Merck & Co., Inc., Rahway, New Jersey 07065-0907 (US)
(72) Inventor: GARCIA, Maria, L., Rahway, NJ 07065-0907 (US); SCHMALHOFER, William, A., Rahway, NJ 07065-0907 (US)
(74) Representative: Horgan, James Michael Frederic
(86) International application number: PCT/US2002/002014
(87) International publication number: WO 2002/060340

(56) References cited:
- WO-A1-00/25770
- WO-A1-96/33719
- US-A- 5 637 470
- AHRING P K ET AL: "Stable expression of the human large-conductance Ca-activated Kchannel alpha- and beta-subunits in HEK293 cells" FEBS LETTERS, ELSEVIER SCIENCE PUBLISHERS, AMSTERDAM, NL, vol. 415, no. 1, 22 September 1997 (1997-09-22), pages 67-70, XP004261138 ISSN: 0014-5793
- GONZALEZ ET AL: 'Cell-based assays and instrumentation for screening ion-channel targets' DDT vol. 4, 1999, pages 431 - 439, XP001026838

## Description

### BACKGROUND OF THE INVENTION

Maxi-K channels are important for controlling a number of physiological processes. They play a role in the regulation of aqueous humor dynamics in the eye.

Topical or intracamaral application of maxi-K channel inhibitors cause the lowering of intraocular pressure (IOP). This effect of maxi-K channel blockers could be a consequence of either blocking channels in the non-pigmented ciliary epithelium to diminish aqueous humor inflow, or blocking channels in the ciliary muscle to facilitate aqueous humor outflow, or from both effects occurring simultaneously.

Potassium channel antagonists are useful for a number of physiological disorders in mammals, including humans. Ion channels, including potassium channels, are found in all mammalian cells and are involved in the modulation of various physiological processes and normal cellular homeostasis. Potassium channels generally control the resting membrane potential, and the efflux of potassium ions causes repolarization of the plasma membrane after cell depolarization. Potassium channel antagonists prevent repolarization and enable the cell to stay in the depolarized, excited state

Diseases having a particular association with such channels include depression, Alzheimer's, dementia, memory loss, ocular hypertension, macular edema, macular degeneration and glaucoma, to name a few. See for example, Lymphocyte Ion Channels as a Target for Immunosuppression" G.J. Kaczorowski and G.C. Koo Perspectives in Drug Discovery and Design 2, (1994) 233-248.

There are a number of different potassium channel subtypes. Physiologically, one of the most important potassium channel subtypes is the Maxi-K channel, which is present in neuronal and endocrine tissue, smooth muscle and epithelial tissue. Intracellular calcium concentration (Ca²⁺ᵢ) and membrane potential gate these channels. For example, Maxi-K channels are opened to enable efflux of potassium ions by an increase in the intracellular Ca²⁺ concentration or by membrane depolarization (change in potential).

Ahring et al, FEBS Letters 415 (1997) 67-70 discloses the stable expression of α-and β-subunits of maxi-K channels (called BK channels therein) in HEK 293 cells.

US-A-5637470 (Merck & Co., Inc.) discloses an assay for identifying maxi-K channel blockers.

WO-A-0025770 (Merck & Co., Inc.) discloses carbocyclic potassium channel inhibitors.

The currently available maxi-K channel blockers, however, do not display the appropriate solubility properties that are expected of a development candidate for topical eye application. In order to discover novel maxi-K channel blockers, a robust and reliable functional assay is required. High throughput screening assays with ion channels have been developed (see Aurora Biosciences J.E. Gonzalez et al., DDT, 4, 431-439, 1999). For example, transient transfection of maxi-K channel alpha and betal subunits into TsA-201 cells leads to the maxi-K channel controlling the membrane potential of those cells, under appropriate experimental conditions. However, transient transfection experiments are limited because, typically, the transfection has to occur for every experiment, the cells are only available for assay twice a week and results can be variable depending on the transfection efficiency. This invention relates to a new functional assay, which utilizes HEK-293 cells, which once transfected remains as such, thereby eliminating the need for further transfection. The claimed method also provides more reliable reading of the maxi-K channel activity.

### SUMMARY OF THE INVENTION

This invention relates to a method for identifying maxi-K channel blockers, which are useful for lowering IOP, using HEK-293 cells, comprising the steps of:
(a) constructing stable HEK-293 cell lines expressing both alpha and beta 1 subunits of the maxi-K channel;
(b) incubating the HEK-293 cells with an inhibitor of endogenous potassium conductance of HEK-293 cells which does not affect maxi-K channel activity.
(c) loading the incubated HEK-293 cells with a test compound and incubating; and
(d) measuring the maxi-K channel blocker activity of the test compound using a voltage/ion probe reader (VIPR) instrument and FRET based membrane potential sensing dyes to monitor membrane potential.

This and other aspects of the invention are realized upon inspection of the specification as a whole.

### BRIEF DESCRIPTION OF THE FIGURES

Figure 1 shows the effect of high-potassium addition to HEK-293 cells.
Figure 2 shows the effect of the potassium channel blockers Compound A and Compound B on maxi-K channel activity using maxi-K channels transiently expressed in TsA-201 cells.
Figure 3 shows the effect of the potassium channel blocker Compound A on maxi-K channel activity using HEK-293 cells stably transfected with both alpha and beta-1 subunits of the maxi-K channel.
Figure 4 shows representative fluorescence traces obtained in the VIPR instrument with HEK-293 cells stably transfected with both alpha and beta 1 subunits of the maxi-K channel.

### DETAILED DESCRIPTION OF THE INVENTION

This invention relates to a method for identifying maxi-K channel blockers, which are useful for lowering intraocular pressure, using HEK-293 cells stably transfected with alpha and beta 1 subunits of the maxi-K channel, comprising the steps of:
(a) constructing stable HEK-293 cell lines expressing both alpha and beta 1 subunits of the maxi-K channel;
(b) incubating the HEK-293 cells with an inhibitor of endogenous potassium conductance of the HEK-293 cells which does not affect the maxi-K channel activity;
(c) loading the incubated HEK-293 cells with a test compound and incubating; and
(d) measuring the maxi-K channel blocker activity of the test compound using a voltage/ion probe reader instrument and fluorescence resonance energy transfer based membrane potential sensing dyes to monitor membrane potential.

An embodiment of this invention is the method recited above wherein the stable cell lines are constructed by transfection.

An embodiment of the invention is the method as recited above wherein the measurement of the membrane resting potential, comprises the steps of:
(a) plating the HEK-293 cells, 2E+06 Cells/mL, on 96-well poly-D-lysine plates at a density of about 100,000 cells/well;
(b) incubating the plates for 16 to 24 hours;
(c) aspirating medium off the cells;
(d) washing the cells one time with Dulbecco's phosphate-buffered saline
(e) adding 100 µl of 6 µM coumarin dye-0,02% pluronic-127 in Dulbecco's phosphate buffered saline per well;
(f) incubating in the dark for about 30 minutes;
(g) washing the cells two times with Dulbecco's phosphate-buffered saline;
(h) adding 100 µl of 4.5 µM oxanol dye in (mM): 140 NaCl, 0.1 KCl, 2 CaCl₂, 1 MgCl₂, 20 Hepes-NaOH, pH at about 7.4, 10 glucose per well;
(i) adding an inhibitor of endogenous potassium conductance of HEK-293 cells;
(j) incubating at room temperature in the dark for about 30 minutes;
(k) loading the plates onto a voltage/ion probe reader and recording fluorescence emission of both dyes for about 10 seconds;
(l) adding 100 µl of high-potassium solution in (mM): 140 KCl, 2 CaCl₂, 1 MgCl₂, 20 Hepes-KOH, pH at about 7.4, 10 glucose; and
(m) recording fluoresence emission of both dyes for about 10 seconds and determining membrane resting potential from these data using a voltage/ion probe reader instrument.

An embodiment of the invention is the method as recited above wherein the measurement of the maxi-K channel blocking activity of a compound, comprises the steps of:
(a) plating the HEK-293 cells, 2E+06 Cells/mL, on 96-well poly-D-lysine plates at a density of about 100,000 cells/well;
(b) incubating the plates for 16 to 24 hours;
(c) aspirating medium off the cells
(d) washing the cells one time with Dulbecco's phosphate-buffered saline;
(e) adding 100 µl of 9 µM coumarin dye-0.02% pluronic-127 in Dulbecco's phosphate-buffered saline per well;
(f) incubating in the dark for 30 minutes;
(g) washing the cells two times with Dulbecco's phosphate-buffered saline ;
(h) adding 100 µl of 4.5 µM oxanol dye in (mM): 140 NaCl, 0.1 KCl, 2 CaCl₂, 1 MgCl₂, 20 Hepes-NaOH, pH at about 7.4, 10 glucose per well;
(i) adding an inhibitor of endogenous potassium conductance of HEK-293 cells;
(j) adding a test compound;
(k) incubating at room temperature for about 30 minutes;
(l) loading the plates on to a voltage/ion probe reader and recording fluorescence emission of both dyes for about 10 seconds;
(m) adding 100 µl of high-potassium solution in (mM): 140 KCl, 2 CaCl₂, 1 MgCl₂, 20 Hepes-KOH, pH at about 7.4, 10 glucose; and
(n) recording fluoresence emission of both dyes for about 10 seconds and determining membrane potential from these data using a voltage/ion probe reader (VIPR) instrument.

The potassium channel blockers employed in the claimed invention and the method of making said compounds are disclosed in US Patent Application number 09/422,143 filed October 21, 1999. The compounds are of structural formulae I and II: 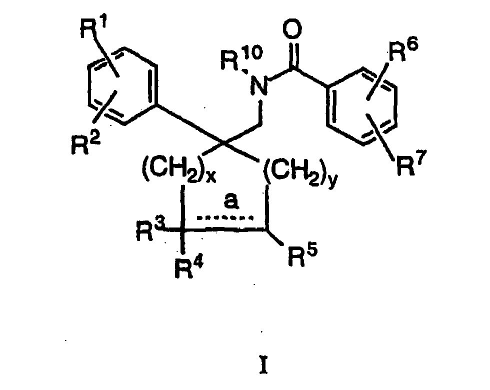 or a pharmaceutically acceptable salt, crystal form, or hydrate, wherein a is a single bond or a double bond when R⁴ is absent, and represented by == in the structure above, with the proviso that a is a single bond when x + y = 0;
- n is:: 0, 1, 2 or 3;
- r is:: 0 or 1;
- s is:: 0 or 1;
x and y are independently 0, 1, or 2;
R¹, R², R⁶ and R⁷ are independently:
   (1) halo, wherein halo is fluoro,chloro,bromo, or iodo,
   (2) hydroxy,
   (3) (C₁-C₆)-alkyl,
   (4) HO(C₁-C₆)-alkyloxy,
   (5) (C₁-C₄)-perfluoroalkyl,
   (6) (C₂-C₆)-alkenyl,
   (7) (C₂-C₆)-alkynyl,
   (8) O[(C-O)Oᵣ]ₛ(C₁-C₆)-alkyl,
   (9) (C₁-C₆)-alkyl-S(O)ₙ-,
   (10) -(O)ᵣ(C₀-C₆)-alkyl-aryl, wherein aryl is phenyl or naphthyl unsubstituted or substituted with up to three substitutents selected from (C₁-C₃)alkyl, trifluoromethyl, and halo;
   (11) -(O)ᵣ-heteroaryl, wherein heteroaryl is pyridinyl or pyrryl,
   (12) cyano,
   (13) nitro,
   (14) CO₂H,
   (15) CO(C₁-C₆)-alkyl,
   (16) CO₂(C₁-C₆)-alkyl,
   (17) CONR⁸R⁹,
   (18) NR⁸R⁹,
   (20) (C₂-C₆)-alkenyloxy,
   (21) (CO)-aryl, wherein aryl is phenyl, naphthyl, benzothienyl, or a benzophenone radical and is unsubstituted or substituted with up to two substituents selected from halo, trifluromethyl, and (C₁-C₃)alkyl,
   (22) hydrogen,
   (23) OCF₃,
   (24) -(CH₂)-O-N=C(CH₃)(aryl), wherein aryl is phenyl or naphthyl and is unsubstituted or substituted with up to three halogen substituents,
   (25) -S(O)ₙ-N R⁸R⁹, or
   (26) R¹ and R² or R⁶ and R⁷ can an be taken together when on adjacent carbons to form a fused benzo, dihydrofuranyl, furanyl, pyrrolidyl, dihydropyrrolidyl or 1,3-dioxolan group;
R³ and R⁴ are independently:
   (1) halo, wherein halo is fluoro, chloro, bromo, or iodo;
   (2) hydroxy, when a is a single bond,
   (3) HO(C₁-C₆)-alkyloxy,
   (4) (C₁-C₄)-perfluoroalkyl,
   (5) O(CO)CCl₃,
   (6) (C₁-C₆)-alkyl-S(O)ₙ-,
   (7) phenyl-(CH₂)ᵣ-S(O)ₙ-,
   (8) cyano,
   (9) nitro,
   (10) CO₂H,
   (11) CO(C₁-C₆)-alkyl,
   (12) CO₂(C₁-C₆)-alkyl,
   (13) CONR⁸R⁹,
   (14) NR⁸R⁹,
   (15) O(CO)NR⁸R⁹,
   (16) azido,
   (17) NR⁸(CO)NR⁸R⁹,
   (18) hydrogen,
   (19) (C₁-C₁₀)-alkyl, wherein alkyl includes cyclic as well as acyclic groups and is unsubstituted or substituted with one or two of the substituents selected from the group consisting of:
      (a) halo, wherein halo is fluoro, chloro, bromo, or iodo,
      (b) hydroxy,
      (c) oxo,
      (d) O[(C=O)Oᵣ]ₛ(C₁-C₆)-alkyl,
      (e) (C₁-C₆)-alkyl-S(O)ₙ-,
      (f) aryl-(C₁-C₆)-alkyloxy,
      (g) cyano,
      (h) nitro,
      (i) vinyl,
      (j) NR⁸R⁹,
      (k) O(CO)NR⁸R⁹,
      (l) CHO,
      (m) CO₂H,
      (n) CO(C₁-C₆)-alkyl,
      (o) CO₂(C₁-C₆)-alkyl,
      (p) CONR⁸R⁹,
      (q) aryl, wherein aryl is defined as phenyl or naphthyl, unsubstituted or substituted with one, two or three of the substituents selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k') cyano,
         (l') nitro,
         (m') CO₂H,
         (n') CO(C₁-C₆)-alkyl,
         (o') CO₂(C₁-C₆)-alkyl,
         (p') CONR⁸R⁹, and
         (q') NR⁸R⁹,
      (r) heteroaryl, wherein heteroaryl is defined as an unsubstituted, monosubstituted, or disubstituted five or six membered aromatic heterocycle containing from 1 to 3 heteroatoms selected from the group consisting of O, N and S and wherein the substituents are members selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k') cyano,
         (l') nitro,
         (m') CO₂H,
         (n') CO(C₁-C₆)-alkyl,
         (o') CO₂(C₁-C₆)-alkyl,
         (p') CONR⁸R⁹,
         (q') NR⁸R⁹, and
         (r') fused benzo or pyridyl group,
      (s) heterocyclyl, wherein heterocyclyl is defined as a 3 to 7 atom cyclic, non-aromatic substituent containing from 1 to 3 heteroatoms selected from the group consisting of O, N, and S, said heterocycle being unsubstituted or substituted with one, two or three substituents selected from the group consisting of:
         (a') halo, as defined above,
         (b') hydroxy,
         (c') (C₁-C₆)-alkyl,
         (d') (C₁-C₄)-perfluoroalkyl,
         (e') (C₂-C₆)-alkenyl,
         (f') (C₂-C₆)-alkynyl,
         (g') (C₁-C₆)-alkyloxy,
         (h) (C₁-C₆)-alkyl-S(O)ₙ-,
         (i') phenyl,
         (j') phenoxy,
         (k) cyano,
         (l') nitro,
         (m') CO₂H,
         (n') CO(C₁-C₆)-alkyl,
         (o') CO₂(C₁-C₆)-alkyl,
         (p') CONR⁸R⁹,
         (q') NR⁸R⁹,
         (r) NR⁸CO(C₁-C₆)-alkyl,
         (s') oxo,
         (t') fused benzo, and
         (u') fused pyridyl group;
      (t) benzyl-S(O)ₙ-,
      (u) O[(C=O)Oᵣ]ₛ(C₂-C₆)-alkenyl,
      (v) O[(C=O)Oᵣ]ₛaryl,
      (w) O[(C=O)Oᵣ]ₛheteroaryl,
      (x) O(CH₂)ₙheteroaryl, or
      (y) O(CH₂)ₙaryl;
   (20) (C₂-C₁₀)-alkenyl, wherein alkenyl is unsubstituted or substituted with one or two of the substituents selected from the group consisting of:
      (a) halo, wherein halo is fluoro, chloro, bromo, or iodo,
      (b) hydroxy,
      (c) oxo,
      (e) (C₁-C₆)-alkyl-S(O)_{n-,}
      (f) phenyl-(C₁-C₆)-alkyloxy,
      (g) cyano,
      (h) nitro,
      (i) NR⁸R⁹,
      (j) CHO,
      (k) CO₂H,
      (l) CO(C₁-C₆)-alkyl,
      (m) CO₂(C₁-C₆)-alkyl,
      (n) CONR⁸R⁹,
      (o) aryl, wherein aryl is as defined above,
      (p) heteroaryl, wherein heteroaryl is as defined above,
      (q) heterocyclyl, wherein heterocyclyl is as defined above,
      (r) O[(C=O)Oᵣ]ₛ(C₁-C₆)-alkyl, alkyl as defined above,
      (s) O[(C=O)Oᵣ]ₛ(C₂-C₆)-alkenyl, as defined above,
      (t) O[(C=O)Oᵣ]ₛaryl, aryl as defined above,
      (u) O[(C=O)Oᵣ]ₛheteroaryl, heteroaryl as defined above,
      (v) O(CH₂)ₙheteroaryl, heteroaryl as defined above, and
      (w) O(CH₂)ₙaryl, aryl as defined above;
   (21) (C₂-C₁₀)-alkynyl, wherein alkynyl is unsubstituted or substituted with one or two of the substituents selected from the group consisting of:
      (a) halo, wherein halo is fluoro, chloro, bromo, or iodo,
      (b) hydroxy,
      (c) oxo,
      (d) (C₁-C₆)-alkyloxy,
      (e) (C₁-C₆)-S(O)ₙ-,
      (f) phenyl-(C₁-C₆)-alkyloxy,
      (g) cyano,
      (h) nitro,
      (i) vinyl,
      (j) NR⁸R⁹,
      (k) NR⁸CO(C₁-C₆)-alkyl,
      (l) CHO,
      (m) CO₂H,
      (n) CO(C₁-C₆)-alkyl,
      (o) CO₂C(C₁-C₆)-alkyl,
      (p) CONR⁸R⁹,
      (q) aryl, wherein aryl is as defined above,
      (r) heteroaryl, wherein heteroaryl is as defined above,
      (s) heterocyclyl, wherein heterocyclyl is as defined above,
      (t) O[(C=O)Oᵣ]ₛ(C₁-C₆)-alkyl, alkyl as defined above,
      (u) O[(C=O)Oᵣ]ₛ(C₂-C₆)-alkenyl, as defined above,
      (v) O[(C=O)Oᵣ]ₛaryl, aryl as defined above,
      (w) O[(C=O)Oᵣ]ₛheteroaryl, heteroaryl as defined above
      (x) O(CH₂)ₙheteroaryl, heteroaryl as defined above, and
      (y) O(CH₂)ₙaryl, aryl as defined above,
   (22) O[(C=O)Oᵣ]ₛ(C₁-C₆)-alkyl, alkyl as defined above,
   (23) O[(C=O)Oᵣ]ₛ(C₂-C₆)-alkenyl, as defined above,
   (24) O[(C=O)Oᵣ]ₛaryl, aryl as defined above,
   (25) O[(C=O)Oᵣ]ₛheteroaryl, heteroaryl as defined above
   (26) O(CH₂)ₙheteroaryl, heteroaryl as defined above,
   (27) aryl, wherein aryl is as defined above,
   (28) O[(C=O)Oᵣ]ₛheteroaryl, heteroaryl as defined above,
   (29) O(CO)NH(CH2-CO-NR⁸R⁹), or
   (30) O(CH₂)ₙaryl, aryl as defined above;
R³ can also be any of the following when a is a single bond and R⁴ is absent:
   (31) oxo,
   (32) =CH-(C₁-C₆)-alkyl, wherein alkyl is as defined above,
   (33) =CH-(C₂-C₆)-alkenyl, wherein alkenyl is as defined above,
   (34) =CH-aryl, wherein aryl is as defined above,
   (35) =CH₂, or
R³ and R⁴ can be taken together to form a spiro-fused heterocyclyl group, wherein heterocyclyl is as defined above, or
R³ and R⁵ can be taken together to form a fused oxirane when a is a single bond, with the proviso that R⁴ is absent when a is a double bond;
R⁵ is:
   (1) hydrogen,
   (2) halogen,
   (3) (C₂-C₆)-alkenyl,
   (4) hydroxy,
   (5) O[(C=O)Oᵣ]ₛ(C₁-C₆)-alkyl,
   (6) O(CO) NR⁸R⁹,
   (7) oxo, when a is a single bond, or
R⁵ and R³ can be taken together to form a fused oxirane when a is a single bond;
R⁸ and R⁹ are independently selected from the group consisting of:
   (1) hydrogen,
   (2) [(C=O)Oᵣ]ₛaryl, wherein aryl is as defined above,
   (3) [(C=O)Oᵣ]ₛ(C₂-C₈)-alkenyl, wherein alkenyl is as defined above,
   (4) ((C=O)Oᵣ]ₛ(C₁-C₈)-alkyl, wherein alkyl is as defined above,
   (5) (C=O)ᵣS(O)ₙ(C₁-C₈)-alkyl, wherein alkyl is as defined above,
   (6) (C=O)ᵣS(O)ₙaryl, wherein aryl is as defined above, and
   (7) heterocyclyl, wherein heterocyclyl is defined above;
R¹⁰ is:
   (1) hydrogen,
   (2) [(C=O)Oᵣ]ₛaryl, wherein aryl is as defined above, or
   (3) [(C=O)Oᵣ]ₛ(C₁-C6)-alkyl, wherein alkyl is as defined above.

A preferred embodiment is the compound of Formula II below wherein x is 2 and y is 1.

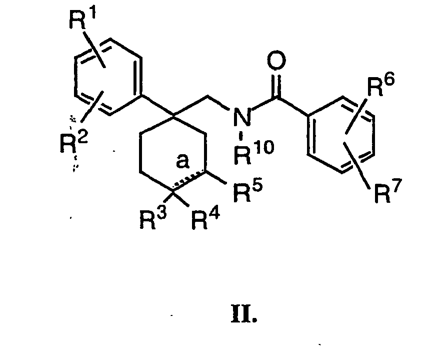

Yet another preferred emobodiment is the compound of Formula II above, wherein a is further defined as a single bond;
R¹, R², R⁶ and R⁷ are independently:
   (1) halo, wherein halo is fluoro, chloro, bromo, or iodo,
   (2) hydroxy,
   (3) (C₁-C₆)-alkyl,
   (4) HO(C₁-C₆)-alkyloxy,
   (5) (C₁-C₆)-alkyloxy, wherein the alkyl is cyclic or straight- chained,
   (6) acetoxy,
   (7) nitro,
   (8) NR⁸R⁹,
   (9) -(O)ᵣ(C₀-C₃)-aryl, wherein aryl is phenyl or naphthyl unsubstituted or substituted with up to three substitutents selected from (C₁-C₃)alkyl, trifluoromethyl, and halo,
   (10) hydrogen,
   (11) (O)ᵣCF₃,
   (12) (C₁-C₆)-alkyl-S(O)ₙ-, wherein n is 0,1, 2 or 3,
   (13) (CO₂)-(C₁-C₆)-alkyl,
   (14) -(O)ᵣ-heteroaryl, wherein heteroaryl is pyridinyl or pyrryl,
   (15) (CO)-aryl, wherein aryl is phenyl, naphthyl, benzothienyl, or benzophenone radical and is unsubstituted or substituted with up to two substituents selected from halo, trifluromethyl, and (C₁-C₃)alkyl,
   (16) -(CH₂)-O-N=C(CH₃)(aryl), wherein aryl is phenyl, unsubstituted or substituted with up to three halogen substituents,
   (17) -S(O)ₙ-NR⁸R⁹, or
   (18) R¹ and R² or R⁶ and R⁷ can an be taken together to form a fused benzo, dihydrofuranyl, furanyl, pyrrolidyl, dihydropyrrolidyl or 1,3-dioxolan group;
R³ and R⁴ are independently:
   (1) halo, wherein halo is fluoro, chloro, bromo, or iodo,
   (2) hydroxy,
   (3) HO(C₁-C₆)-alkyloxy,
   (4) (C=O)O(C₁-C₆)-alkyl,
   (5) O(CO)CCl₃,
   (6) (C₁-C₆-alkyl-S(O)ₙ-, wherein n is 0, 1, 2 or 3,
   (7) CH₂CO₂-(C₁-C₆)-alkyl,
   (8) cyano,
   (9) benzyloxy,
   (10) CH₂OAc,
   (11) OAc,
   (12) (C₂-C₆)-alkenyl,
   (13) (C₁-C₆)-alkyl, wherein alkyl can be unsubstituted or substituted with bromide
   (14) NR⁸R⁹,
   (15) O(CO)NR⁸R⁹,
   (16) azido,
   (17) NR⁸(CO)NR⁸R⁹,
   (18) hydrogen,
   (19) CH₂OH,
   (20) CH₂O(C=O)phenyl, wherein phenyl is unsubstituted or monosubstituted with methoxy,
   (21) O(C₂-C₆)-alkenyl,
   (22) O(C=O)-phenyl, wherein phenyl is unsubstituted or monosubstituted with bromide,
   (23) O(C=O)O-phenyl, wherein phenyl is unsubstituted or monosubstituted with nitro,
   (24) CH₂(CO)NR⁸R⁹,
   (25) O(C=O)O-(C₂-C₆)-alkenyl,
   (26) O(C=O)-(C₁-C₃)-alkyl, wherein the alkyl can be unsubstituted or substituted with bromide or -CO₂CH₃,
   (27) O(C₁-C₆)-alkyl, wherein alkyl can be unsubstituted or substituted with phenyl,
   (28) O(C=O)O-(C₁-C₆)-alkyl,
   (29) CH₂O(CO)NR⁸R⁹, or
   (30) CH₂ (C=O)O-(C₁-C₆)-alkyl,
R³ can also be any of the following when R⁴ is absent:
   (31) oxo,
   (32) =CH₂,
   (33) =CH-CO₂-(C₁-C₆)-alkyl,
   (34) =CH-(CO)-NR⁸R⁹, or
   (35) =CH-CO₂H, or
R³ and R⁴ can be taken together to form a spiro-fused heterocyclyl group, wherein heterocyclyl is defined as:
   (36) oxirane,
   (37) 1,3-dioxolan,
   (38) 2,2-dimethyl-1,3-dioxolan, or
   (39) glycol sulfite, or
R³ and R⁵ can be taken together to form a fused oxirane;
R⁵ is:
   (1) hydrogen,
   (2) halogen,
   (3) (C₂-C₆)-alkenyl,
   (4) hydroxy,
   (5) O(C=O)(C₁-C₃)-alkyl,
   (6) O(CO) NR⁸R⁹,
   (7) oxo, when a is a single bond, or
R⁵ and R³ can be taken together to form a fused oxirane when a is a single bond;
R⁸ and R⁹ are independently selected from the group consisting of:
   (1) hydrogen,
   (2) (C=O)O(C₁-C₆)-alkyl, wherein alkyl is optionally substituted with phenyl or methoxy,
   (3) (C=O)phenyl, wherein phenyl is optionally substituted with bromide or methoxy,
   (4) (C₁-C₆)-alkyl, wherein alkyl is optionally substituted with phenyl, methoxy, hydroxy, OCH₂OCH₃, benzylSO₃, phenylSO₃, or carboxymethyl,
   (5) (C₂-C₆)-alkenyl,
   (6) (C=O)O-phenyl, wherein phenyl is optionally substituted with nitro,
   (7) (C=O)O(C₂-C₆)-alkenyl,
   (8) (C=O)(C₁-C₃)-alkyl, wherein alkyl is optionally substituted with phenyl,
   (9) (C=O)(C₂-C₄)-alkenyl,
   (10) phenyl,
   (11) SO₂-phenyl,
   (12) SO₂-benzyl,
   (13) CH₂(CO)CH₃,
   (14) CH₂(CO)NH-benzyl,
   (15) CH₂(CO)NH-allyl,
   (16) CH₂(CO)N(CH₃)₂,
   (17) CH₂(CO)NH(CH₃),
   (18) 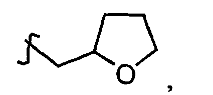
   (19) 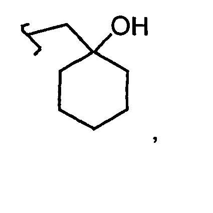
   (20) 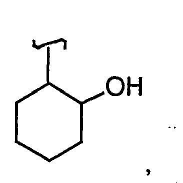
   (21) 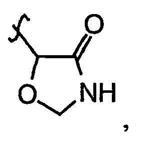
   (22) 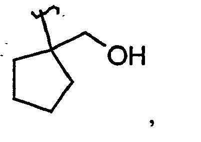
   (23) 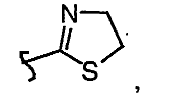
   (24) CH₂CH₂NHCO₂(C₁-C₃)alkyl,
   (25) CH₂CH₂O(CO)NHCH₃,
   (26) CH₂CH₂O(CO)NH-allyl,
   (27) CH₂CH₂NH(SO₂)CH₃,
   (28) CH₂CH₂NH₂,
   (29) CH₂CH₂NH(CO)CH₂CH₃, and
   (30) benzyl;
R¹⁰ is:
   (1) hydrogen,
   (2) (C=O)phenyl, wherein phenyl is unsubstituted or substituted with F, Cl, Br, or I, or
   (3) (C₁-C₃)-alkyl.

A most preferred embodiment of formula I or II is a compound selected from the group consisting of: trans 1-(N-ethylcarbamoyloxy)-4-phenyl-4-(3-(2-methoxyphenyl)-3-oxo-2-azaprop1-1yl)cyclohexane, trans 1-(N-allylcarbamoyloxy)-4-phenyl-4-(3-(2-hydroxy-5-fluorophenyl)-3-oxo-2-azaprop-1-yl)cyclohexane, trans 1-(N-n-propylcarbamoyloxy)-4-phenyl-4-(3-(2-methoxyphenyl)-3-oxo-2-azaprop-1-yl)cyclohexane, trans 1-(N-methylcarbamoyloxy)-4-phenyl-4-(3-(2-methoxyphenyl)-3-oxo-2-azaprop-1-yl)cyclohexane, and trans 1-(N-allylcarbamoyloxy)-4-phenyl-4-(3-(2-methoxyphenyl)-3-oxo-2-azapropyl)cyclohexane.

Another most preferred embodiment of formula I or II is an enantiomerically pure compound or an enantiomerically enriched compound with the following structural formula:

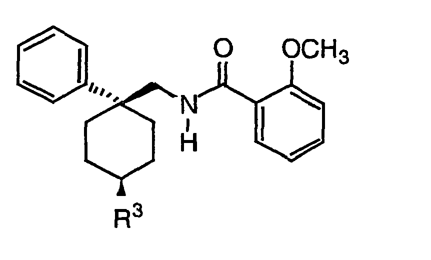

Another most preferred embodiment of formula I or II is an enantiomerically pure compound or an enantiomerically enriched compound with the following structural formula:

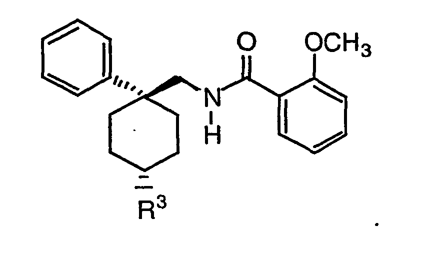

Other potassium channel blockers employed to selectively eliminate endogenous conductances of the HEK-293 cells and the method of making said compounds are disclosed in US Patent Application 09/422,499, filed October 21, 1999, incorporated herein by reference. The compounds are of structural formula III: 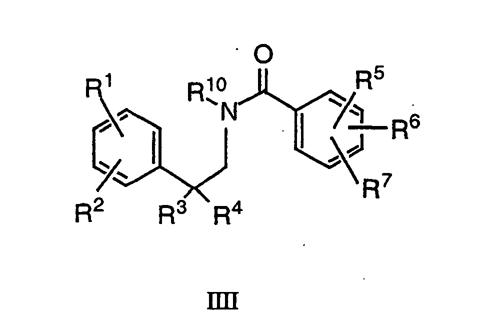 or a pharmaceutically acceptable salt, crystal form, or hydrate, wherein:
- n is:: 0, 1, 2 or 3;
- r is:: 0 or 1;
- s is:: 0 or 1;

R¹, R², R⁵, R⁶ and R⁷ are independently:
   (1) halo, wherein halo is fluoro, chloro, bromo, or iodo,
   (2) hydroxy,
   (3) (C₁-C₆)-alkyl,
   (4) HO(C₁-C₆)-alkyloxy,
   (5) (C₁-C₄)-perfluoroalkyl,
   (6) (C₂-C₆)-alkenyl,
   (7) (C₂-C₆)-alkynyl,
   (8) O[(C=O)Oᵣ]ₛ(C₁-C₆)-alkyl, wherein the alkyl may be cyclic or straight-chained,
   (9) (C₁-C₆)-alkyl-S(O)ₙ-,
   (10) phenyl,
   (11) phenoxy,
   (12) cyano,
   (13) nitro,
   (14) CO₂H,
   (15) CO(C₁-C₆)-alkyl,
   (16) CO₂(C₁-C₆)-alkyl,
   (17) CONR⁸R⁹,
   (18) NR⁸R⁹,
   (20) (C₂-C₆)-alkenyloxy,
   (21) benzyloxy,
   (22) hydrogen,
   (23) OCF₃, or
   (24) R¹ and R² or R⁶ and R⁷ can an be taken together when on adjacent carbons to form a fused benzo, dihydrofuranyl, furanyl, pyrrolidyl, dihydropyrrolidyl or 1,3-dioxolan group;
R³ and R⁴ are independently:
   (1) halo, wherein halo is fluoro, chloro, bromo, or iodo,
   (2) hydroxy,
   (3) HO(C₁-C₆)-alkyloxy,
   (4) (C₁-C₄)-perfluoroalkyl,
   (5) O(CO)CCl₃,
   (6) (C₁-C₆)-alkyl-S(O)ₙ-,
   (7) phenyl-(CH₂)ᵣ-S(O)ₙ-,
   (8) cyano,
   (9) nitro,
   (10) CO₂H,
   (11) CO(C₁-C₆)-alkyl,
   (12) CO₂(C₁-C₆)-alkyl,
   (13) CONR⁸R⁹,
   (14) NR⁸R⁹,
   (15) O(CO)NR⁸R⁹,
   (16) azido,
   (17) NR⁸(CO)NR⁸R⁹,
   (18) hydrogen,
   (19) (C₁-C₁₀)-alkyl, wherein alkyl includes cyclic as well as acyclic groups and is unsubstituted or substituted with one or two of the substituents selected from the group consisting of:
      (a) halo, wherein halo is fluoro, chloro, bromo, or iodo,
      (b) hydroxy,
      (c) oxo,
      (d) O[(C=O)Oᵣ]ₛ(C₁-C₆)-alkyl,
      (e) (C₁-C₆)-alkyl-S(O)ₙ-,
      (f) aryl-(C₁-C₆)-alkyloxy,
      (g) cyano,
      (h) nitro,
      (i) vinyl,
      (j) NR⁸R⁹,
      (k) O(CO)NR⁸R⁹,
      (l) CHO,
      (m) CO₂H,
      (n) CO(C₁-C₆)-alkyl,
      (o) CO₂(C₁-C₆)-alkyl, wherein alkyl may be substituted with phenyl,
      (p) CONR⁸R⁹,
      (q) aryl, wherein aryl is defined as phenyl or naphthyl, unsubstituted or substituted with one, two or three of the substituents selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k') cyano,
         (l') nitro,
         (m') CO₂H,
         (n') CO(C₁-C₆)-alkyl,
         (o') CO₂(C₁-C₆)-alkyl,
         (p') CONR⁸R⁹,
         (q') NR⁸R⁹, and
      (r) heteroaryl, wherein heteroaryl is defined as an unsubstituted, monosubstituted, or disubstituted five or six membered aromatic heterocycle containing from 1 to 3 heteroatoms selected from the group consisting of O, N and S and wherein the substituents are members selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k') cyano,
         (l') nitro,
         (m') CO₂H,
         (n') CO(C₁-C₆)-alkyl,
         (o') CO₂(C₁-C₆)-alkyl,
         (p') CONR⁸R⁹,
         (q') NR⁸R⁹, and
         (r') fused benzo or pyridyl group,
      (s) heterocyclyl, wherein heterocyclyl is defined as a 3 to 7 atom cyclic, non-aromatic substituent containing from 1 to 3 heteroatoms selected from the group consisting of O, N, and S, said heterocycle being unsubstituted or substituted with one, two or three substituents selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k') cyano,
         (l') nitro,
         (m') CO₂H,
         (n') CO(C₁-C₆)-alkyl,
         (o') CO₂(C₁-C₆)-alkyl,
         (p') CONR⁸R⁹,
         (q') NR⁸R⁹,
         (r') NR⁸CO(C₁-C₆)-alkyl,
         (s') oxo,
         (t') fused benzo, and
         (u') fused pyridyl group;
      (t) benzyl-S(O)ₙ-,
      (u) O[(C=O)Oᵣ]ₛ(C₂-C₆)-alkenyl,
      (v) O[(C=O)Oᵣ]ₛaryl,
      (w) O[(C=O)Oᵣ]ₛheteroaryl,
      (x) O(CH₂)ₙheteroaryl, or
      (y) O(CH₂)ₙaryl;
   (20) (C₂-C₁₀)-alkenyl, wherein alkenyl is unsubstituted or substituted with one or two of the substituents selected from the group consisting of:
      (a) halo, wherein halo is fluoro, chloro, bromo, or iodo,
      (b) hydroxy,
      (c) oxo,
      (e) (C₁-C₆)-alkyl-S(O)ₙ-,
      (f) phenyl-(C₁-C₆)-alkyloxy,
      (g) cyano,
      (h) nitro,
      (i) NR⁸R⁹,
      (j) CHO,
      (k) CO₂H,
      (l) CO(C₁-C₆)-alkyl,
      (m) CO₂(C₁-C₆)-alkyl,
      (n) CONR⁸R⁹,
      (o) aryl, wherein aryl is as defined above,
      (p) heteroaryl, wherein heteroaryl is as defined above,
      (q) heterocyclyl, wherein heterocyclyl is as defined above,
      (r) O[(C=O)Oᵣ]ₛ(C₁-C₆)-alkyl, alkyl as defined above,
      (s) O[(C=O)Oᵣ]ₛ(C₂-C₆)-alkenyl, as defined above,
      (t) O[(C=O)Oᵣ]ₛaryl, aryl as defined above,
      (u) O[(C=O)Oᵣ]ₛheteroaryl, heteroaryl as defined above,
      (v) O(CH₂)ₙheteroaryl, heteroaryl as defined above, and
      (w) O(CH₂)ₙaryl, aryl as defined above;
   (21) (C₂-C₁₀)-alkynyl, wherein alkynyl is unsubstituted or substituted with one or two of the substituents selected from the group consisting of:
      (a) halo, wherein halo is fluoro, chloro, bromo, or iodo,
      (b) hydroxy,
      (c) oxo,
      (d) (C₁-C₆)-alkyloxy,
      (e) (C₁-C₆)-S(O)ₙ-,
      (f) phenyl-(C₁-C₆)-alkyloxy,
      (g) cyano,
      (h) nitro,
      (i) vinyl,
      (j) NR⁸R⁹,
      (k) NR⁸CO(C₁-C₆)-alkyl,
      (l) CHO,
      (m) CO₂H,
      (n) CO(C₁-C₆)-alkyl,
      (o) CO₂C(C₁-C₆)-alkyl,
      (p) CONR⁸R⁹,
      (q) aryl, wherein aryl is as defined above,
      (r) heteroaryl, wherein heteroaryl is as defined above,
      (s) heterocyclyl, wherein heterocyclyl is as defined above,
      (t) O[(C=O)Oᵣ]ₛ(C₁-C₆)-alkyl, alkyl as defined above,
      (u) O[(C=O)Oᵣ]ₛ(C₂-C₆)-alkenyl, as defined above,
      (v) O[(C=O)Oᵣ]ₛaryl, aryl as defined above,
      (w) O[(C=O)Oᵣ]ₛheteroaryl, heteroaryl as defined above
      (x) O(CH₂)ₙheteroaryl, heteroaryl as defined above, and
      (y) O(CH₂)ₙaryl, aryl as defined above,
   (22) O[(C=O)Oᵣ]ₛ(C₁-C₆)-alkyl, alkyl as defined above,
   (23) O[(C=O)Oᵣ]ₛ(C₂-C₆)-alkenyl, as defined above,
   (24) O[(C=O)Oᵣ]ₛaryl, aryl as defined above,
   (25) O[(C=O)Oᵣ]ₛheteroaryl, heteroaryl as defined above
   (26) O(CH₂)ₙheteroaryl, heteroaryl as defined above,
   (27) aryl, wherein aryl is as defined above or
   (28) O(CH₂)ₙaryl, aryl as defined above;
R³ can also be any of the following when R⁴ is absent:
   (29) oxo,
   (30) =CH-(C₁-C₆)-alkyl, wherein alkyl is as defined above,
   (31) =CH-(C₂-C₆)-alkenyl, wherein alkenyl is as defined above,
   (32) =CH-aryl, wherein aryl is as defined above, or
   (33) =CH₂;
R⁸ and R⁹ are independently selected from the group consisting of:
   (1) hydrogen,
   (2) [(C=O)Oᵣ]ₛaryl, wherein aryl is as defined above,
   (3) [(C=O)Oᵣ]ₛ(C₂-C₈)-alkenyl, wherein alkenyl is as defined above,
   (4) [(C=O)Oᵣ]ₛ(C₁-C₈)-alkyl, wherein alkyl is as defined above,
   (5) (C=O)ᵣS(O)ₙ(C₁-C₈)-alkyl, wherein alkyl is as defined above,
   (6) (C=O)ᵣS(O)ₙ-aryl, wherein aryl is as defined above, and
   (7) heterocyclyl, wherein heterocyclyl is defined above;
R¹⁰ is:
   (1) hydrogen,
   (2) [(C=O)Oᵣ]ₛaryl, wherein aryl is as defined above, or
   (3) [(C=O)Oᵣ]ₛ(C₁-C₆)-alkyl, wherein alkyl is as defined above.

Another embodiment of the invention is the compound of Formula III,
wherein R³ and R⁴ are independently:
   (1) halo, wherein halo is fluoro, chloro, bromo, or iodo,
   (2) hydroxy,
   (3) HO(C₁-C₆)-alkyloxy,
   (4) (C₁-C₄)-perfluoroalkyl,
   (5) O(CO)CCl₃,
   (6) (C₁-C₆)-alkyl-S(O)ₙ-,
   (7) phenyl-(CH₂)ᵣ-S(O)ₙ-,
   (8) cyano,
   (9) nitro,
   (10) CO₂H,
   (11) CO(C₁-C₆)-alkyl,
   (12) CO₂(C₁-C₆)-alkyl,
   (13) CONR⁸R⁹,
   (14) NR⁸R⁹,
   (15) O(CO)NR⁸R⁹,
   (16) azido,
   (17) NR⁸(CO)NR⁸R⁹,
   (18)hydrogen,
   (19) (C₁-C6)-alkyl, wherein alkyl includes cyclic as well as acyclic groups and is unsubstituted or substituted with one or two of the substituents selected from the group consisting of:
      (a) halo, wherein halo is fluoro, chloro, bromo, or iodo,
      (b) hydroxy,
      (c) oxo,
      (d) O[(C=O)Oᵣ]ₛ(C₁-C₆)-alkyl,
      (e) (C₁-C₆)-alkyl-S(O)ₙ-,
      (f) aryl-(C₁-C₆)-alkyloxy,
      (g) cyano,
      (h) nitro,
      (i) vinyl,
      (j) NR⁸R⁹,
      (k) O(CO)NR⁸R⁹,
      (l) CHO,
      (m) CO₂H,
      (n) CO(C₁-C₆)-alkyl,
      (o) CO₂(C₁-C₆)-alkyl, wherein alkyl may be substituted with phenyl,
      (p) CONR⁸R⁹,
      (q) aryl, wherein aryl is defined as phenyl or naphthyl, unsubstituted or substituted with one, two or three of the substituents selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k') cyano,
         (l') nitro,
         (m) CO₂H,
         (n') CO(C₁-C₆)-alkyl,
         (o') CO₂(C₁-C₆)-alkyl,
         (p') CONR⁸R⁹,
         (q) NR⁸R⁹, and
      (r) heteroaryl, wherein heteroaryl is defined as an unsubstituted, monosubstituted, or disubstituted five or six membered aromatic heterocycle containing from 1 to 3 heteroatoms selected from the group consisting of O, N and S and wherein the substituents are members selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k') cyano,
         (l') nitro,
         (m') CO₂H,
         (n') CO(C₁-C₆)-alkyl,
         (o') CO₂(C₁-C₆)-alkyl,
         (p') CONR⁸R⁹,
         (q') NR⁸R⁹, and
         (r') fused benzo or pyridyl group,
      (s) heterocyclyl, wherein heterocyclyl is defined as a 3 to 7 atom cyclic, non-aromatic substituent containing from 1 to 3 heteroatoms selected from the group consisting of O, N, and S, said heterocycle being unsubstituted or substituted with one, two or three substituents selected from the group consisting of:
         (a') halo, as defined above,
         (b') hydroxy,
         (c') (C₁-C₆)-alkyl,
         (d') (C1-C4)-perfluoroalkyl,
         (e') (C₂-C₆)-alkenyl,
         (f') (C₂-C₆)-alkynyl,
         (g') (C₁-C₆)-alkyloxy,
         (h') (C₁-C₆)-alkyl-S(O)ₙ-,
         (i') phenyl,
         (j') phenoxy,
         (k) cyano,
         (l') nitro,
         (m') CO₂H,
         (n') CO(C₁-C₆)-alkyl,
         (o') CO₂(C₁-C₆)-alkyl,
         (p') CONR⁸R⁹,
         (q') NR⁸R⁹,
         (r') NR⁸CO(C₁-C₆)-alkyl,
         (s') oxo,
         (t') fused benzo, and
         (u') fused pyridyl group;
      (t) benzyl-S(O)ₙ-,
      (u) O[(C=O)Oᵣ]ₛ(C₂-C₆)-alkenyl,
      (v) O[(C=O)Oᵣ]ₛaryl,
      (w) O[(C=O)Oᵣ]ₛheteroaryl,
      (x) O(CH₂)ₙheteroaryl, or
      (y) O(CH₂)ₙaryl;
   (20) (C₂-C₆)-alkenyl, wherein alkenyl is unsubstituted or substituted with one or two of the substituents selected from the group consisting of:
      (a) halo, wherein halo is fluoro, chloro, bromo, or iodo,
      (b) hydroxy,
      (c) oxo,
      (e) (C₁-C₆)-alkyl-S(O)ₙ-,
      (f) phenyl-(C₁-C₆)-alkyloxy,
      (g) cyano,
      (h) nitro,
      (i) NR⁸R⁹,
      (j) CHO,
      (j) CHO,
      (k) CO₂H,
      (l) CO(C₁-C₆)-alkyl,
      (m) CO₂(C₁-C₆)-alkyl,
      (n) CONR⁸R⁹,
      (o) aryl, wherein aryl is as defined above,
      (p) heteroaryl, wherein heteroaryl is as defined above,
      (q) heterocyclyl, wherein heterocyclyl is as defined above,
      (r) O[(C=O)Oᵣ]ₛ(C₁-C₆)-alkyl, alkyl as defined above,
      (s) O[(C=O)Oᵣ]ₛ(C₂-C₆)-alkenyl, as defined above,
      (t) O[(C=O)Oᵣ]ₛaryl, aryl as defined above,
      (u) O[(C=O)Oᵣ]ₛheteroaryl, heteroaryl as defined above,
      (v) O(CH₂)ₙheteroaryl, heteroaryl as defined above, and
      (w) O(CH₂)ₙaryl, aryl as defined above;
   (21) (C₂-C6)-alkynyl, wherein alkynyl is unsubstituted or substituted with one or two of the substituents selected from the group consisting of:
      (a) halo, wherein halo is fluoro, chloro, bromo, or iodo,
      (b) hydroxy,
      (c) oxo,
      (d) (C₁-C₆)-alkyloxy,
      (e) (C₁-C₆)-S(O)ₙ-,
      (f) phenyl-(C₁-C₆)-alkyloxy,
      (g) cyano,
      (h) nitro,
      (i) vinyl,
      (j) NR⁸R⁹,
      (k) NR⁸CO(C₁-C₆)-alkyl,
      (l) CHO,
      (m) CO₂H,
      (n) CO(C₁-C₆)-alkyl,
      (o) CO₂C(C₁-C₆)-alkyl,
      (p) CONR⁸R⁹,
      (q) aryl, wherein aryl is as defined above,
      (r) heteroaryl, wherein heteroaryl is as defined above,
      (s) heterocyclyl, wherein heterocyclyl is as defined above,
      (t) O[(C=O)Oᵣ]ₛ(C₁-C₆)-alkyl, alkyl as defined above,
      (u) O[(C=O)Oᵣ]ₛ(C₂-C₆)-alkenyl, as defined above,
      (v) O[(C=O)Oᵣ]ₛaryl, aryl as defined above,
      (w) O[(C=O)Oᵣ]ₛheteroaryl, heteroaryl as defined above
      (x) O(CH₂)ₙheteroaryl, heteroaryl as defined above, and
      (y) O(CH₂)ₙaryl, aryl as defined above,
   (22) O[(C=O)Oᵣ]ₛ(C₁-C₆)-alkyl, alkyl as defined above,
   (23) O[(C=O)Oᵣ]ₛ(C₂-C₆)-alkenyl, as defined above,
   (24) O[(C=O)Oᵣ]ₛaryl, aryl as defined above,
   (25) O[(C=O)Oᵣ]ₛheteroaryl, heteroaryl as defined above
   (26) O(CH₂)ₙheteroaryl, heteroaryl as defined above,
   (27) aryl, wherein aryl is as defined above or
   (28) O(CH₂)ₙaryl, aryl as defined above;
R³ can also be any of the following when R⁴ is absent:
   (29) oxo,
   (30) =CH-(C₁-C₆)-alkyl, wherein alkyl is as defined above,
   (31) =CH-(C₂-C₆)-alkenyl, wherein alkenyl is as defined above,
   (32) =CH-aryl, wherein aryl is as defined above, or
   (33) =CH₂.

A preferred embodiment is the compound of Formula III, wherein R¹,
R², R⁵, R⁶ and R⁷ are independently:
   (1) halo, wherein halo is fluoro, chloro, bromo, or iodo,
   (2) hydroxy,
   (3) (C₁-C₆)-alkyl,
   (4) HO(C₁-C₆)-alkyloxy,
   (5) (C₁-C₄)-perfluoroalkyl,
   (6) (C₂-C₆)-alkenyl,
   (7) O[(C=O)Oᵣ]ₛ(C₁-C₆)-alkyl, wherein the alkyl may be cyclic or straight-chained,
   (8) phenyl,
   (9) CO(C₁-C₆)-alkyl,
   (10) CO₂(C₁-C₆)-alkyl,
   (11) CONR⁸R⁹,
   (12) NR⁸R⁹,
   (13) (C₂-C₆)-alkenyloxy,
   (14) benzyloxy,
   (15) hydrogen,
   (16) OCF₃,
   (17) R¹ and R² or R⁶ and R⁷ can an be taken together when on adjacent carbons to form a fused benzo, dihydrofuranyl, furanyl, pyrrolidyl, dihydropyrrolidyl or 1,3-dioxolan group;
R³ and R⁴ are independently:
   (1) halo, wherein halo is fluoro, chloro, bromo, or iodo,
   (2) hydroxy,
   (3) HO(C₁-C₆)-alkyloxy,
   (4) (C₁-C₄)-perfluoroalkyl,
   (5) O(CO)CCl₃,
   (6) (C₁-C₆)-alkyl-S(O)ₙ-,
   (7) phenyl-(CH₂)ᵣ-S(O)ₙ-,
   (8) cyano,
   (9) nitro,
   (10) CO₂H,
   (11) CO(C₁-C₆)-alkyl,
   (12) CO₂(C₁-C₆)-alkyl,
   (13) CONR⁸R⁹,
   (14) NR⁸R⁹,
   (15) O(CO)NR⁸R⁹,
   (16) azido,
   (17) NR⁸(CO)NR⁸R⁹,
   (18) hydrogen,
   (19) (C₁-C₆)-alkyl, wherein alkyl includes cyclic as well as acyclic groups and is unsubstituted or substituted with one or two of the substituents selected from the group consisting of:
      (a) halo, wherein halo is fluoro, chloro, bromo, or iodo,
      (b) hydroxy,
      (c) oxo,
      (d) O[(C=O)Oᵣ]ₛ(C₁-C₆)-alkyl,
      (e) aryl-(C₁-C₆)-alkyloxy,
      (f) NR⁸R⁹,
      (g) O(CO)NR⁸R⁹,
      (h) CHO,
      (i) CO₂H,
      (j) CO(C₁-C₆)-alkyl,
      (k) CO₂(C₁-C₆)-alkyl, wherein alkyl may be substituted with phenyl,
      (l) CO₂(C₁-C₆)-alkenyl,
      (m) CONR⁸R⁹,
      (n) aryl, wherein aryl is defined as phenyl or naphthyl, unsubstituted or substituted with a substituent selected from the group consisting of:
         (a') halo, as defined above,
         (b') hydroxy,
         (c') (C₁-C₆)-alkyl,
         (d') (C₁-C₆)-alkyloxy,
         (e') (C₁-C₆)-alkyl-S(O)ₙ-,
         (f') phenyl,
         (g') phenoxy,
         (h') cyano,
         (i') CO₂H,
         (j') CO(C₁-C₆)-alkyl,
         (k') CO₂(C₁-C₆)-alkyl,
         (l') CONR⁸R⁹,
         (m') NR⁸R⁹, and
      (o) benzyl-S(O)ₙ-,
      (p) O[(C=O)Oᵣ]ₛ(C₂-C₆)-alkenyl,
      (q) O[(C=O)Oᵣ]ₛaryl,
      (r) O[(C=O)Oᵣ]ₛheteroaryl,
      (s) O(CH₂)ₙheteroaryl, or
      (t) O(CH₂)ₙaryl;
   (20) (C₂-C₆)-alkenyl, wherein alkenyl is unsubstituted or substituted with a substituent selected from the group consisting of:
      (a) halo, wherein halo is fluoro, chloro, bromo, or iodo,
      (b) hydroxy,
      (c) oxo,
      (d) phenyl-(C₁-C₆)-alkyloxy,
      (e) NR⁸R⁹,
      (f) CHO,
      (g) CO₂H,
      (h) CO(C₁-C₆)-alkyl,
      (i) CO₂(C₁-C₆)-alkyl,
      (j) CONR⁸R⁹,
      (k) aryl, wherein aryl is as defined above,
      (l) O[(C=O)Oᵣ]ₛ(C₁-C₆)-alkyl, alkyl as defined above,
      (m) O[(C=O)Oᵣ]ₛaryl, aryl as defined above,
      (n) O[(C=O)Oᵣ]ₛheteroaryl, heteroaryl as defined above,
      (o) O(CH₂)ₙheteroaryl, heteroaryl as defined above, and
      (p) O(CH₂)ₙaryl, aryl as defined above;
   (21) O[(C=O)Oᵣ]ₛ(C₁-C₆)-alkyl, alkyl as defined above,
   (22) O[(C=O)Oᵣ]ₛ(C₂-C₆)-alkenyl, as defined above,
   (23) O[(C=O)Oᵣ]ₛaryl, aryl as defined above,
   (24) O[(C=O)Oᵣ]ₛheteroaryl, heteroaryl as defined above
   (25) O(CH₂)ₙheteroaryl, heteroaryl as defined above,
   (26) aryl, wherein aryl is as defined above or
   (27) O(CH₂)ₙaryl, aryl as defined above;
R³ can also be any of the following when R⁴ is absent:
   (28) oxo,
   (29) =CH-(C₁-C₆)-alkyl, wherein alkyl is as defined above,
   (30) =CH-(C₂-C₆)-alkenyl, wherein alkenyl is as defined above,
   (31) =CH-aryl, wherein aryl is as defined above, or
   (32) =CH₂;
R⁸ and R⁹ are independently selected from the group consisting of:
   (1) hydrogen,
   (2) [(C=O)Oᵣ]ₛaryl, wherein aryl is as defined above,
   (3) [(C=O)Oᵣ]ₛ(C₂-C₈)-alkenyl, wherein alkenyl is as defined above, and
   (4) [(C=O)Oᵣ]ₛ(C₁-C₈)-alkyl, wherein alkyl is as defined above, and
R¹⁰ is:
   (1) hydrogen, or
   (2) [(C=O)Oᵣ]ₛ(C₁-C6)-alkyl, wherein alkyl is as defined above.

Another preferred emodiment is the compound of Formula III,
wherein R¹, R², R⁵, R⁶ and R⁷ are independently:
   (1) halo, wherein halo is fluoro, chloro, bromo, or iodo,
   (2) hydroxy,
   (3) (C₁-C₃)-alkyl,
   (4) (C₂-C₃)-alkenyl,
   (5) O(C₁-C₄)-alkyl, wherein the alkyl may be cyclic or straight-chained,
   (6) O(CO)CH₃,
   (7) CO(C₁-C₃)-alkyl,
   (8) CO₂(C₁-C₃)-alkyl,
   (9) hydrogen,
   (10) R¹ and R² or R⁶ and R⁷ can an be taken together when on adjacent carbons to form a fused benzo, dihydrofuranyl, furanyl, pyrrolidyl, dihydropyrrolidyl or 1,3-dioxolan group;
R³ and R⁴ are independently:
   (1) hydrogen,
   (2) (C₁-C₆)-alkyl, wherein alkyl includes cyclic as well as acyclic groups and is unsubstituted or substituted with one or two of the substituents selected from the group consisting of:
      (a) halo, wherein halo is fluoro, chloro, bromo, or iodo,
      (b) hydroxy,
      (c) oxo,
      (d) O[(C=O)Oᵣ]ₛ(C₁-C₆)-alkyl, wherein r and s are independently 0 or 1,
      (e) CO₂(C₂-C₃)-alkenyl,
      (f) O[(C=O)Oᵣ]ₛ(C₁-C₆)-alkenyl, wherein r and s are independently 0 or 1,
      (g) NR⁸R⁹,
      (h) O(CO)NR⁸R⁹,
      (i) CHO,
      (j) CO₂H,
      (k) CO(C₁-C₆)-alkyl,
      (l) CO₂(C₁-C₆)-alkyl, wherein alkyl may be substituted with phenyl,
      (m) CONR⁸R⁹,
      (n) aryl, wherein aryl is defined as phenyl, unsubstituted or substituted with a substituent selected from the group consisting of:
         (a') halo, as defined above,
         (b') hydroxy,
         (c') (C₁-C₆)-alkyl, and
         (d') (C₁-C₆)-alkyloxy, and
      (o) O[(C=O)Oᵣ]ₛ(C₂-C₆)-alkenyl, wherein r and s are independently 0 or 1,
   (3) (C₂-C₆)-alkenyl,
   (4) aryl, wherein aryl is as defined above or
   (5) O(CH₂)ₙaryl, wherein aryl is as defined above;
R³ can also be any of the following when R⁴ is absent:
   (6) =CH-(C₁-C₆)-alkyl, wherein alkyl is as defined above,
   (7) =CH-(C₂-C₆)-alkenyl, wherein alkenyl is as defined above,
R⁸ and R⁹ are independently selected from the group consisting of:
   (1) hydrogen,
   (2) [(C=O)Oᵣ]ₛaryl, wherein aryl is as defined above and r and s are independently 0 or 1,
   (3) (C₂-C₈)-alkenyl, wherein alkenyl is as defined above,and
   (4) (C₁-C₆)-alkyl, wherein alkyl is as defined above, and
R¹⁰ is:
   (1) hydrogen, or
   (2) (C=O)(C₁-C₃)-alkyl, wherein alkyl is as defined above.

As used herein, the term "alkyl", unless otherwise indicated, includes those alkyl groups of a designated number of carbon atoms of either a straight, branched, or cyclic configuration (carbocycles). Examples of "alkyl" include methyl, ethyl, propyl, isopropyl, butyl, sec-and tert-butyl, pentyl, hexyl, heptyl, cyclopropyl, cyclobutyl, cyclopentyl, cyclohexyl, cycloheptyl, norbornyl, and the like. "Alkoxy" represents an alkyl group of indicated number of carbon atoms attached through an oxygen bridge, such as methoxy, ethoxy, propoxy, butoxy and pentoxy. The following illustrate the foregoing definitions: "(C₁-C₃)-alkyl" may be methyl, ethyl, propyl, isopropyl, or cyclopropyl. Similarly, "O-(C₁-C₃)-alkyl" may be methoxy, ethoxy, n-propoxy, i-propoxy, or cyclopropoxy. In some cases, a C₀ designation is used, as in "-(C₀-C₂)-alkyl-phenyl." In such a case, the substituent is intended to be any of the following: phenyl, benzyl, 1-phenylethyl,or 2-phenylethyl. In certain definitions, the alkyl may be substituted with one or more substituents. For example a definition which reads "(C₁-C₂)-alkyl, substituted with one or two substitutents selected from oxo, hydroxy, and halo" is intended to include C(O)CH₃, CH₂BrCH₃, CO₂H, C(OH)CH₃, CH₂CH₂(OH), CH₂CO₂H, CHBrCH₂Cl, CHO, and so on.

"Alkenyl" is intended to include hydrocarbon chains of a specified number of carbon atoms of either a straight- or branched- configuration and at least one unsaturation, which may occur at any point along the chain, such as ethenyl, propenyl, butenyl, pentenyl, dimethyl pentenyl, and the like, and includes E and Z forms, where applicable. "Halogen" and "halo", as used herein, mean fluoro, chloro, bromo and iodo.

The term "aryl," unless specifically defined otherwise, is defined as phenyl or naphthyl, unsubstituted or substituted with one, two or three of the substituents selected from the group consisting of halo, hydroxy, alkyl, perfluoroalkyl, alkenyl, alkynyl, alkyloxy, alkyl-S(O)ₙ-, phenyl, phenoxy, cyano, nitro, CO₂H, CO-alkyl, CO₂-alkyl, CONR⁸R⁹, and NR⁸R⁹.

The term "heteroaryl" as utilized herein, unless specifically defined otherwise, is intended to include the following: an unsubstituted, monosubstituted, or disubstituted five or six membered aromatic heterocycle containing from 1 to 3 heteroatoms selected from the group consisting of O, N and S and wherein the substituent is halo, hydroxy, alkyl, perfluoroalkyl, alkenyl, alkynyl, alkyloxy, -alkylS(O)ₙ-, phenyl, phenoxy, cyano, nitro, CO₂H, CO-alkyl, CO₂-alkyl, CONR⁸R⁹, NR⁸R⁹, or a fused benzo or pyridyl group. Heteroaryl groups within the scope of this definition include but are not limited to: acridinyl, carbazolyl, cinnolinyl, quinoxalinyl, pyrrazolyl, indolyl, benzotriazolyl, furanyl, thienyl, benzothienyl, benzofuranyl, quinolinyl, isoquinolinyl, pyrazinyl, pyridazinyl, pyridinyl, pyrimidinyl, and pyrrolyl which are substituted or unsubstituted as defined above.

In the compounds of Formula I, II and III, the heteroaryl or aryl groups may be optionally substituted with the substituents listed above at any available carbon atom or nitrogen atom (if present), but compounds bearing certain substitutents, directly substituted to a nitrogen may be relatively unstable and are not preferred. The heteroaryl may also be fused to a second 5-, 6-, or 7-membered ring containing one or two oxygens such as: dioxolanyl, dihydrofuranyl, dihydropyranyl, and dioxanyl. Disubstituted aryl groups may be ortho, para or meta and all three are intended unless specifically defined otherwise.

"Heterocyclyl" is defined as a 3 to 7 atom cyclic, non-aromatic substituent containing from 1 to 3 heteroatoms selected from the group consisting of O, N, and S, which is unsubstituted or substituted with one, two or three substituents selected from the group consisting of halo, hydroxy, alkyl, perfluoroalkyl, alkenyl, alkynyl, alkyloxy, alkyl-S(O)ₙ-, phenyl, phenoxy, cyano, nitro, CO₂H, COalkyl, CO₂-alkyl, CONR⁸R⁹, NR⁸R⁹, NR⁸CO-alkyl, oxo, fused benzo, and fused pyridyl group.

Pharmaceutically acceptable salts include both the metallic (inorganic) salts and organic salts; a list of which is given in Remington's Pharmaceutical Sciences, 17th Edition, pg. 1418 (1985).

A most preferred embodiment of the compounds of formula III is a compound which is selected from the group consisting of: 1-(2-methoxyphenyl)-1-oxo-2-aza-(S)-4-i-butyl-4-phenylbutane, 1-(2-methoxyphenyl)-1-oxo-2-aza-4-(S)-((3-allyloxycarbonyloxy)propyl))-4-phenylbutane, 1-(2-methoxyphenyl)-1-oxo-2-aza-4,4-diethyl-4-phenylbutane, 1-(2,3-dihydrobenzofuran-7-yl)-1-oxo-2-aza-4,4-diethyl-4-(phenyl)-butane, and 1-(2-methoxyphenyl)-1-oxo-2-aza-4-(S)-(3-hydroxypropyl)-4-phenylbutane.

The most preferred potassium channel blockers of the claimed invention are: 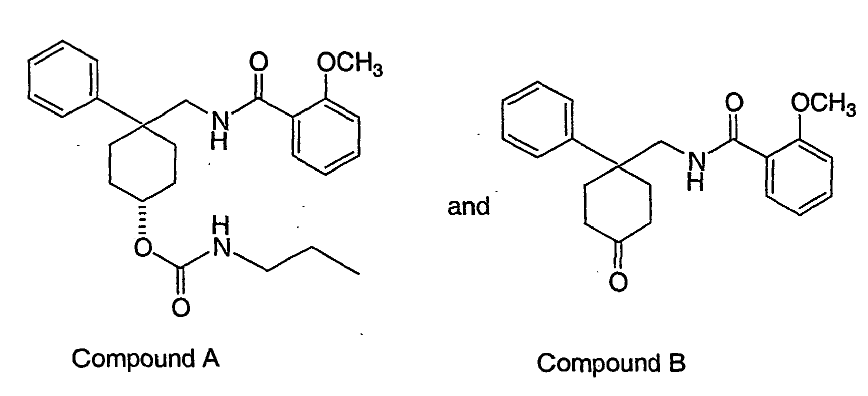 or a pharmaceutically acceptable salt, ester, diatereomer or enantiomer thereof. Generally, 3 to 10 micromolar of the potassium channel blocker is added in the well, preferable 3 to 5 micromolar.

An aspect of this embodiment is realized when the potassium channel blocker that selectively eliminates endogenous potassium conductances of the HEK-293 cells does so without affecting the maxi-K channel activity.

Still another preferred embodiment of this invention is realized when the HEK-293 cells are transfected with maxi-K channel alpha and beta 1 subunits before incubation with the potassium channel blocker.

The above method is used to identify maxi-K channel blockers, which are useful for lowering IOP. Under appropriate conditions, as described herein, the maxi-K channel sets the resting potential of the HEK-293 cells. Generally, addition of high-potassium solution causes the cells to depolarize and this activity can be monitored with fluorescence dyes using a voltage/ion probe reader (VIPR) instrument. Preincubation of the cells with an inhibitor of the maxi-K channel will lead to cell depolarization. Under these conditions, addition of the high-potassium solution will not cause any change in the emission properties of the fluorescence dyes because the cells are already predepolarized. Because HEK-293 cells have endogenous potassium conductances, these conductances have to be eliminated so that the maxi-K channel is the predominate one setting the resting potential at Eₖ. Elimination is achieved when the HEK-293 cells are incubated with a potassium channel blocker prior to adding a test compound. The consequence of this pharmacological manipulation is the generation of a very large screening window where the fluorescence signal denoting a hyperpolarized resting potential is abolished by selective maxi-K channel blockers.

Untransfected HEK-293 cells are commercially available. The HEK-293 cells can be transfected as described herein.

The identification of inhibitors of the Maxi-K channel is based on the ability of expressed Maxi-K channels to set cellular resting potential after transfection of both alpha and beta1 subunits of the channel in HEK-293 cells and after being incubated with potassium channel blockers that selectively eliminate the endogenous potassium conductances of HEK-293 cells. In the absence of maxi-K channel inhibitors, the transfected HEK-293 cells display a hyperpolarized membrane potential, negative inside, close to E_{K} (-80 mV) which is a consequence of the activity of the maxi-K channel. Blockade of the Maxi-K channel by incubation with maxi-K channel blockers will cause cell depolarization. Changes in membrane potential can be determined with voltage-sensitive fluorescence resonance energy transfer (FRET) dye pairs that use two components, a donor coumarin (CC₂DMPE) and an acceptor oxanol (DiSBAC₂(3)).

Oxanol is a lipophilic anion and distributes across the membrane according to membrane potential. Under normal conditions, when the inside of the cell is negative with respect to the outside, oxanol is accumulated at the outer leaflet of the membrane and excitation of coumarin will cause FRET to occur. Conditions that lead to membrane depolarization will cause the oxanol to redistribute to the inside of the cell, and, as a consequence, to a decrease in FRET. Thus, the ratio change (donor/acceptor) increases after membrane depolarization, which determines if a test compound actively blocks the maxi-K channel.

The HEK-293 cells were obtained from the American Type Culture Collection, 12301 Parklawn Drive, Rockville, Maryland, 20852 under accession number ATCC CRL-1573. Any restrictions relating to public access to the cell lines shall be irrevocably removed upon patent issuance.

Transfection of the alpha and beta1 subunits of the maxi-K channel in HEK-293 cells was carried out as follows: HEK-293 cells were plated in 100 mm tissue culture treated dishes at a density of 3x10⁶ cells per dish, and a total of five dishes were prepared. Cells were grown in a medium consisting of Dulbecco's Modified Eagle Medium (DMEM) supplemented with 10% Fetal Bovine serum, 1X L-Glutamine, and 1X Penicillin/Streptomycin, at 37°C, 10% CO₂. For transfection with Maxi-K hα (pCIneo) and Maxi-K hβ1 (pIRESpuro) DNAs, 150 µl FuGENE6^{™} was added dropwise into 10 ml of serum free/phenol-red free DMEM and allowed to incubate at room temperature for 5 minutes. Then, the FuGENE6^{™} solution was added dropwise to a DNA solution containing 25 µg of each plasmid DNA, and incubated at room temperature for 30 minutes. After the incubation period, 2 ml of the FuGENE6^{™}/DNA solution was added dropwise to each plate of cells and the cells were allowed to grow two days under the same conditions as described above. At the end of the second day, cells were put under selection media which consisted of DMEM supplemented with both 600 µg/ml G418 and 0.75 µg/ml puromycin. Cells were grown until separate colonies were formed. Five colonies were collected and transferred to a 6 well tissue culture treated dish. A total of 75 colonies were collected. Cells were allowed to grow until a confluent monolayer was obtained. Cells were then tested for the presence of maxi-K channel alpha and beta 1 subunits using an assay that monitors binding of ¹²⁵I-iberiotoxin-D19Y/Y36F to the channel. Cells expressing ¹²⁵I-iberiotoxin-D19Y/Y36F binding activity were then evaluated in a functional assay that monitors the capability of maxi-K channels to control the membrane potential of transfected HEK-293 cells using fluorescence resonance energy transfer (FRET) ABS technology with a VIPR instrument. The colony giving the largest signal to noise ratio was subjected to limiting dilution. For this, cells were resuspended at approximately 5 cells/ml, and 200 µl were plated in individual wells in a 96 well tissue culture treated plate, to add ca. one cell per well. A total of two 96 well plates were made. When a confluent monolayer was formed, the cells were transferred to 6 well tissue culture treated plates. A total of 62 wells were transferred. When a confluent monolayer was obtained, cells were tested using the FRET-functional assay. Transfected cells giving the best signal to noise ratio were identified and used in subsequent functional assays.

1. To measure binding of ¹²⁵I-iberiotoxin-D19Y/Y36F to transfected HEK-293 cells, cells were plated in poly-D-lysine treated 96 wells at a density of 40,000 cells/well. Cells were grown overnight under selection medium. Then, the medium is removed and 200 µl of a solution containing about 70 pM ¹²⁵I-iberiotoxin-D19Y/Y36F in selection medium is added per well. For determination of nonspecific binding the same medium also contained 100 nM unlabeled iberiotoxin. Cells are incubated with this solution for four hours at 37°C, 10% CO₂. After incubation, radioactive medium is removed and cells are washed one time with D-PBS. Then, 200 µl of Microscint-20 is added to each well and radioactivity associated with the cells is determined in a Packard Topcount instrument.

The transfected cells (2E+06 Cells/mL) are then plated on 96-well poly-D-lysine plates at a density of about 100,000 cells/well and incubated for about 16 to about 24 hours. The medium is aspirated of the cells and the cells washed one time with 100 µl of Dulbecco's phosphate buffered saline (D-PBS). One hundred microliters of about 9 µM coumarin (CC₂DMPE)-0.02% pluronic-127 in D-PBS per well is added and the wells are incubated in the dark for about 30 minutes. The cells are washed two times with 100 µl of Dulbecco's phosphate-buffered saline and 100 µl of about 4.5 µM of oxanol (DiSBAC₂(3)) in (mM) 140 NaCl, 0.1 KCl, 2 CaCl₂, 1 MgCl₂, 20 Hepes-NaOH, pH 7.4, 10 glucose is added. Three micromolar of an inhibitor of endogenous potassium conductance of HEK-293 cells such as Compounds A or B is added. A maxi-K channel blocker is added (about 3 micromolar to about 10 micromolar) and the cells are incubated at room temperature in the dark for about 30 minutes.

The plates are loaded into a voltage/ion probe reader (VIPR) instrument, and the fluorescence emission of both CC₂DMPE and DiSBAC₂(3) are recorded for 10 sec. At this point, 100 µl of high-potassium solution (mM): 140 KCl, 2 CaCl₂, 1 MgCl₂, 20 Hepes-KOH, pH 7.4, 10 glucose are added and the fluorescence emission of both dyes recorded for an additional 10 sec. The ratio CC₂DMPE/DiSBAC₂(3), before addition of high-potassium solution equals 1. In the absence of maxi-K channel inhibitor, the ratio after addition of high-potassium solution varies between 1.65-2.0. When the Maxi-K channel has been completely inhibited by either a known standard such as compounds 1-6 or test compound, this ratio remains at 1. It is possible, therefore, to titrate the activity of a Maxi-K channel inhibitor by monitoring the concentration-dependent change in the fluorescence ratio.

Maxi-K channel blockers that can be used in the claimed method are those disclosed in Merck & Co., Inc. Attorney Docket number 20798PV, filed January 30, 2001 under U.S.S.N 60/264,954, and simultaneously with this application. U.S.S.N. 60/176,695, filed January 18, 2000 and U.S.SN 60/176,694, filed January 19, 2000 also disclose maxi-K channel blockers that can be used in the claimed method. Examples of the maxi-K channel blockers that can be evaluated with this method are:

### Compound

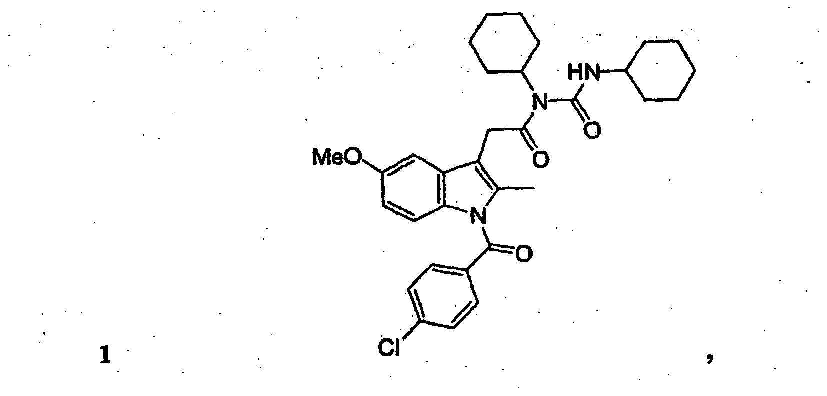

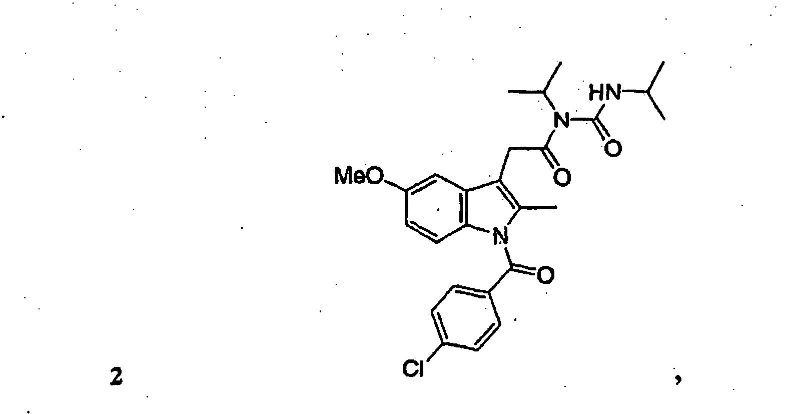

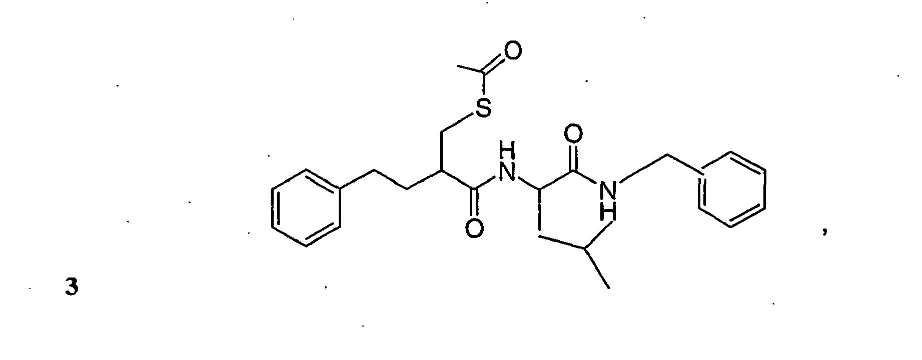

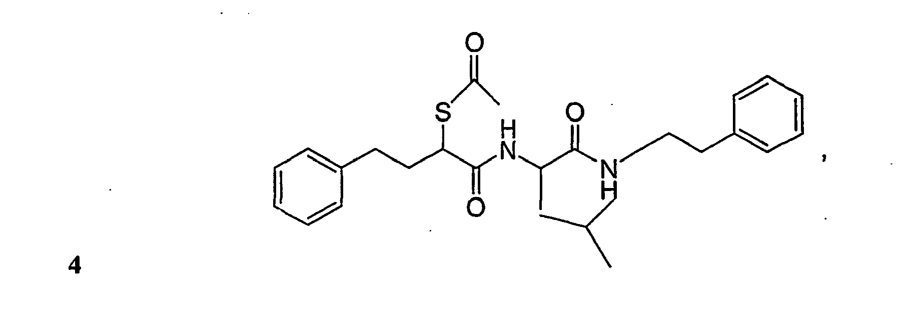

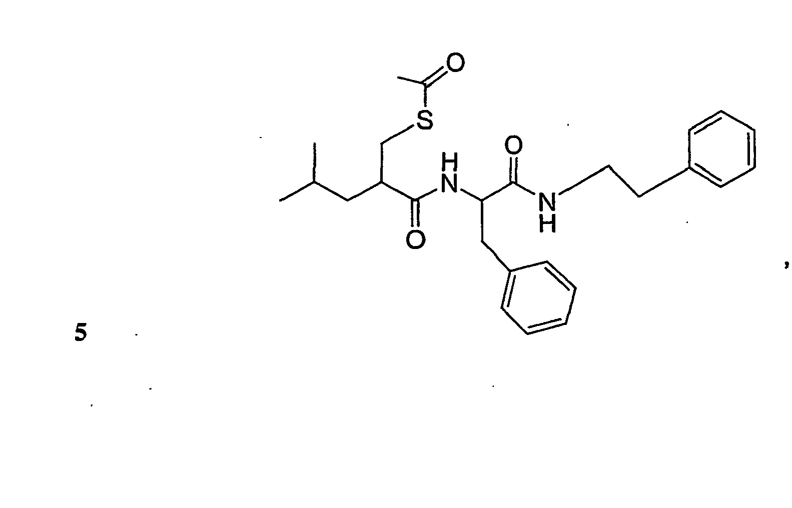

or

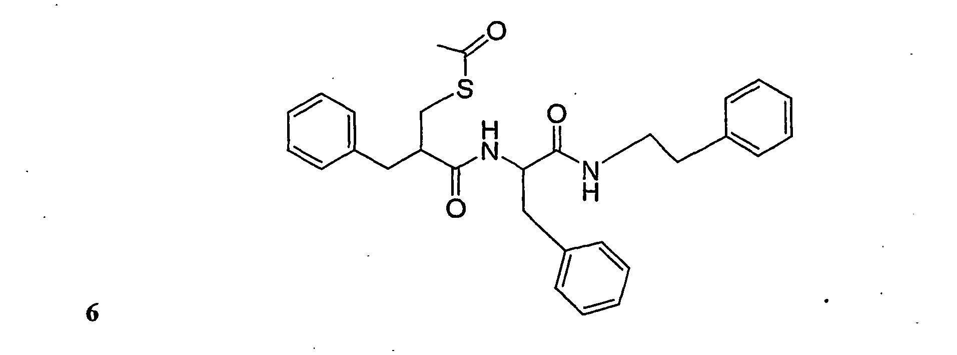

The process of this invention can be understood further by the following examples, which do not constitute a limitation of the invention.

### EXAMPLE

HEK-293 cells were plated on 96-well poly-D-lysine plates and incubated for about 16 to 24 hours at 37°C. The media was aspirated and the cells were washed once with D-PBS. Cells were incubated with 9 µM coumarin (CC2DMPE)-0.02% pluronic-127 in D-PBS for 30 minutes in the dark. Cells were washed twice with D-PBS, and incubated with 4.5 µM oxanol (DiSBAC₂(3)) in the absence (Control) or presence of increasing concentrations of test compound, in the dark for 30 minutes. Plates were loaded onto a VIPR instrument and the fluorescence emission of both dyes was recorded for 10 seconds. A solution containing high-potassium was then added and the fluorescence emission of the dyes was recorded for another 10 seconds. The coumarin/oxanol ratio was calculated under both conditions. Before addition of high-potassium, this ratio was always normalized to 1.

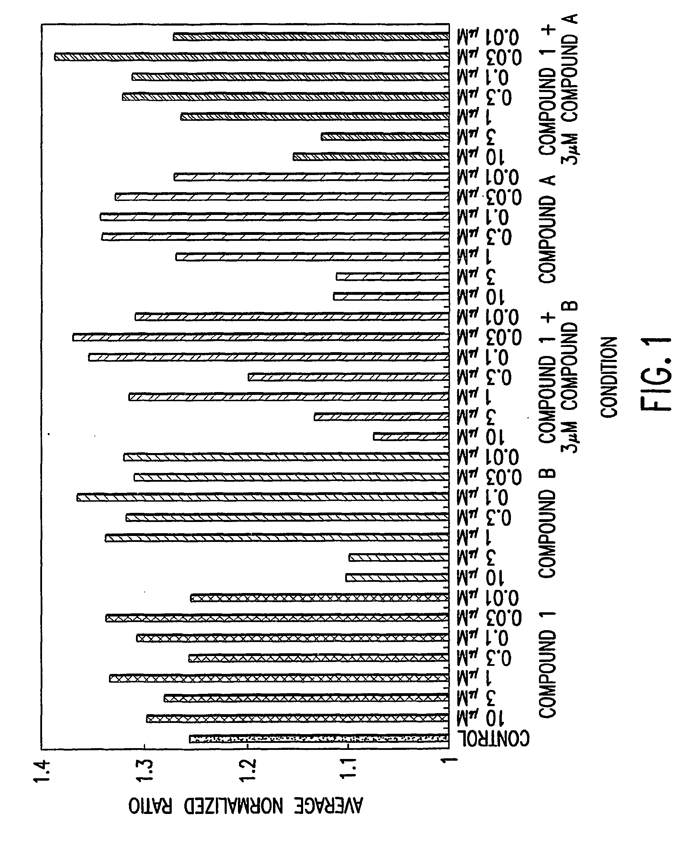

Figure 1 shows the effect of high-potassium addition to HEK-293 cells. Control cells display a signal suggesting the presence of some potassium conductance that contributes to setting the resting potential of the cells. The signal is not affected by the presence of different concentrations of the selective maxi-K channel blocker, Compound 1. However, in the presence of either Compound A or Compound B there is a concentration-dependent inhibition of the fluorescence signal suggesting that those channels that contribute to setting the resting potential of the cells are sensitive to the presence of these agents. A useful concentration range for these agents is 3-10 µM. Importantly, in the presence of the selective maxi-K channel blocker Compound 1, the efficacy of either Compound A or Compound B is unaffected.

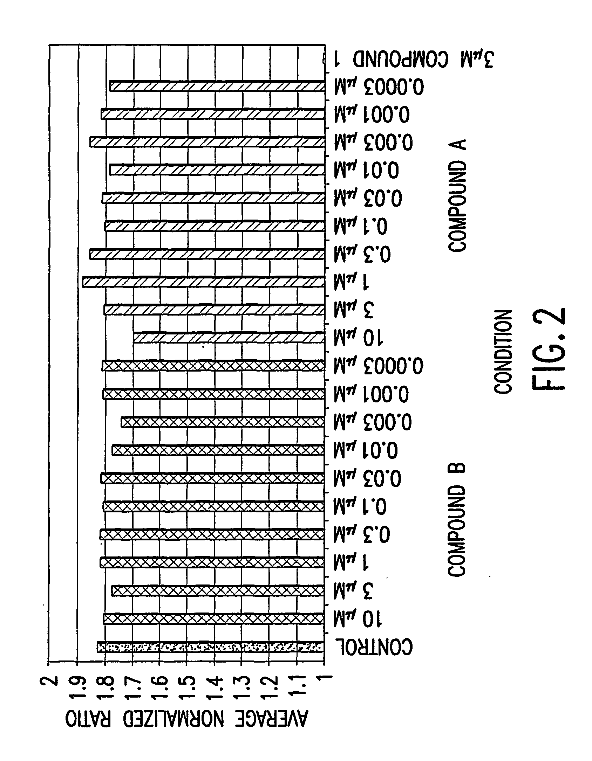

Figure 2 shows the effect of the potassium channel blockers Compound A and Compound B on maxi-K channel activity using maxi-K channels transiently expressed in TsA-201 cells. The presence of 3 µM Compound 1 completely inhibits the control signal. However, neither Compound A, nor Compound B have any effect on maxi-K channel activity.

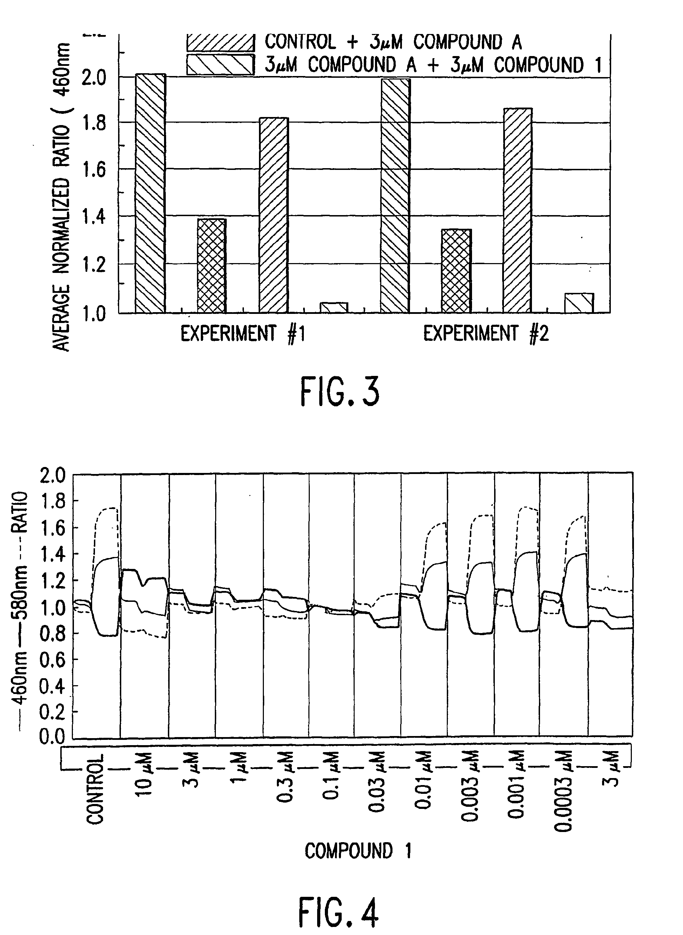

Figure 3 shows the effect of the potassium channel blocker Compound A. on maxi-K channel activity using HEK-293 cells stably transfected with both alpha and beta1 subunits of the maxi-K channel. The maxi-K channel blocker Compound 1 only partially blocks the signal generated by addition of a high-potassium solution suggesting that other potassium conductances, in addition to maxi-K channels, control the resting potential of the transfected HEK cells. However, in the presence of the potassium channel backer Compound A, addition of Compound 1 causes complete inhibition of the signal. Importantly, Compound A has no significant effect on the magnitude of the signal caused by addition of high-potassium solution. These data indicate that by blocking the endogenous potassium conductances of HEK cells with the potassium channel blockers, the maxi-K channel becomes the only contributor to the control of cell resting potential of the transfected cells.

Figure 4 shows representative fluorescence traces obtained in the VIPR instrument with HEK-293 cells stably transfected with both alpha and beta1 subunits of the maxi-K channel. Recordings from twelve different wells are shown. The horizontal scale represents time, whereas the vertical axis shows the fluorescence readings of both coumarin (460 nm) and oxanol (580 nm). The ratio coumarin/oxanol is also indicated. In all wells, 3 µM Compound A was present to block endogenous potassium conductances of HEK cells. Addition of increasing concentrations of the selective maxi-K channel blocker, Compound 1, causes a concentration-dependent inhibition of the control signal. This assay can thus be used to identify inhibitors of the maxi-K channel.

## Claims

1. A method for identifying maxi-K channel blockers, which are useful for lowering intraocular pressure (1), using HEK-293 cells, comprising the steps of:
(a) constructing stable HEK-293 cell lines expressing both alpha and beta 1 subunits of the maxi-K channel;
(b) incubating the HEK-293 cells with a potassium blocker which selectively eliminates the endogenous potassium conductance of HEK-293 cells without affecting the maxi-K channel activity; (2)
(c) loading the incubated HEK-293 cells with a test compound and incubating; and
(d) measuring the maxi-K channel blocker activity of the test compound using a voltage/ion probe reader instrument and fluorescence resonance energy transfer (3) based membrane potential sensing dyes to monitor membrane potential.

2. A method according to claim 1 which comprises the steps of:
(a) plating the HEK-293 cells, 2E+06 Cells/mL, on 96-well poly-D-lysine plates at a density of about 100,000 cells/well;
(b) incubating the plates for 16 to 24 hours;
(c) aspirating medium off the cells;
(d) washing the cells 1 time with Dulbecco's phosphate-buffered saline;
(e) adding 100 µl of 9 µM coumarin dye-0.02% pluronic-127 in Dulbecco's phosphate-buffered saline (4) per well;
(f) incubating in the dark for about 30 minutes;
(g) washing the cells 2 times with Dulbecco's phosphate-buffered saline;
(h) adding 100 µl of 4.5 µM oxanol dye in (mM): 140 NaCl, 0.1 KCl, 2 CaCl₂, 1 MgCl₂, 20 Hepes-NaOH, pH at about 7.4, 10 glucose per well;
(i) adding an inhibitor of endogenous potassium conductance of HEK-293 cells;
(j) adding a test compound;
(k) incubating at room temperature for about 30 minutes;
(l) loading the plates on to a voltage/ion probe reader and recording fluorescence emission of both dyes for about 10 seconds;
(m) adding 100 µl of high-potassium solution in (mM) :140 KCl, 2 CaCl₂, 1 MgCl₂, 20 Hepes-KOH, pH at about 7.4, 10 glucose; and recording fluoresence emission of both dyes for about 10 seconds and determining membrane potential from those data using a voltage/ion probe reader instrument.

3. A method in accordance with claim 1 or 2 wherein the potassium channel blocker is a compound of structural formula I structural Formula I: 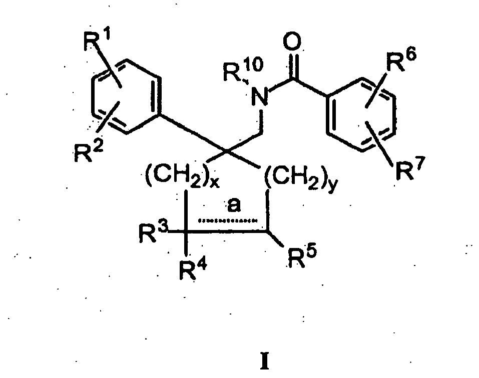 or a pharmaceutically acceptable salt, crystal form, or hydrate, wherein
a is a single bond or a double bond when R⁴ is absent, and represented by ===, in the structure above, with the proviso that a is a single bond when x + y = 0;
nis: 0, 1, 2 or 3;
r is: 0 or 1;
s is: 0 or 1;
x and y are independently 0, 1, or 2;
R¹, R², R⁶ and R⁷ are independently:
(1) halo, wherein halo is fluoro, chloro, bromo, or iodo,
(2) hydroxy,
(3) (C₁-C₆)-alkyl,
(4) HO(C₁-C₆)-alkyloxy,
(5) (C₁-C₄)-perfluoroalkyl,
(6) (C₂-C₆)-alkenyl,
(7) (C₂-C₆)-alkynyl,
(8) O[(C=O)Oᵣ]ₛ(C₁-C₆)-alkyl,
(9) (C₁-C₆)-alkyl-S(O)ₙ-,
(10) -(O)ᵣ-(C₀-C₆)-alkyl-aryl, wherein aryl is phenyl or naphthyl unsubstituted or substituted with up to three substitutents selected from(C₁-C₃)alkyl, trifluoromethyl, and halo;
(11) -(O)ᵣ-heteroaryl, wherein heteroaryl is pyridinyl or pyrryl,
(12) cyano,
(13) nitro,
(14) CO₂H,
(15) CO(C₁-C₆)-alkyl,
(16) CO₂(C₁-C₆)-alkyl,
(17) CONR⁸R⁹,
(18) NR⁸R⁹,
(20) (C₂-C₆)-alkenyloxy,
(21) (CO)-aryl, wherein aryl is phenyl, naphthyl, benzothienyl, or a benzophenone radical and is unsubstituted or substituted with up to two substituents selected from halo, trifluromethyl, and (C₁-C₃)alkyl,
(22) hydrogen,
(23) OCF₃,
(24) -(CH₂)-O-N=C(CH₃)(aryl), wherein aryl is phenyl or naphthyl and is unsubstituted or substituted with up to three halogen substituents,
(25) -S(O)ₙ-N R⁸R⁹, or
(26) R¹ and R² or R⁶ and R⁷ can an be taken together when on adjacent carbons to form a fused benzo, dihydrofuranyl, furanyl, pyrrolidyl, dihydropyrrolidyl or 1,3-dioxolan group;
R³ and R⁴ are independently:
(1) halo, wherein halo is fluoro, chloro, bromo, or iodo,
(2) hydroxy, when a is a single bond,
(3) HO(C₁-C₆)-alkyloxy,
(4) (C₁-C₄)-perfluoroalkyl,
(5) O(CO)CCl₃,
(6) (C₁-C₆)-alkyl-S(O)ₙ-,
(7) phenyl-(CH₂)ᵣ-S(O)ₙ-,
(8) cyano,
(9) nitro,
(10) CO₂H,
(11) CO(C₁-C₆)-alkyl,
(12) CO₂(C₁-C₆)-alkyl,
(13) CONR⁸R⁹,
(14) NR⁸R⁹,
(15) O(CO)NR⁸R⁹,
(16) azido,
(17) NR⁸(CO)NR⁸R⁹,
(18) hydrogen,
(19) (C₁-C₁₀)-alkyl, wherein alkyl includes cyclic as well as acyclic groups and is unsubstituted or substituted with one or two of the substituents selected from the group consisting of:
(a) halo, wherein halo is fluoro, chloro, bromo, or iodo,
(b) hydroxy,
(c) oxo,
(d) O[(C=O)Oᵣ]ₛ(C₁-C₆)-alkyl,
(e) (C₁-C₆)-alkyl-S(O)ₙ-,
(f) aryl-(C₁-C₆)-alkyloxy,
(g) cyano,
(h) nitro,
(i) vinyl,
(j) NR⁸R⁹,
(k) O(CO)NR⁸R⁹,
(l) CHO,
(m) CO₂H,
(n) CO(C₁-C₆)-alkyl,
(o) CO₂(C₁-C₆)-alkyl,
(p) CONR⁸R⁹,
(q) aryl, wherein aryl is defined as phenyl or naphthyl, unsubstituted or substituted with one, two or three of the substituents selected from the group consisting of:
(a') halo, as defined above,
(b') hydroxy,
(c') (C₁-C₆)-alkyl,
(d') (C1-C4)-perfluoroalkyl,
(e') (C₂-C₆)-alkenyl,
(f') (C₂-C₆)-alkynyl,
(g') (C₁-C₆)-alkyloxy,
(h') (C₁-C₆)-alkyl-S(O)ₙ-,
(i') phenyl,
(j') phenoxy,
(k') cyano,
(l') nitro,
(m') CO₂H,
(n') CO(C₁-C₆)-alkyl,
(o') CO₂(C₁-C₆)-alkyl,
(p') CONR⁸R⁹, and
(q') NR⁸R⁹,
(r) heteroaryl, wherein heteroaryl is defined as an unsubstituted, monosubstituted, or disubstituted five or six membered aromatic heterocycle containing from 1 to 3 heteroatoms selected from the group consisting of O, N and S and wherein the substituents are members selected from the group consisting of:
(a') halo, as defined above,
(b') hydroxy,
(c') (C₁-C₆)-alkyl,
(d') (C1-C4)-perfluoroalkyl,
(e') (C₂-C₆)-alkenyl,
(f') (C₂-C₆)-alkynyl,
(g') (C₁-C₆)-alkyloxy,
(h') (C₁-C₆)-alkyl-S(O)ₙ-,
(i') phenyl,
(j') phenoxy,
(k') cyano,
(l') nitro,
(m') CO₂H,
(n') CO(C₁-C₆)-alkyl,
(o') CO₂(C₁-C₆)-alkyl,
(p') CONR⁸R⁹,
(q') NR⁸R⁹, and
(r') fused benzo or pyridyl group,
(s) heterocyclyl, wherein heterocyclyl is defined as a 3 to 7 atom cyclic, non-aromatic substituent containing from 1 to 3 heteroatoms selected from the group consisting of O, N, and S, said heterocycle being unsubstituted or substituted with one, two or three substituents selected from the group consisting of:
(a') halo, as defined above,
(b') hydroxy,
(c') (C₁-C₆)-alkyl,
(d') (C1-C4)-perfluoroalkyl,
(e') (C₂-C₆)-alkenyl,
(f') (C₂-C₆)-alkynyl,
(g') (C₁-C₆)-alkyloxy,
(h') (C₁-C₆)-alkyl-S(O)ₙ-,
(i') phenyl,
(j') phenoxy,
(k') cyano,
(l') nitro,
(m') CO₂H,
(n') CO(C₁-C₆)-alkyl,
(o') CO₂(C₁-C₆)-alkyl,
(p') CONR⁸R⁹,
(q') NR⁸R⁹,
(r') NR⁸CO(C₁-C₆)-alkyl,
(s') oxo,
(t') fused benzo, and
(u') fused pyridyl group;
(t) benzyl-S(O)ₙ-,
(u) O[(C=O)Oᵣ]ₛ(C₂-C₆)-alkenyl,
(v) O[(C=O)Oᵣ]ₛaryl,
(w) O[(C=O)Oᵣ]ₛheteroaryl,
(x) O(CH₂)ₙheteroaryl, or
(y) O(CH₂)ₙaryl;
(20) (C₂-C₁₀)-alkenyl, wherein alkenyl is unsubstituted or substituted with one or two of the substituents selected from the group consisting of:
(a) halo, wherein halo is fluoro, chloro, bromo, or iodo,
(b) hydroxy,
(c) oxo,
(e) (C₁-C₆)-alkyl-S(O)ₙ-,
(f) phenyl-(C₁-C₆)-alkyloxy,
(g) cyano,
(h) nitro,
(i) NR⁸R⁹,
(j) CHO,
(k) CO₂H,
(l) CO(C₁-C₆)-alkyl,
(m) CO₂(C₁-C₆)-alkyl,
(n) CONR⁸R⁹,
(o) aryl, wherein aryl is as defined above,
(p) heteroaryl, wherein heteroaryl is as defined above,
(q) heterocyclyl, wherein heterocyclyl is as defined above,
(r) O[(C=O)Oᵣ]ₛ(C₁-C₆)-alkyl, alkyl as defined above,
(s) O[(C=O)Oᵣ]ₛ(C₂-C₆)-alkenyl, as defined above,
(t) O[(C=O)Oᵣ]ₛaryl, aryl as defined above,
(u) O[(C=O)Oᵣ]ₛheteroaryl, heteroaryl as defined above,
(v) O(CH₂)ₙheteroaryl, heteroaryl as defined above, and
(w) O(CH₂)ₙaryl, aryl as defined above;
(21) (C₂-C₁₀)-alkynyl, wherein alkynyl is unsubstituted or substituted with one or two of the substituents selected from the group consisting of:
(a) halo, wherein halo is fluoro, chloro, bromo, or iodo,
(b) hydroxy,
(c) oxo,
(d) (C₁-C₆)-alkyloxy,
(c) (C₁-C₆)-S(O)ₙ-,
(f) phenyl-(C₁-C₆)-alkyloxy,
(g) cyano,
(h) nitro,
(i) vinyl,
(j) NR⁸R⁹,
(k) NR⁸CO(C₁-C₆)-alkyl,
(l) CHO,
(m) CO₂H,
(n) CO(C₁-C₆)-alkyl,
(o) CO₂C(C₁-C₆)-alkyl,
(p) CONR⁸R⁹,
(q) aryl, wherein aryl is as defined above,
(r) heteroaryl, wherein heteroaryl is as defined above,
(s) heterocyclyl, wherein heterocyclyl is as defined above,
(t) O[(C=O)Oᵣ]ₛ(C₁-C₆)-alkyl, alkyl as defined above,
(u) O[(C=O)Oᵣ]ₛ(C₂-C₆)-alkenyl, as defined above,
(v) O[(C=O)Oᵣ]ₛaryl, aryl as defined above,
(w) O[(C=O)Oᵣ]ₛheteroaryl, heteroaryl as defined above
(x) O(CH₂)ₙheteroaryl, heteroaryl as defined above, and
(y) O(CH₂)ₙaryl, aryl as defined above,
(22) O[(C=O)Oᵣ]ₛ(C₁-C₆)-alkyl, alkyl as defined above,
(23) O[(C=O)Oᵣ]ₛ(C₂-C₆)-alkenyl, as defined above,
(24) O[(C=O)Oᵣ]ₛaryl, aryl as defined above,
(25) O[(C=O)Oᵣ]ₛheteroaryl, heteroaryl as defined above
(26) O(CH₂)ₙheteroaryl, heteroaryl as defined above,
(27) aryl, wherein aryl is as defined above,
(28) O[(C=O)Oᵣ]ₛheteroaryl, heteroaryl as defined above,
(29) (O(CO)NH(CH2-CO-NR⁸R⁹), or
(30) O(CH₂)ₙaryl, aryl as defined above;
R³ can also be any of the following when a is a single bond and R⁴ is absent:
(31) oxo,
(32) =CH-(C₁-C₆)-alkyl, wherein alkyl is as defined above,
(33) =CH-(C₂-C₆)-alkenyl, wherein alkenyl is as defined above,
(34) =CH-aryl, wherein aryl is as defined above,
(35) =CH₂, or
R³ and R⁴ can be taken together to form a spiro-fused heterocyclyl group, wherein heterocyclyl is as defined above, or
R³ and R⁵ can be taken together to form a fused oxirane when a is a single bond, with the proviso that R⁴ is absent when a is a double bond;
R⁵ is:
(1) hydrogen,
(2) halogen,
(3) (C₂-C₆)-alkenyl,
(4) hydroxy,
(5) O[(C=O)Oᵣ]ₛ(C₁-C₆)-alkyl,
(6) O(CO) NR⁸R⁹,
(7) oxo, when a is a single bond, or
R⁵ and R³ can be taken together to form a fused oxirane when a is a single bond;
R⁸ and R⁹ are independently selected from the group consisting of:
(1) hydrogen,
(2) [(C=O)Oᵣ]ₛaryl, wherein aryl is as defined above,
(3) [(C=O)Oᵣ]ₛ(C₁-C₈)-alkenyl, wherein alkenyl is as defined above,
(4) [(C=O)Oᵣ]ₛ(C₁-C₈)-alkyl, wherein alkyl is as defined above,
(5) (C=O)ᵣS(O)ₙ(C₁-C₈)-alkyl, wherein alkyl is as defined above,
(6) (C=O)ᵣS(O)ₙaryl, wherein aryl is as defined above, and
(7) heterocyclyl, wherein heterocyclyl is defined above;
R¹⁰ is:
(1) hydrogen,
(2) [(C=O)Oᵣ]ₛaryl, wherein aryl is as defined above, or
(3) [(C=O)Oᵣ]ₛ(C₁-C6)-alkyl, wherein alkyl is as defined above.

4. A method according to claim 3 wherein compound of formula I is further defined as in the compound of Formula II wherein x is 2 and y is 1.

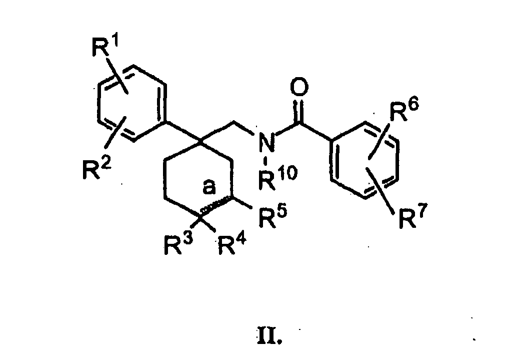

5. A method in accordance with claim 4 wherein a is further defined as a single bond;
R¹, R², R⁶ and R⁷ are independently:
(1) halo, wherein halo is fluoro, chloro, bromo, or iodo,
(2) hydroxy,
(3) (C₁-C₆)-alkyl,
(4) HO(C₁-C₆)-alkyloxy,
(5) (C₁-C₆)-alkyloxy, wherein the alkyl is cyclic or straight- chained,
(6) acetoxy,
(7) nitro,
(8) NR⁸R⁹,
(9) -(O)ᵣ(C₀-C₃)-aryl, wherein aryl is phenyl or naphthyl unsubstituted or substituted with up to three substitutents selected from(C₁-C₃)alkyl, trifluoromethyl, and halo,
(10) hydrogen,
(11) (O)ᵣCF₃,
(12) (C₁-C₆)-alkyl-S(O)ₙ-, wherein n is 0, 1, 2 or 3,
(13) (CO₂)-(C₁-C₆)-alkyl,
(14) -(O)ᵣ-heteroaryl, wherein heteroaryl is pyridinyl or pyrryl,
(15) (CO)-aryl, wherein aryl is phenyl, naphthyl, benzothienyl, or benzophenone radical and is unsubstituted or substituted with up to two substituents selected from halo, trifluromethyl, and (C₁-C₃)alkyl,
(16) -(CH₂)-O-N=C(CH₃)(aryl), wherein aryl is phenyl, unsubstituted or substituted with up to three halogen substituents,
(17) -S(O)ₙ-N R⁸R⁹, or
(18) R¹ and R² or R⁶ and R⁷ can an be taken together to form a fused benzo, dihydrofuranyl, furanyl, pyrrolidyl, dihydropyrrolidyl or 1,3-dioxolan group;
R³ and R⁴ are independently:
(1) halo, wherein halo is fluoro, chloro, bromo, or iodo,
(2) hydroxy,
(3) HO(C₁-C₆)-alkyloxy,
(4) (C=O)O(C₁-C₆)-alkyl,
(5) O(CO)CCl₃,
(6) (C₁-C₆)-alkyl-S(O)ₙ-, wherein n is 0, 1, 2 or 3,
(7) CH₂CO₂-(C₁-C₆)-alkyl,
(8) cyano,
(9) benzyloxy,
(10) CH₂OAc,
(11) OAc,
(12) (C₂-C₆)-alkenyl,
(13) (C₁-C₆)-alkyl, wherein alkyl can be unsubstituted or substituted with bromide
(14) NR⁸R⁹,
(15) O(CO)NR⁸R⁹,
(16) azido,
(17) NR⁸(CO)NR⁸R⁹,
(18) hydrogen,
(19) CH₂OH,
(20) CH₂O(C=O)phenyl, wherein phenyl is unsubstituted or monosubstituted with methoxy,
(21) O(C₂-C₆)-alkenyl,
(22) O(C=O)-phenyl, wherein phenyl is unsubstituted or monosubstituted with bromide,
(23) O(C=O)O-phenyl, wherein phenyl is unsubstituted or monosubstituted with nitro,
(24) CH₂(CO)NR⁸R⁹,
(25) O(C=O)O-(C₂-C₆)-alkenyl,
(26) O(C=O)-(C₁-C₃)-alkyl, wherein the alkyl can be unsubstituted or substituted with bromide or -CO₂CH₃,
(27) O(C₁-C₆)-alkyl, wherein alkyl can be unsubstituted or substituted with phenyl,
(28) O(C=O)O-(C₁-C₆)-alkyl,
(29) CH₂O(CO)-NR⁸R⁹, or
(30) CH₂ (C=O)O-(C₁-C₆)-alkyl,
R³ can also be any of the following when R⁴ is absent:
(31) oxo,
(32) =CH₂,
(33) =CH-CO₂-(C₁-C₆)-alkyl,
(34) =CH-(CO)-NR⁸R⁹, or
(35) =CH-CO₂H, or
R³ and R⁴ can be taken together to form a spiro-fused heterocyclyl group, wherein heterocyclyl is defined as:
(36) oxirane,
(37) 1,3-dioxolan,
(38) 2,2-dimethyl-1,3-dioxolan, or
(39) glycol sulfite, or
R³ and R⁵ can be taken together to form a fused oxirane;
R⁵ is:
(1) hydrogen,
(2) halogen,
(3) (C₂-C₆)-alkenyl,
(4) hydroxy,
(5) O(C=O)(C₁-C₃)-alkyl,
(6) O(CO)NR⁸R⁹,
(7) oxo, when a is a single bond, or
R⁵ and R³ can be taken together to form a fused oxirane when a is a single bond;
R⁸ and R⁹ are independently selected from the group consisting of:
(1) hydrogen,
(2) (C=O)O(C₁-C₆)-alkyl, wherein alkyl is optionally substituted with phenyl or methoxy,
(3) (C=O)phenyl, wherein phenyl is optionally substituted with bromide or methoxy,
(4) (C₁-C₆)-alkyl, wherein alkyl is optionally substituted with phenyl, methoxy, hydroxy, OCH₂OCH₃, benzylSO₃, phenylSO₃, or carboxymethyl,
(5) (C₂-C₆)-alkenyl,
(6) (C=O)O-phenyl, wherein phenyl is optionally substituted with nitro,
(7) (C=O)O(C₂-C₆)-alkenyl,
(8) (C=O)(C₁-C₃)-alkyl, wherein alkyl is optionally substituted with phenyl,
(9) (C=O)(C₂-C₄)-alkenyl,
(10) phenyl,
(11) SO₂-phenyl,
(12) SO₂-benzyl,
(13) CH₂(CO)CH₃,
(14) CH₂(CO)NH-benzyl,
(15) CH₂(CO)NH-allyl,
(16) CH₂(CO)N(CH₃)₂,
(17) CH₂(CO)NH(CH₃),
(18) 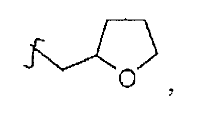
(19) 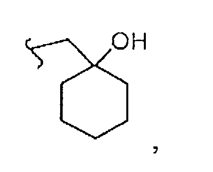
(20) 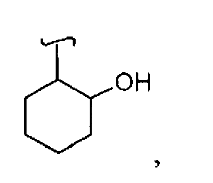
(21) 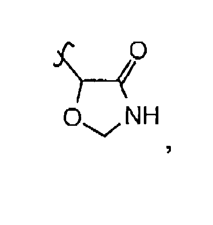
(22) 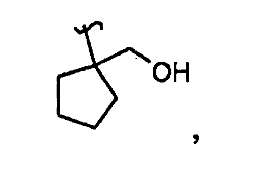
(23) 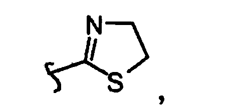
(24) CH₂CH₂NHCO₂(C₁-C₃)alkyl,
(25) CH₂CH₂O(CO)NHCH₃,
(26) CH₂CH₂O(CO)NH-allyl,
(27) CH₂CH₂NH(SO₂)CH₃,
(28) CH₂CH₂NH₂,
(29) CH₂CH₂NH(CO)CH₂CH₃, and
(30) benzyl;
R¹⁰ is:
(1) hydrogen,
(2) (C=O)phenyl, wherein phenyl is unsubstituted or substituted with F, Cl, Br, or I, or
(3) (C₁-C₃)-alkyl.

6. A method in accordance with claim 1 or 2 wherein the potassium channel blocker is a compound of structural formula III: 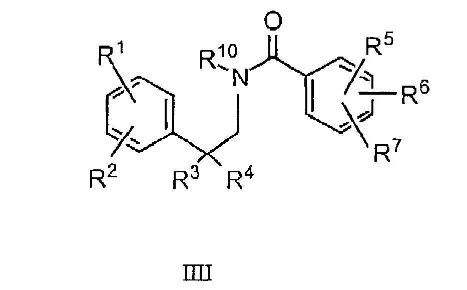 or a pharmaceutically acceptable salt, crystal form, or hydrate, wherein:
n is: 0, 1, 2 or 3;
r is: 0 or 1;
s 1s: 0 or 1 ;
R ¹, R², R⁵, R⁶ and R⁷ are independently:
(1) halo, wherein halo is fluoro, chloro, bromo, or iodo,
(2) hydroxy,
(3) (C₁-C₆)-alkyl,
(4) HO(C₁-C₆)-alkyloxy,
(5) (C₁-C₄)-perfluoroalkyl,
(6) (C₂-C₆)-alkenyl,
(7) (C₂-C₆)-alkynyl,
(8) O[(C=O)Oᵣ]ₛ(C₁-C₆)-alkyl, wherein the alkyl may be cyclic or straight-chained,
(9) (C₁-C₆)-alkyl-S(O)ₙ-,
(10) phenyl,
(11) phenoxy,
(12) cyano,
(13) nitro,
(14) CO₂H,
(15) CO(C₁-C₆)-alkyl,
(16) CO₂(C₁-C₆)-alkyl,
(17) CONR⁸R⁹,
(18) NR⁸R⁹,
(20) (C₂-C₆)-alkenyloxy,
(21) benzyloxy,
(22) hydrogen,
(23) OCF₃, or
(24) R¹ and R² or R⁶ and R⁷ can an be taken together when on adjacent carbons to form a fused benzo, dihydrofuranyl, furanyl, pyrrolidyl, dihydropyrrolidyl or 1,3-dioxolan group;
R³ and R⁴ are independently:
(1) halo, wherein halo is fluoro, chloro, bromo, or iodo,
(2) hydroxy,
(3) HO(C₁-C₆)-alkyloxy,
(4) (C₁-C₄)-perfluoroalkyl,
(5) O(CO)CCl₃,
(6) (C₁-C₆)-alkyl-S(O)ₙ-,
(7) phenyl-(CH₂)ᵣ-S(O)ₙ-,
(8) cyano,
(9) nitro,
(10) CO₂H,
(11) CO(C₁-C₆)-alkyl,
(12) CO₂(C₁-C₆)-alkyl,
(13) CONR⁸R⁹,
(14) NR⁸R⁹,
(15) O(CO)NR⁸R⁹,
(16) azido,
(17) NR⁸(CO)NR⁸R⁹,
(18) hydrogen,
(19) (C₁-C₁₀)-alkyl, wherein alkyl includes cyclic as well as acyclic groups and is unsubstituted or substituted with one or two of the substituents selected from the group consisting of:
(a) halo, wherein halo is fluoro, chloro, bromo, or iodo,
(b) hydroxy,
(c) oxo,
(d) O[(C=O)Oᵣ]ₛ(C₁-C₆)-alkyl,
(e) (C₁-C₆)-alkyl-S(O)ₙ-,
(f) aryl-(C₁-C₆)-alkyloxy,
(g) cyano,
(h) nitro,
(i) vinyl,
(j) NR⁸R⁹,
(k) O(CO)NR⁸R⁹,
(l) CHO,
(m) CO₂H,
(n) CO(C₁-C₆)-alkyl,
(o) CO₂(C₁-C₆)-alkyl, wherein alkyl may be substituted with phenyl,
(p) CONR⁸R⁹,
(q) aryl, wherein aryl is defined as phenyl or naphthyl, unsubstituted or substituted with one, two or three of the substituents selected from the group consisting of:
(a') halo, as defined above,
(b') hydroxy,
(c') (C₁-C₆)-alkyl,
(d') (C1-C4)-perfluoroalkyl,
(e') (C₂-C₆)-alkenyl,
(f') (C₂-C₆)-alkynyl,
(g') (C₁-C₆)-alkyloxy,
(h') (C₁-C₆)-alkyl-S(O)ₙ-,
(i') phenyl,
(j') phenoxy,
(k') cyano,
(l') nitro,
(m') CO₂H,
(n') CO(C₁-C₆)-alkyl,
(o') CO₂(C₁-C₆)-alkyl,
(p') CONR⁸R⁹,
(q') NR⁸R⁹, and
(r) heteroaryl, wherein heteroaryl is defined as an unsubstituted, monosubstituted, or disubstituted five or six membered aromatic heterocycle containing from 1 to 3 heteroatoms selected from the group consisting of O, N and S and wherein the substituents are members selected from the group consisting of:
(a') halo, as defined above,
(b') hydroxy,
(c') (C₁-C₆)-alkyl,
(d') (C1-C4)-perfluoroalkyl,
(e') (C₂-C₆)-alkenyl,
(f') (C₂-C₆)-alkynyl,
(g') (C₁-C₆)-alkyloxy,
(h') (C₁-C₆)-alkyl-S(O)ₙ-,
(i') phenyl,
(j') phenoxy,
(k') cyano,
(l') nitro,
(m') CO₂H,
(n') CO(C₁-C₆)-alkyl,
(o') CO₂(C₁-C₆)-alkyl,
(p') CONR⁸R⁹,
(q') NR⁸R⁹, and
(r') fused benzo or pyridyl group,
(s) heterocyclyl, wherein heterocyclyl is defined as a 3 to 7 atom cyclic, non-aromatic substituent containing from 1 to 3 heteroatoms selected from the group consisting of O, N, and S, said heterocycle being unsubstituted or substituted with one, two or three substituents selected from the group consisting of:
(a') halo, as defined above,
(b') hydroxy,
(c') (C₁-C₆)-alkyl,
(d') (C1-C4)-perfluoroalkyl,
(e') (C₂-C₆)-alkenyl,
(f') (C₂-C₆)-alkynyl,
(g') (C₁-C₆)-alkyloxy,
(h') (C₁-C₆)-alkyl-S(O)ₙ,
(i') phenyl,
(j') phenoxy,
(k') cyano,
(l') nitro,
(m') CO₂H,
(n') CO(C₁-C₆)-alkyl,
(o') CO₂(C₁-C₆)-alkyl,
(p') CONR⁸R⁹,
(q') NR⁸R⁹,
(r') NR⁸CO(C₁-C₆)-alkyl,
(s') oxo,
(t') fused benzo, and
(u') fused pyridyl group;
(t) benzyl-S(O)ₙ-,
(u) O[(C=O)Oᵣ]ₛ(C₂-C₆)-alkenyl,
(v) O[(C=O)Oᵣ]ₛaryl,
(w) O[(O=O)Oᵣ]ₛheteroayl,
(x) O(CH₂)ₙheteroaryl, or
(y) O(CH₂)ₙaryl;
(20) (C₂-C₁₀)-alkenyl, wherein alkenyl is unsubstituted or substituted with one or two of the substituents selected from the group consisting of:
(a) halo, wherein halo is fluoro, chloro, bromo, or iodo,
(b) hydroxy,
(c) oxo,
(e) (C₁-C₆)-alkyl-S(O)ₙ-,
(f) phenyl-(C₁-C₆)-alkyloxy,
(g) cyano,
(h) nitro,
(i) NR⁸R⁹,
(j) CHO,
(k) CO₂H,
(l) CO(C₁-C₆)-alkyl,
(m) CO₂(C₁-C₆)-alkyl,
(n) CONR⁸R⁹,
(o) aryl, wherein aryl is as defined above,
(p) heteroaryl, wherein heteroaryl is as defined above,
(q) heterocyclyl, wherein heterocyclyl is as defined above,
(r) O[(C=O)Oᵣ]ₛ(C₁,C₆)-alkyl, alkyl as defined above,
(s) O[(C=O)Oᵣ]ₛ(C₂-C₆)-alkenyl, as defined above,
(t) O[(C=O)Oᵣ]ₛaryl, aryl as defined above,
(u) O[(C=O)Oᵣ]ₛheteroaryl, heteroaryl as defined above,
(v) O(CH₂)ₙheteroaryl, heteroaryl as defined above, and
(w) O(CH₂)ₙaryl, aryl as defined above;
(21) (C₂-C₁₀)-alkynyl, wherein alkynyl is unsubstituted or substituted with one or two of the substituents selected from the group consisting of:
(a) halo, wherein halo is fluoro, chloro, bromo, or iodo,
(b) hydroxy,
(c) oxo,
(d) (C₁-C₆)-alkyloxy,
(e) (C₁-C₆)-S(O)ₙ-,
(f) phenyl-(C₁-C₆)-alkyloxy,
(g) cyano,
(h) nitro,
(i) vinyl,
(j) NR⁸R⁹,
(k) NR⁸CO(C₁-C₆)-alkyl,
(l) CHO,
(m) CO₂H,
(n) CO(C₁-C₆)-alkyl,
(o) CO₂C(C₁-C₆)-alkyl,
(p) CONR⁸R⁹,
(q) aryl, wherein aryl is as defined above,
(r) heteroaryl, wherein heteroaryl is as defined above,
(s) heterocyclyl, wherein heterocyclyl is as defined above,
(t) O[(C=-O)Oᵣ]ₛ(C₁-C₆)-alkyl, alkyl as defined above,
(u) O[(C=O)Oᵣ]ₛ(C₁-C₆)-alkenyl, as defined above,
(v) O[(C=O)Oᵣ]ₛaryl, aryl as defined above,
(w) O[(C=O)Qᵣ]ₛheteroaryl, heteroaryl as defined above
(x) O(CH₂)ₙheteroaryl, heteroaryl as defined above, and
(y) O(CH₂)ₙaryl, aryl as defined above.
(22) O[(C=O)Oᵣ]ₛ(C₁-C₆)-alkyl, alkyl as defined above,
(23) O[(C=O)Oᵣ]ₛ(C₂-C₆)-alkenyl, as defined above,
(24) O[(C=O)Oᵣ]ₛaryl, aryl as defined above,
(25) O[(C=O)Oᵣ]ₛheteroaryl, heteroaryl as defined above
(26) O(CH₂)ₙheteroaryl, heteroaryl as defined above,
(27) aryl, wherein aryl is as defined above or
(28) O(CH₂)ₙaryl, aryl as defined above;
R³ can also be any of the following when R⁴ is absent:
(29) oxo,
(30) =CH-(C₁-C₆)-alkyl, wherein alkyl is as defined above,
(31) =CH-(C₂-C₆)-alkenyl, wherein alkenyl is as defined above,
(32) =CH-aryl, wherein aryl is as defined above, or
(33) =CH₂;
R⁸ and R⁹ are independently selected from the group consisting of:
(1) hydrogen,
(2) [(C=O)Oᵣ]ₛaryl, wherein aryl is as defined above,
(3) [(C=O)Oᵣ]ₛ(C₂-C₈)-alkenyl, wherein alkenyl is as defined above,
(4) [(C=O)Oᵣ]ₛ(C₁-C₈)-alkyl, wherein alkyl is as defined above,
(5) (C=O)ᵣS(O)ₙ(C₁-C₈)-alkyl, wherein alkyl is as defined above,
(6) (C=O)ᵣS(O)ₙ-aryl, wherein aryl is as defined above, and
(7) heterocyclyl, wherein heterocyclyl is defined above;
R¹⁰ is ;
(1) hydrogen,
(2) [(C=O)Oᵣ]ₛaryl, wherein aryl is as defined above, or
(3) [(C=O)Oᵣ]ₛ(C₁-C6)-alkyl, wherein alkyl is as defined above.

7. A method according to claim 6 wherein R¹,R²,R⁵,R⁶ and R⁷ of formula III are independently:
(1) halo, wherein halo is fluoro, chloro, bromo, or iodo,
(2) hydroxy,
(3) (C₁-C₃)-alkenyl,
(4) (C₂-C₃)-alkenyl,
(5) O(C₁-C₄)-alkyl, wherein the alkyl may be cyclic or straight-chained,
(6) O(CO)CH₃,
(7) CO(C₁-C₃)-alkyl,
(8) CO₂(C₁-C₃)-alkyl,
(9) hydrogen,
(10) R¹ and R² or R⁶ and R⁷ can an be taken together when on adjacent carbons to form a fused benzo, dihydrofuranyl, furanyl, pyrrolidyl, dihydropyrrolidyl or 1,3-dioxolan group;
R³ and R⁴ are independently:
(1) hydrogen,
(2) (C₁-C₆)-alkyl, wherein alkyl includes cyclic as well as acyclic groups and is unsubstituted or substituted with one or two of the substituents select from the group consisting of :
(a) halo, wherein halo is chloro,bromo, or iodo,
(b) hydroxy,
(c) oxo,
(d) O[(C=O)Oᵣ]ₛ(C₁-C₆)-alkyl, wherein r and s are independently 0 or 1,
(e) CO₂(C₂-C₃)-alkenyl,
(f) O[(C=O)Oᵣ]ₛ(C₁-C₆)-alkenyl, wherein r and s are independently 0 or 1,
(g) NR⁸R⁹,
(h) O(CO)NR⁸R⁹,
(i) CHO,
(j) CO₂H,
(k) CO(C₁-C₆)-alkyl,
(l) CO₂(C₁-C₆)-alkyl, wherein alkyl may be substituted with phenyl,
(m) CONR⁸R⁹,
(n) aryl, wherein aryl is defined as phenyl, unsubstituted or substituted with a substituent selected from the group consisting of:
(a') halo, as defined above,
(b') hydroxy,
(c') (C₁-C₆)-alkyl, and
(d') (C₁-C₆)-alkyloxy, and
(o) O[(C=O)Oᵣ]ₛ(C₂-C₆)-alkenyl, wherein r and s are independently 0 or 1,
(3) (C₂-C₆)-alkenyl,
(4) aryl, wherein aryl is as defined above or
(5) O(CH₂)ₙaryl, wherein aryl is as defined above;
R³ can also be any of the following when R⁴ is absent:
(6) =CH-(C₁-C₆)-alkyl, wherein alkyl is as defined above,
(7) =CH-(C₂-C₆)-alkenyl, wherein alkenyl is as defined above,
R⁸ and R⁹ are independently selected from the group consisting of:
(1) hydrogen,
(2) [(C=O)Oᵣ]ₛaryl, wherein aryl is as defined above and r and s are independently 0 or 1,
(3) (C₂-C₈)-alkenyl, wherein alkenyl is as defined above,and
(4) (C₁-C₆)-alkyl, wherein alkyl is as defined above, and
R¹⁰ is:
(1) hydrogen, or
(2) (C=O)(C₁-C₃)-alkyl, wherein alkyl is as defined above.

8. A method according to claim 1 or 2 wherein the potassium channel blocker is:
trans 1-(N-ethylcarbamoyloxy)-4-phenyl-4-(3-(2-methoxyphenyl)-3-oxo-2-azaprop1-lyl)cyclohexane, trans 1-(N-allylcarbamoyloxy)-4-phenyl-4-(3-(2-hydroxy-5-fluorophenyl)-3-oxo-2-azaprop-1-yl)cyclohexane, trans 1-(N-n-propylcarbamoyloxy)-4-phenyl-4-(3-(2-methoxyphenyl)-3-oxo-2-azaprop-1-yl)cyclohexane, trans 1-(N-methylcarbamoyloxy)-4-phenyl-4-(3-(2-methoxyphenyl)-3-oxo-2-azaprop-1-yl)cyclohexane, trans 1-(N-allylcarbamoyloxy)-4-phenyl-4-(3-(2-methoxyphenyl)-3-oxo-2-azapropyl)cyclohexane, 1-(2-methoxyphenyl)-1-oxo-2-aza-(S)-4-i-butyl-4-phenylbutane, 1-(2-methoxyphenyl)-1-oxo-2-aza-4-(S)-((3-allyloxycarbonyloxy)propyl))-4-phenylbutane, 1-(2-methoxyphenyl)-1-oxo-2-aza-4,4-diethyl-4-phenylbutane, 1-(2,3-dihydrobenzofuran-7-yl)-1-oxo-2-aza-4,4-diethyl-4-(phenyl)-butane, and 1-(2-methoxyphenyl)-1-oxo-2-aza-4-(S)-(3-hydroxypropyl)-4-phenylbutane.

9. A method according to claim 1 or 2 wherein the potassium channel blocker is: 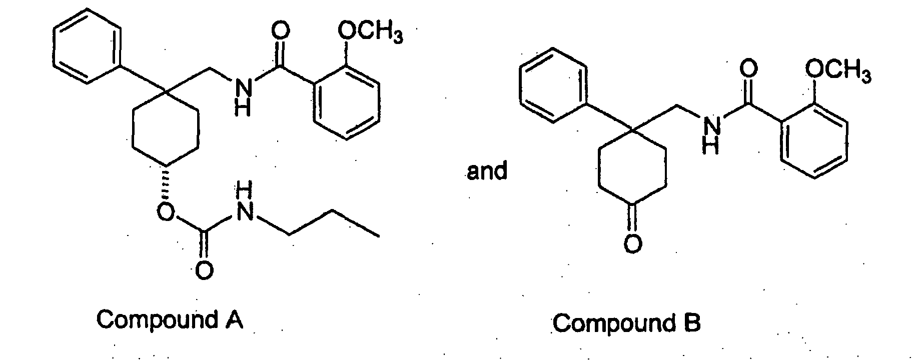 or a pharmaceutically acceptable salt, ester, diastereomer or enantiomer thereof.

## Patentansprüche

1. Verfahren zum Identifizieren von Maxi-K-Kanalblockern, die zur Absenkung des intraokularen Druckes (1) unter Anwendung von HEK-293-Zellen verwendbar sind, welches Verfahren die Schritte umfasst:
(a) Aufbauen von stabilen HEK-293-Zell-Linien, die sowohl alpha- als auch beta-1-Subeinheiten des Maxi-K-Kanals exprimieren;
(b) Inkubieren der HEK-293-Zellen mit einem Kalium-Kanalblocker, welche selektiv die endogene Kalium-Leitfähigkeit von HEK-293-Zellen eliminiert, ohne die Maxi-K-Kanal-Aktivität zu beeinträchtigen (2)
(c) Beladen der inkubierten HEK-K-293-Zellen mit einer Testverbindung und Inkubieren sowie
(d) Messen der Maxi-K-Kanalblockeraktivität der Testverbindung unter Verwendung eines Spannung/Ionensonden-Leseinstrumentes und auf Fluoreszenz-Resonanzenergietransfer(3)-basierenden Membranpotentials, indem Farbstoffe zur Beobachtung des Membranpotentials erfasst werden.

2. Verfahren nach Anspruch 1, welches Verfahren die Schritte umfasst:
(a) Anlegen einer Plattenkultur der HEK-293-Zellen, 2E+06-Zellen/ml, auf 96er Mikrotiter-Poly-D-Lysin-Platten mit einer Dichte von etwa 100.000 Zellen/Mulde;
(b) Inkubieren der Platten für 16 bis 24 Stunden;
(c) Absaugen des Mediums von den Zellen;
(d) Waschen der Zellen einmal mit Dulbecco's Phosphat-gepufferter Salzlösung;
(e) Zugeben von 100 µl von 9 µM-Cumarin-Farbstoff-0,02% Pluronic-127 in Dulbecco's Phosphat-gepufferter Salzlösung (4) pro Mulde;
(f) Inkubieren bei Dunkelheit für etwa 30 Minuten;
(g) Waschen der Zellen zweimal mit Dulbecco's Phosphat-gepufferter Salzlösung;
(h) Zugeben von 100 µl 4,5 µM Oxanol-Farbstoff in (mM): 140 NaCl, 0,1 KCl, 2 CaCl₂, 1 MgCl₂, 20 Hepes-NaOH, pH-Wert bei etwa 7,4, 10 Glucose pro Mulde;
(i) Zugeben eines Inhibitors der endogenen Kalium-Leitfähigkeit von HEK-293-Zellen;
(j) Zugeben einer Testverbindung;
(k) Inkubieren bei Raumtemperatur für etwa 30 Minuten;
(l) Laden der Platten auf einem Spannung/Ionen-Sondenleser und Aufzeichnen der Fluoreszenzemission beider Farbstoffe für etwa 10 Sekunden;
(m) Zugeben von 100 µl kaliumreicher Lösung in (mM): 140 KCl, 2 CaCl₂, 1 MgCl₂, 20 Hepes-KOH, pH-Wert bei etwa 7,4, 10 Glucose; und Aufzeichnen der Fluoreszenzemission beider Farbstoffe für etwa 10 Sekunden und Bestimmen des Membranpotentials dieser Daten unter Anwendung eines Spannung/Ionensonden-Lesegeräts.

3. Verfahren nach Anspruch 1 oder 2, bei welchem der Kalium-Kanalblocker eine Verbindung mit der Strukturformel I ist: 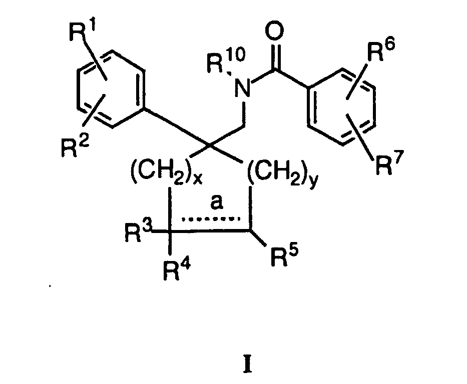 oder ein pharmazeutisch duldbares Salz, eine Kristallform davon oder ein Hydrat, worin:
a eine Einfachbindung oder eine Doppelbindung ist, wenn R⁴ nicht vorhanden ist und in der vorgenannten Struktur dargestellt wird durch = unter der Voraussetzung, dass a eine Einfachbindung ist, wenn x + y = 0 gilt;
n beträgt 0, 1, 2 oder 3;
r beträgt 0 oder 1;
s beträgt 0 oder 1;
x oder y sind unabhängig 0, 1 oder 2;
R¹, R², R⁶ und R⁷ sind unabhängig:
(1) Halogen, worin Halogen Fluor, Chlor, Brom oder Iod ist,
(2) Hydroxy,
(3) (C₁-C₆)-Alkyl,
(4) HO(C₁-C₆)-Alkyloxy,
(5) (C₁-C₄)-Perfluoralkyl,
(6) (C₂-C₆)-Alkenyl,
(7) (C₂-C₆)-Alkinyl,
(8) O[C=O)Oᵣ]ₛ(C₁-C₆)-Alkyl,
(9) (C₁-C₆)-Alkyl-S(O)ₙ⁻,
(10) -(O)ᵣ(C₀-C₆)-Alkylaryl, worin Aryl Phenyl ist oder Naphthyl ist, unsubstituiert ist oder substituiert ist mit bis zu drei Substituenten, die ausgewählt sind aus (C₁-C₃)-Alkyl, Trifluormethyl und Halogen;
(11) -(O)ᵣ-Heteroaryl, worin das Heteroaryl Pyridinyl oder Pyrryl ist,
(12) Cyano,
(13) Nitro,
(14) CO₂H,
(15) CO(C₁-C₆)-Alkyl,
(16) CO₂(C₁-C₆)-Alkyl,
(17) CONR⁸R⁹,
(18) NR⁸R⁹,
(20) (C₂-C₆)-Alkenyloxy,
(21) (CO)-Aryl, worin Aryl Phenyl, Naphthyl, Benzothienyl ist oder ein Benzophenon-Rest und unsubstituiert ist oder substituiert ist mit bis zu zwei Substituenten, die ausgewählt sind aus Halogen, Trifluormethyl und (C₁-C₃)-Alkyl,
(22) Wasserstoff,
(23) OCF₃,
(24) -(CH₂)-O-N=C(CH₃)(Aryl), worin Aryl Phenyl ist oder Naphthyl ist und unsubstituiert ist oder substituiert ist mit bis zu drei Halogen-Substituenten;
(25) -S(O)ₙ-N R⁸R⁹ oder
(26) R¹ und R² oder R⁶ und R⁷ können zusammengenommen werden, sofern an angrenzenden Kohlenstoffen, um eine kondensierte Benzo-Dihydrofuranyl-, Furanyl-, Pyrrolidyl-, Dihydropyrrolidyl- oder 1,3-Dioxolan-Gruppe zu bilden;
R³ und R⁴ sind unabhängig:
(1) Halogen, worin Halogen Fluor, Chlor, Brom oder Iod ist,
(2) Hydroxy, wenn a eine Einfachbindung ist,
(3) HO(C₁-C₆)-Alkyloxy,
(4) (C₁-C₄)-Perfluoralkyl,
(5) O(CO)CCl₃,
(6) (C₁-C₆)-Alkyl-S(O)ₙ-,
(7) Phenyl-(CH₂)ᵣ-S(O)ₙ-,
(8) Cyano,
(9) Nitro,
(10) CO₂H,
(11) CO(C₁-C₆)-Alkyl,
(12) CO₂(C₁-C₆)-Alkyl,
(13) CONR⁸R⁹,
(14) NR⁸R⁹,
(15) O(CO)NR⁸R⁹,
(16) Azido,
(17) NR⁸(CO)NR⁸R⁹,
(18) Wasserstoff
(19) (C₁-C₁₀)-Alkyl, worin Alkyl cyclische sowie acyclische Gruppen einschließt und unsubstituiert ist oder substituiert ist mit einem oder zwei der Substituenten, die ausgewählt sind aus der Gruppe, bestehend aus:
(a) Halogen, worin Halogen Fluor, Chlor, Brom oder Iod ist,
(b) Hydroxy,
(c) Oxo,
(d) O[(C=O)Oᵣ]ₛ(C₁-C₆)-Alkyl,
(e) (C₁-C₆)-Alkyl-S(O)ₙ-,
(f) Aryl-(C₁-C₆)-Alkyloxy,
(g) Cyano,
(h) Nitro,
(i) Vinyl,
(j) NR⁸R⁹,
(k) O(CO)NR⁸R⁹,
(l) CHO,
(m) CO₂H,
(n) CO(C₁-C₆)-Alkyl,
(o) CO₂(C₁-C₆)-Alkyl,
(p) CONR⁸R⁹,
(q) Aryl, wenn Aryl festgelegt ist als Phenyl oder Naphthyl, unsubstituiert oder substituiert mit einem, zwei oder drei der Substituenten, die ausgewählt sind aus der Gruppe, bestehend aus:
(a') Halogen, wie vorstehend festgelegt,
(b') Hydroxy,
(c') (C₁-C₆)-Alkyl,
(d') (C₁-C₄)-Perfluoralkyl,
(e') (C₂-C₆)-Alkenyl,
(f') (C₂-C₆)-Alkinyl,
(g') (C₁-C₆)-Alkyloxy,
(h') (C₁-C₆)Alkyl-S(O)ₙ-,
(i') Phenyl,
(j') Phenoxy,
(k') Cyano,
(l') Nitro,
(m') CO₂H,
(n') CO(C₁-C₆)-Alkyl,
(o') CO₂(C₁-C₆)-Alkyl,
(p') CONR⁸R⁹ und
(q') NR⁸R⁹,
(r) Heteroaryl, worin Heteroaryl festgelegt ist als ein unsubstituierter, monosubstituierter oder disubstituierter, fünf- oder sechsgliedriger aromatischer Heterocyclus, der 1 bis 3 Heteroatome enthält, die ausgewählt sind aus der Gruppe, bestehend aus O, N und S, und worin die Substituenten Vertreter sind, die ausgewählt sind aus der Gruppe, bestehend aus:
(a') Halogen, wie vorstehend festgelegt,
(b') Hydroxy,
(c') (C₁-C₆)-Alkyl,
(d') (C₁-C₄)-Perfluoralkyl,
(e') (C₂-C₆)-Alkenyl,
(f') (C₂-C₆)-Alkinyl,
(g') (C₁-C₆)-Alkyloxy,
(h') (C₁-C₆)Alkyl-S(O)ₙ-,
(i') Phenyl,
(j') Phenoxy,
(k') Cyano,
(l') Nitro,
(m') CO₂H,
(n') CO(C₁-C₆)-Alkyl,
(o') CO₂(C₁-C₆)-Alkyl,
(p') CONR⁸R⁹ und
(q') NR⁸R⁹ und
(r') kondensierte Benzo- oder Pyridyl-Gruppe,
(s) Heterocyclyl, worin Heterocyclyl festgelegt ist als ein cyclischer, nichtaromatischer Substituent mit 3 bis 7 Atomen, der 1 bis 3 Heteroatome enthält, die ausgewählt sind aus der Gruppe, bestehend aus O, N und S, wobei der Heterocyclus unsubstituiert ist oder substituiert ist mit einem, zwei oder drei Substituenten, die ausgewählt sind aus der Gruppe, bestehend aus:
(a') Halogen, wie vorstehend festgelegt,
(b') Hydroxy,
(c') (C₁-C₆)-Alkyl,
(d') (C₁-C₄)-Perfluoralkyl,
(e') (C₂-C₆)-Alkenyl,
(f') (C₂-C₆)-Alkinyl,
(g') (C₁-C₆)-Alkyloxy,
(h') (C₁-C₆)Alkyl-S(O)ₙ-,
(i') Phenyl,
(j') Phenoxy,
(k') Cyano,
(l') Nitro,
(m') CO₂H,
(n') CO(C₁-C₆)-Alkyl,
(o') CO₂(C₁-C₆)-Alkyl,
(p') CONR⁸R⁹ und
(q') NR⁸R⁹,
(r') NR⁸CO(C₁-C₆)-Alkyl,
(s') Oxo,
(t') kondensierte Benzo- und
(u') kondensierte Pyridyl-Gruppe;
(t) Benzyl-S(O)ₙ-,
(u) O[(C=O)Oᵣ]ₛ(C₁-C₆)-Alkenyl,
(v) O[(C=O)Oᵣ]ₛ-Aryl,
(w) O[(C=O)Oᵣ]ₛ-Heteroaryl,
(x) O(CH₂)ₙ-Heteroaryl oder
(y) O(CH₂)ₙ-Aryl;
(20) (C₂-C₁₀)-Alkenyl, worin Alkenyl unsubstituiert ist oder substituiert ist mit einem oder zwei der Substituenten, die ausgewählt sind aus der Gruppe, bestehend aus:
(a) Halogen, worin Halogen Fluor, Chlor, Brom oder Iod ist,
(b) Hydroxy,
(c) Oxo,
(e) (C₁-C₆)-Alkyl-S(O)ₙ-,
(f) Phenyl-(C₁-C₆)-Alkyloxy,
(g) Cyano,
(h) Nitro,
(i) NR⁸R⁹,
(j) CHO,
(k) CO₂H,
(l) CO(C₁-C₆)-Alkyl,
(m) CO₂(C₁-C₆)-Alkyl,
(n) CONR⁸R⁹,
(o) Aryl, worin Aryl wie vorstehend festgelegt ist,
(p) Heteroaryl, worin Heteroaryl wie vorstehend festgelegt ist,
(q) Heterocyclyl, worin Heterocyclyl wie vorstehend festgelegt ist,
(r) O[(C=O)Oᵣ]ₛ(C₁-C₆)-Alkyl, worin Alkyl wie vorstehend festgelegt ist,
(s) O[(C=O)Oᵣ]ₛ(C₂-C₆)-Alkenyl, worin Alkenyl wie vorstehend festgelegt ist,
(t) O[(C=O)Oᵣ]ₛ(C₁-C₆)-Aryl, worin Aryl wie vorstehend festgelegt ist,
(u) O[(C=O)Oᵣ]ₛ-Heteroaryl, worin Heteroaryl wie vorstehend festgelegt ist,
(v) ((CH₂)ₙ-Heteroaryl, worin Heteroaryl wie vorstehend festgelegt ist,
(w) O(CH₂)ₙ-Aryl, worin Aryl wie vorstehend festgelegt ist;
(21) (C₂-C₁₀)-Alkinyl, worin Alkinyl unsubstituiert ist oder substituiert ist mit einem oder zwei der Substituenten, die ausgewählt sind aus der Gruppe, bestehend aus:
(a) Halogen, worin Halogen Fluor, Chlor, Brom oder Iod ist,
(b) Hydroxy,
(c) Oxo,
(d) (C₁-C₆)-Alkyloxy,
(e) (C₁-C₆)-S(O)ₙ-,
(f) Phenyl-(C₁-C₆)-Alkyloxy,
(g) Cyano,
(h) Nitro,
(i) Vinyl,
(j) NR⁸R⁹,
(k) NR⁸CO(C₁C₆)-Alkyl,
(l) CHO,
(m) CO₂H,
(n) CO(C₁-C₆)-Alkyl,
(o) CO₂C(C₁-C₆)-Alkyl,
(p) CONR⁸R⁹,
(q) Aryl, worin Aryl wie vorstehend festgelegt ist,
(r) Heteroaryl, worin Heteroaryl wie vorstehend festgelegt ist,
(s) Heterocyclyl, worin Heterocyclyl wie vorstehend festgelegt ist,
(t) O[(C=O)Oᵣ]ₛ(C₁-C₆)-Alkyl, worin Alkyl wie vorstehend festgelegt ist,
(u) O[(C=O)Oᵣ]ₛ(C2-C6)-Alkenyl, worin Alkenyl wie vorstehend festgelegt ist,
(v) O[(C=O)Oᵣ]ₛ-Aryl, worin Aryl wie vorstehend festgelegt ist,
(w) O[(C=O)Oᵣ]ₛ-Heteroaryl, worin Heteroaryl wie vorstehend festgelegt ist,
(x) O(CH₂)ₙ-Heteroaryl, worin Heteroaryl wie vorstehend festgelegt ist,
(y) O(CH₂)ₙ-Aryl, worin Aryl wie vorstehend festgelegt ist,
(22) O[(C=O)Oᵣ]ₛ(C₁-C₆)-Alkyl, worin Alkyl wie vorstehend festgelegt ist,
(23) O[(C=O)Oᵣ]ₛ(C₂-C₆)-Alkenyl, worin Alkenyl wie vorstehend festgelegt ist,
(24) O[(C=O)Oᵣ]ₛ-Aryl, worin Aryl wie vorstehend festgelegt ist,
(25) O[(C=O)Oᵣ]ₛ-Heteroaryl, worin Heteroaryl wie vorstehend festgelegt ist,
(26) O(CH₂)ₙ-Heteroaryl, worin Heteroaryl wie vorstehend festgelegt ist,
(27) Aryl, worin Aryl wie vorstehend festgelegt ist,
(28) O[(C=O)Oᵣ]ₛ-Heteroaryl, worin Heteroaryl wie vorstehend festgelegt ist,
(29) O(CO)NH(CH₂-CO-NR⁸R⁹) oder
(30) O(CH₂)ₙ-Aryl, worin Aryl wie vorstehend festgelegt ist;
R³ kann ebenfalls jeder beliebige der Folgenden sein, wenn a eine Einfachbindung ist und R⁴ nicht vorhanden ist;
(31) Oxo,
(32) =CH-(C₁-C₆)-Alkyl, worin Alkyl wie vorstehend festgelegt ist,
(33) =CH-(C₂-C₆)-Alkenyl, worin Alkenyl wie vorstehend festgelegt ist,
(34) =CH-Aryl, worin Aryl wie vorstehend festgelegt ist,
(35) =CH₂ oder
R³ und R⁴ können zusammengenommen eine spiro-kondensierte Heterocyclyl-Gruppe bilden, worin Heterocyclyl wie vorstehend festgelegt ist, oder
R³ und R⁵ können zusammengenommen ein kondensiertes Oxiran bilden, wenn a eine Einfachbindung ist unter der Voraussetzung, dass R⁴ nicht vorhanden ist, wenn a eine Doppelbindung ist;
R⁵ ist:
(1) Wasserstoff,
(2) Halogen,
(3) (C₂-C₆)-Alkenyl,
(4) Hydroxy,
(5) O[(C=O)Oᵣ]ₛ(C₁-C₆)-Alkyl,
(6) O(CO)NR⁸R⁹,
(7) Oxo, wenn a eine Einfachbindung ist, oder
R⁵ und R³ können zusammengenommen ein kondensiertes Oxiran bilden, wenn a eine Einfachbindung ist;
R⁸ und R⁹ sind unabhängig ausgewählt aus der Gruppe, bestehend aus:
(1) Wasserstoff,
(2) [(C=O)Oᵣ]ₛ-Aryl, worin Aryl wie vorstehend festgelegt ist,
(3) [(C=O)Oᵣ]ₛ(C₂-C₈)-Alkenyl, worin Alkenyl wie vorstehend festgelegt ist,
(4) [(C=O)Oᵣ]ₛ(C₁-C₈)-Alkyl, worin Alkyl wie vorstehend festgelegt ist,
(5) (C=O)ᵣS(O)ₙ(C₁-C₈)-Alkyl, worin Alkyl wie vorstehend festgelegt ist,
(6) (C=O)ᵣS(O)ₙ-Aryl, worin Aryl wie vorstehend festgelegt ist und
(7) Heterocyclyl, worin Heterocyclyl wie vorstehend festgelegt ist;
R¹⁰ ist:
(1) Wasserstoff,
(2) [(C=O)Oᵣ]ₛ-Aryl, worin Aryl wie vorstehend festgelegt ist oder
(3) [(C=O)Oᵣ]ₛ(C₁-C₆)-Alkyl, worin Alkyl wie vorstehend festgelegt ist.

4. Verfahren nach Anspruch 3, worin die Verbindung der Formel I ferner wie in der Verbindung von Formel II festgelegt ist, worin x 2 und y 1 betragen.

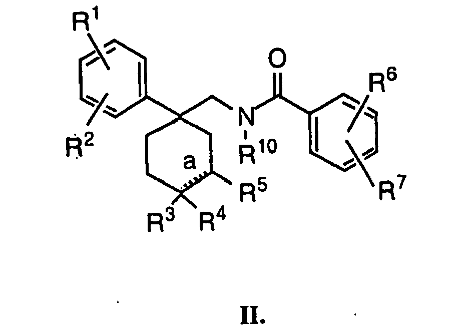

5. Verfahren nach Anspruch 4, worin a ferner festgelegt ist als eine Einfachbindung;
R¹, R², R⁶ und R⁷ sind unabhängig:
(1) Halogen, worin Halogen Fluor, Chlor, Brom oder Iod ist,
(2) Hydroxy,
(3) (C₁-C₆)-Alkyl,
(4) HO(C₁-C₆)-Alkyloxy,
(5) (C₁-C₆)-Alkyloxy, worin das Alkyl cyclisch ist oder geradkettig,
(6) Acetoxy,
(7) Nitro,
(8) NR⁸R⁹,
(9) -(O)ᵣ(C₀-C₃)-Aryl, worin Aryl Phenyl oder Napthyl ist, unsubstituiert ist oder substituiert ist mit bis zu drei Substituenten, die ausgewählt sind aus (C₁-C₃)-Alkyl, Trifluormethyl und Halogen,
(10) Wasserstoff,
(11) (O)ᵣCF₃,
(12) (C₁-C₆)-Alkyl-S(O)ₙ-, worin n 0, 1, 2 oder 3 beträgt,
(13) (CO₂)-(C₁-C₆)-Alkyl,
(14) -(O)ᵣ-Heteroaryl, worin Heteroaryl Pyridinyl oder Pyrryl ist,
(15) (CO)-Aryl, worin Aryl Phenyl ist, Naphthyl, Benzothienyl oder ein Benzophenon-Rest ist und unsubstituiert ist oder substituiert ist mit bis zu zwei Substituenten, die ausgewählt sind aus Halogen, Trifluormethyl und (C₁-C₃)-Alkyl,
(16) -(CH₂)-O-N-C(CH₃)(Aryl), worin Aryl Phenyl ist, unsubstituiert ist oder substituiert ist mit bis zu drei Halogen-Substituenten,
(17) -S(O)ₙ-N R⁸R⁹ oder
(18) R¹ und R² oder R⁶ und R⁷ können zusammengenommen werden, um ein kondensierte Benzo-, Dihydrofuranyl-, Furanyl-, Pyrrolidyl-, Dihydropyrrolidyl- oder 1,3-Dioxan-Gruppe zu bilden;
R³ und R⁴ sind unabhängig:
(1) Halogen, worin Halogen Fluor, Chlor, Brom oder Iod ist,
(2) Hydroxy,
(3) HO(C₁-C₆-Alkyloxy),
(4) (C=O)O(C₁-C₆)-Alkyl,
(5) O(CO)CCl₃,
(6) (C₁-C₆)-Alkyl-S(O)ₙ-, worin n 0, 1, 2 oder 3 beträgt,
(7) CH₂CO₂-(C₁-C₆)-Alkyl,
(8) Cyano,
(9) Benzyloxy,
(10) CH₂OAc,
(11) OAc,
(12) (C₂-C₆)-Alkenyl,
(13) (C₁-C₆)-Alkyl, worin Alkyl unsubstituiert sein kann oder substituiert ist mit Bromid,
(14) NR⁸R⁹,
(15) O(CO)NR⁸R⁹,
(16) Azido,
(17) NR⁸(CO)NR⁸R⁹,
(18) Wasserstoff
(19) CH₂OH,
(20) CH₂O(C=O)-Phenyl, worin Phenyl unsubstituiert ist oder monosubstituiert ist mit Methoxy,
(21) O(C₂-C₆)-Alkenyl,
(22) O(C=O)-Phenyl; worin Phenyl unsubstituiert ist oder monosubstituiert ist mit Bromid,
(23) O(C=O)O-Phenyl, worin Phenyl unsubstituiert ist oder monosubstituiert ist mit Nitro,
(24) CH₃(CO)NR⁸R⁹,
(25) O(C=O)O-(C₂-C₆)-Alkenyl,
(26) O(C=O)-(C₁-C₃)-Alkyl, worin das Alkyl unsubstituiert sein kann oder substituiert ist mit Bromid oder -CO₂CH₃,
(27) O(C₁-C₆)-Alkyl, worin Alkyl unsubstituiert sein kann oder substituiert ist mit Phenyl,
(28) O(C=O)O-(C₁-C₆)-Alkyl,
(29) CH₂O(CO)NR⁸R⁹ oder
(30) CH₂(C=O)O-(C₁-C₆)-Alkyl,
R³ kann jedes beliebige der Folgenden sein, wenn R⁴ nicht vorhanden ist:
(31) Oxo,
(32) =CH₂,
(33) =CH-CO₂-(C₁-C₆)-Alkyl,
(34) =CH-(CO)-NR⁸R⁹ oder
(35) =CH-CO₂H oder
R³ und R⁴ können zusammengenommen werden, um eine spiro-kondensierte Heterocyclyl-Gruppe zu bilden, worin Heterocyclyl festgelegt ist als:
(36) Oxiran,
(37) 1,3-Dioxolan,
(38) 2,2-Dimethyl-1,3-dioxolan oder
(39) Glykolsulfit oder
R³ und R⁵ können zusammengenommen werden, um ein kondensiertes Oxiran zu bilden;
R⁵ ist:
(1) Wasserstoff,
(2) Halogen
(3) (C₂-C₆)-Alkenyl,
(4) Hydroxy,
(5) O(C=O)(C₁-C₃)-Alkyl,
(6) O(CO)NR⁸R⁹,
(7) Oxo, wenn a eine Einfachbindung ist oder
R⁵ und R³ können zusammengenommen werden, um ein kondensiertes Oxiran zu bilden, wenn a eine Einfachbindung ist;
R⁸ und R⁹ sind unabhängig ausgewählt aus der Gruppe, bestehend aus:
(1) Wasserstoff,
(2) (C=O)O(C₁-C₆)-Alkyl, worin Alkyl wahlweise substituiert ist mit Phenyl oder Methoxy,
(3) (C=O)-Phenyl, worin Phenyl wahlweise substituiert ist mit Bromid oder Methoxy,
(4) (C₁-C₆)-Alkyl, worin Alkyl wahlweise substituiert ist mit Phenyl, Methoxy, Hydroxy, OCH₂OCH₃, Benzyl-SO₃, Phenyl-SO₃ oder Carboxymethyl,
(5) (C₂-C₆)-Alkenyl,
(6) (C=O)O-Phenyl, worin Phenyl wahlweise substituiert ist mit Nitro,
(7) (C=O)O(C₂-C₆)-Alkenyl,
(8) (C=O)(C₁-C₃)-Alkyl, worin Alkyl wahlweise substituiert ist mit Phenyl,
(9) (C=O)(C₂-C₄)-Alkenyl,
(10) Phenyl,
(11) SO₂-Phenyl,
(12) SO₂-Benzyl,
(13) CH₂(CO)CH₃,
(14) CH₂(CO)NH-Benzyl,
(15) CH₂(CO)NH-Allyl,
(16) CH₂(CO)N(CH₃)₂,
(17) CH₂(CO)NH(CH₃),
(18) 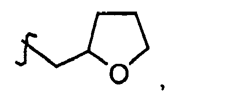
(19) 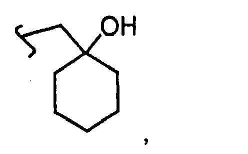
(20) 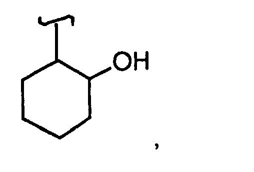
(21) 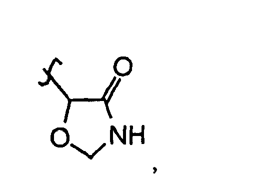
(22) 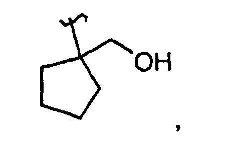
(23) 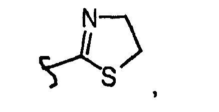
(24) CH₂CH₂NHCO₂(C₁-C₃)-Alkyl,
(25) CH₂CH₂O(CO)NHCH₃,
(26) CH₂CH₂O(CO)NH-Allyl,
(27) CH₂CH₂NH(SO₂)CH₃,
(28) CH₂CH₂NH₂,
(29) CH₂CH₂NH(CO)CH₂CH₃ und
(30) Benzyl;
R¹⁰ ist:
(1) Wasserstoff
(2) (C=O)-Phenyl, worin Phenyl unsubstituiert ist oder substituiert ist mit F, Cl, Br oder I oder
(3) (C₁-C₃)-Alkyl.

6. Verfahren nach Anspruch 1 oder 2, bei welchem der Kalium-Kanalblocker eine Verbindung der Strukturformel III ist; 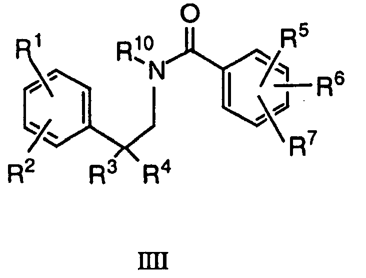 oder ein pharmazeutisch duldbares Salz davon, eine Kristallform davon oder ein Hydrat, worin sind:
n ist: 0, 1, 2 oder 3;
r ist: 0 oder 1;
s ist: 0 oder 1;
R¹, R² R⁵, R⁶ und R⁷ sind unabhängig:
(1) Halogen, worin Halogen Fluor, Chlor, Brom oder Iod ist,
(2) Hydroxy,
(3) (C₁-C₆)-Alkyl,
(4) HO(C₁-C₆)-Alkyloxy,
(5) (C₁-C₄)-Perfluoralkyl,
(6) (C₂-C₆)-Alkenyl,
(7) (C₂-C₆)-Alkinyl,
(8) O[(C=O)Oᵣ]ₛ(C₁-C₆)-Alkyl, worin das Alkyl cyclisch sein kann oder geradkettig,
(9) (C₁-C₆)-Alkyl-S(O)ₙ-,
(10) Phenyl,
(11) Phenoxy,
(12) Cyano,
(13) Nitro,
(14) CO₂H,
(15) CO(C₁-C₆)-Alkyl,
(16) CO₂(C₁-C₆)-Alkyl,
(17) CONR⁸R⁹,
(18) NR⁸R⁹,
(20) (C₂-C₆)-Alkenyloxy,
(21) Benzyloxy,
(22) Wasserstoff,
(23) OCF₃ oder
(24) R¹ und R² oder R⁶ und R⁷ können zusammengenommen werden, wenn an angrenzenden Kohlenstoffen, um eine kondensierte Benzo-, Dihydrofuranyl-, Furanyl-, Pyrrolidyl-, Dihydropyrrolidyl- oder 1,3-Dioxolan-Gruppe zu bilden;
R³ und R⁴ sind unabhängig:
(1) Halogen, worin Halogen Fluor, Chlor, Brom oder Iod ist,
(2) Hydroxy,
(3) HO(C₁-C₆)-Alkyloxy,
(4) (C₁-C₄)-Perfluoralkyl,
(5) O(CO)CCl₃,
(6) (C₁-C₆)-Alkyl-S(O)ₙ₋,
(7) Phenyl-(CH₂)ᵣ-S(O)ₙ-,
(8) Cyano,
(9) Nitro,
(10) CO₂H,
(11) CO(C₁-C₆)-Alkyl"
(12) CO₂(C₁-C₆)-Alkyl,
(13) CONR⁸R⁹,
(14) NR⁸R⁹,
(15) O(CO)NR⁸R⁹,
(16) Azido,
(17) NR⁸(CO)NR⁸R⁹,
(18) Wasserstoff,
(19) (C₁-C₁₀)-Alkyl, worin Alkyl sowohl cyclische als auch acyclische Gruppen einschließt und unsubstituiert ist oder substituiert ist mit einem oder zwei der Substituenten, die ausgewählt sind aus der Gruppe, bestehend aus:
(a) Halogen, worin Halogen, Fluor, Chlor, Brom oder Iod ist,
(b) Hydroxy,
(c) Oxo,
(d) O[(C=O)Oᵣ]ₛ(C₁-C₆)-Alkyl,
(e) (C₁-C₆)-Alkyl-S(O)ₙ-,
(f) Aryl-(C₁-C₆)-Alkyloxy,
(g) Cyano,
(h) Nitro,
(i) Vinyl,
(j) NR⁸R⁹,
(k) O(CO)NR⁸R⁹,
(l) CHO,
(m) CO₂H,
(n) CO(C₁-C₆)-Alkyl,
(o) CO₂(C₁-C₆)-Alkyl, worin Alkyl substituiert sein kann mit Phenyl,
(p) CONR⁸R⁹,
(q) Aryl, worin Aryl festgelegt ist als Phenyl oder Naphthyl, unsubstituiert ist oder substituiert ist mit einem, zwei oder drei der Substituenten, die ausgewählt sind aus der Gruppe, bestehend aus:
(a') Halogen, wie vorstehend festgelegt,
(b') Hydroxy,
(c') (C₁-C₆)-Alkyl,
(d') (C₁-C₄)-Perfluoralkyl,
(e') (C₂-C₆)-Alkenyl,
(f') (C₂-C₆)-Alkinyl,
(g') (C₁-C₆)-Alkyloxy,
(h') (C₁-C₆)-Alkyl-S(O)ₙ-,
(i') Phenyl,
(j') Phenoxy,
(k') Cyano,
(l') Nitro,
(m') CO₂H,
(n') CO(C₁-C₆)-Alkyl,
(o') CO₂(C₁-C₆)-Alkyl,
(p') CONR⁸R⁹,
(q') NR⁸R⁹ und
(r) Heteroaryl, worin Heteroaryl festgelegt ist als ein unsubstituierter, monosubstituierter oder disubstituierter fünfgliedriger oder sechsgliedriger aromatischer Heterocyclus, der 1 bis 3 Heteroatome enthält, die ausgewählt sind aus der Gruppe, bestehend aus O, N und S, und worin die Substituenten Vertreter sind, die ausgewählt sind aus der Gruppe, bestehend aus:
(a') Halogen, wie vorstehend festgelegt,
(b') Hydroxy,
(c') (C₁-C₆)-Alkyl,
(d') (C₁-C₄)-Perfluoralkyl,
(e') (C₂-C₆)-Alkenyl,
(f') (C₂-C₆)-Alkinyl,
(g') (C₁-C₆)-Alkyloxy,
(h') (C₁-C₆)-Alkyl-S(O)ₙ-,
(i') Phenyl,
(j') Phenoxy,
(k') Cyano,
(l') Nitro,
(m') CO₂H,
(n') CO(C₁-C₆)-Alkyl,
(o') CO₂(C₁-C₆)-Alkyl,
(p') CONR⁸R⁹,
(q') NR⁸R⁹ und
(r') kondensierte Benzo- oder Pyridyl-Gruppe,
(s) Heterocyclyl, worin Heterocyclyl festgelegt ist als ein cyclischer, nichtaromatischer Substituent mit 3 bis 7 Atomen, der 1 bis 3 Heteroatome enthält, die ausgewählt sind aus der Gruppe, bestehend aus O, N und S, wobei der Heterocyclus unsubstituiert ist oder substituiert ist mit einem oder zwei oder drei Substituenten, die ausgewählt sind aus der Gruppe, bestehend aus:
(a') Halogen, wie vorstehend festgelegt,
(b') Hydroxy,
(c') (C₁-C₆)-Alkyl,
(d') (C₁-C₄)-Perfluorelkyl,
(e') (C₂-C₆)-Alkenyl,
(f') (C₂-C₆)-Alkinyl,
(g') (C₁-C₆)-Alkyloxy,
(h') (C₁-C₆)-Alkyl-S(O)ₙ-,
(i') Phenyl,
(j') Phenoxy,
(k') Cyano,
(l') Nitro,
(m') CO₂H,
(n') CO(C₁-C₆)-Alkyl,
(o') CO₂(C₁-C₆)-Alkyl,
(p') CONR⁸R⁹,
(q') NR⁸R⁹,
(r') NR⁸CO(C₁-C₆)-Alkyl,
(s') Oxo,
(t') kondensierte Benzo- und
(u') kondensierte Pyridyl-Gruppe;
(t) Benzyl-S(O)ₙ-,
(u) O[(C=O)Oᵣ]ₛ(C₂-C₆)-Alkenyl,
(v) O[(C=O)Oᵣ]ₛ-Aryl,
(w) O[(C=O)Oᵣ]ₛ-Heteroaryl,
(x) O(CH₂)ₙ-Heteroaryl oder
(y) O(CH₂)ₙ-Aryl;
(20) (C₂-C₁₀)-Alkenyl, worin Alkenyl unsubstituiert ist oder substituiert ist mit einem oder zwei der Substituenten, die ausgewählt sind aus der Gruppe, bestehend aus:
(a) Halogen, worin Halogen Fluor, Chlor, Brom oder Iod ist,
(b) Hydroxy,
(c) Oxo,
(e) (C₁-C₆)-Alkyl-S(O)ₙ-,
(f) Phenyl-(C₁-C₆)-Alkyloxy,
(g) Cyano,
(h) Nitro,
(i) NR⁸R⁹,
(j) CHO,
(k) CO₂H,
(l) CO(C₁-C₆)-Alkyl,
(m) CO₂(C₁-C₆)-Alkyl,
(n) CONR⁸R⁹,
(o) Aryl, worin Aryl wie vorstehend festgelegt ist,
(p) Heteroaryl, worin Heteroaryl wie vorstehend festgelegt ist,
(q) Heterocyclyl, worin Heterocyclyl wie vorstehend festgelegt ist,
(r) O[(C=O)Oᵣ]ₛ(C₁-C₆)-Alkyl, worin Alkyl wie vorstehend festgelegt ist,
(s) O[(C=O)Oᵣ]ₛ(C₂-C₆)-Alkenyl, worin Alkenyl wie vorstehend festgelegt ist,
(t) O[(C=O)Oᵣ]ₛAryl, worin Aryl wie vorstehend festgelegt ist,
(u) O[(C=O)Oᵣ]ₛ-Heteroaryl, worin Heteroaryl wie vorstehend festgelegt ist,
(v) O(CH₂)ₙ-Heteroaryl, worin Heteroaryl wie vorstehend festgelegt ist,
(w) O(CH₂)ₙ-Aryl, worin Aryl wie vorstehend festgelegt ist;
(21) (C₂-C₁₀)-Alkinyl, worin Alkinyl unsubstituiert ist oder substituiert ist mit einem oder zwei der Substituenten, die ausgewählt aus der Gruppe, bestehend aus:
(a) Halogen, worin Halogen Fluor, Chlor, Brom oder Iod ist,
(b) Hydroxy,
(c) Oxo,
(d) (C₁-C₆)-Alkyloxy,
(e) (C₁-C₆)-S(O)ₙ-,
(f) Phenyl-(C₁-C₆)-Alkyloxy,
(g) Cyano,
(h) Nitro,
(i) Vinyl,
(j) NR⁸R⁹,
(k) NR⁸CO(C₁-C₆)-Alkyl,
(l) CHO,
(m) CO₂H,
(n) CO(C₁-C₆)-Alkyl,
(o) CO₂C(C₁-C₆)-Alkyl,
(p) CONR⁸R⁹,
(q) Aryl, worin Aryl wie vorstehend festgelegt ist,
(r) Heteroaryl, worin Heteroaryl wie vorstehend festgelegt ist,
(s) Heterocyclyl, worin Heterocyclyl wie vorstehend festgelegt ist,
(t) O[(C=O)Oᵣ]ₛ(C₁-C₆)-Alkyl, worin Alkyl wie vorstehend festgelegt ist,
(u) O[(C=O)Oᵣ]ₛ/C₂-C₆)-Alkenyl, worin Alkenyl wie vorstehend festgelegt ist,
(v) O[(C=O)Oᵣ]ₛ-Aryl, worin Aryl wie vorstehend festgelegt ist,
(w) O[(C=O)Oᵣ]ₛ-Heteroparyl, worin Heteroaryl wie vorstehend festgelegt ist;
(x) O(CH₂)ₙ-Heteroaryl, worin Heteroaryl wie vorstehend festgelegt ist,
(y) O(CH₂)ₙ-Aryl, worin Aryl wie vorstehend festgelegt ist,
(22) O[(C=O)Oᵣ]ₛ(C₁-C₆)-Alkyl, worin Alkyl wie vorstehend festgelegt ist,
(23) O[(C=O)Oᵣ]ₛ(C₂-C₆)-Alkenyl, worin Alkenyl wie vorstehend festgelegt ist,
(24) O[(C=O)Oᵣ]ₛ-Aryl, worin Aryl wie vorstehend festgelegt ist,
(25) O[(C=O)Oᵣ]ₛ-Heteroaryl, worin Heteroaryl wie vorstehend festgelegt ist,
(26) O(CH₂)ₙ-Heteroaryl, worin Heteroaryl wie vorstehend festgelegt ist,
(27) Aryl, worin Aryl wie vorstehend festgelegt ist oder
(28) O(CH₂)ₙ-Aryl, worin Aryl wie vorstehend festgelegt ist;
R³ kann ebenfalls ein beliebiger der Folgenden sein, wenn R⁴ nicht vorhanden ist:
(29) Oxo,
(30) =CH-(C₁-C₆)-Alkyl, worin Alkyl wie vorstehend festgelegt ist,
(31) =CH-(C₂-C₆)-Alkenyl, worin Alkenyl wie vorstehend festgelegt ist,
(32) =CH-Aryl, worin Aryl wie vorstehend festgelegt ist oder
(33) =CH₂;
R⁸ und R⁹ sind unabhängig ausgewählt aus der Gruppe, bestehend aus:
(1) Wasserstoff,
(2) [(C=O)Oᵣ]ₛ-Aryl, worin Aryl wie vorstehend festgelegt ist,
(3) [(C=O)Oᵣ]ₛ(C₂-C₈)-Alkenyl, worin Alkenyl wie vorstehend festgelegt ist,
(4) [(C=O)Oᵣ]ₛ(C₁-C₈)-Alkyl, worin Alkyl wie vorstehend festgelegt ist,
(5) (C=O)ᵣS(O)ₙ(C₁-C₈)-Alkyl, worin Alkyl wie vorstehend festgelegt ist,
(6) (C=O)ᵣS(O)ₙ-Aryl, worin Aryl wie vorstehend festgelegt ist,
(7) Heterocyclyl, worin Heterocyclyl wie vorstehend festgelegt ist;
R¹⁰ ist:
(1) Wasserstoff,
(2) [(C=O)Oᵣ]ₛ-Aryl, worin Aryl wie vorstehend festgelegt ist oder
(3) [(C=O)Oᵣ]ₛ(C₁-C₆)-Alkyl, worin Alkyl wie vorstehend festgelegt ist.

7. Verfahren nach Anspruch 6, worin R¹, R², R⁵, R⁶ und R⁷ der Formel III unabhängig sind:
(1) Halogen, worin Halogen Fluor, Chlor, Brom oder Iod ist,
(2) Hydroxy,
(3) (C₁-C₃)-Alkyl,
(4) (C₂-C₃)-Alkenyl,
(5) O(C₁-C₄)-Alkyl, worin das Alkyl cyclisch oder geradkettig sein kann,
(6) O(CO)CH₃,
(7) CO(C₁-C₃)-Alkyl,
(8) CO₂(C₁-C₃)-Alkyl,
(9) Wasserstoff,
(10) R¹ und R² oder R⁶ und R⁷ können zusammengenommen werden, wenn an angrenzenden Kohlenstoffatomen, um eine kondensierte Benzo-, Dihydrofuranyl-, Furanyl-, Pyrrolidyl-, Dihydropyrrolidyl- oder 1,3-Dioxolan-Gruppe zu bilden;
R³ und R⁴ sind unabhängig:
(1) Wasserstoff,
(2) (C₁-C₆)-Alkyl, worin Alkyl cyclische sowie acyclische Gruppen einschließt und unsubstituiert ist oder substituiert ist mit einem oder zwei der Substituenten, die ausgewählt sind aus der Gruppe, bestehend aus:
(a) Halogen, worin Halogen Fluor, Chlor, Brom oder Iod ist,
(b) Hydroxy,
(c) Oxo,
(d) O[(C=O)Oᵣ]ₛ(C₁-C₆)-Alkyl, worin r und s unabhängig Null oder 1 sind,
(e) CO₂(C₂-C₃)-Alkenyl,
(f) O[(C=O)Oᵣ]ₛ(C₁-C₆)-Alkenyl, worin r und s unabhängig Null oder 1 sind,
(g) NR⁸R⁹,
(h) O(CO)NR⁸R⁹,
(i) CHO,
(j) CO₂H,
(k) CO(C₁-C₆)-Alkyl,
(l) CO₂(C₁-C₆)-Alkyl, worin Alkyl substituiert sein kann mit Phenyl,
(m) CONR⁸R⁹,
(n) Aryl, worin Aryl festgelegt ist als Phenyl, unsubstituiert oder substituiert mit einem Substituenten, der ausgewählt ist aus der Gruppe, bestehend aus:
(a') Halogen, wie vorstehend festgelegt,
(b') Hydroxy,
(c') (C₁-C₆)-Alkyl und
(d') (C₁-C₆)-Alkyloxy und
(o) O[(C=O)Oᵣ]ₛ(C₂-C₆)-Alkenyl, worin r und s unabhängig Null oder 1 sind,
(3) (C₂-C₆)-Alkenyl,
(4) Aryl, worin Aryl wie vorstehend festgelegt ist oder
(5) O(CO₂)ₙ-Aryl, worin Aryl wie vorstehend festgelegt ist;
R³ kann ebenfalls ein beliebiger der Folgenden sein, wenn R⁴ nicht vorhanden ist:
(6) =CH-(C₁-C₆)-Alkyl, worin Alkyl wie vorstehend festgelegt ist,
(7) =CH-(C₂-C₆)-Alkenyl, worin Alkenyl wie vorstehend festgelegt ist;
R⁸ und R⁹ sind unabhängig ausgewählt aus der Gruppe, bestehend aus:
(1) Wasserstoff,
(2) [(C=O)Oᵣ]ₛ-Aryl, worin Aryl wie vorstehend festgelegt ist und r und s unabhängig Null oder 1 sind,
(3) (C₂-C₈)-Alkenyl, worin Alkenyl wie vorstehend festgelegt ist und
(4) (C₁-C₆)-Alkyl, worin Alkyl wie vorstehend festgelegt ist, und
R¹⁰ ist,
(1) Wasserstoff oder
(2) (C=O)(C₁-C₃)-Alkyl, worin Alkyl wie vorstehend festgelegt ist.

8. Verfahren nach Anspruch 1 oder 2, bei welchem der Kalium-Kanalblocker ist: *trans-*1-(N-Ethylcarbamoyloxy)-4-phenyl-4-(3-(2-methoxyphenyl)-3-oxo-2-azaprop-1-yl)cyclohexan, trans-1-(N-Allylcarbamoyloxy)-4-phenyl-4-(3-(2-hydroxy-5-fluorphenyl)-3-oxo-2-azaprop-1-yl)cyclohexan, *trans*-1-(N-n-Propylcarbamoyloxy)-4-phenyl-4-(3-(2-methoxyphenyl)-3-oxo-2-azaprop-1-yl)cyclohexan, *trans-*1-(N-Methylcarbamoyloxy)-4-phenyl-4-(3-(2-methoxyphenyl)-3-oxo-2-azaprop-1-yl)cyclohexan, *trans*-1-(N-Allylcarbamoyloxy)-4-phenyl-4-(3-(2-methoxyphenyl)-3-oxo-2-azapropyl)cyclohexan, 1-(2-Methoxyphenyl)-1-oxo-2-aza-(S)-4-isobutyl-4-phenylbutan, 1-(2-Methoxyphenyl)-1-oxo-2-aza-4-(S)-((3-allyloxycarbonyloxy)propyl))-4-phenylbutan, 1-(2-Methoxyphenyl)-1-oxo-2-aza-4,4-diethyl-4-phenylbutan, 1-(2,3-Dihydrobenzofuran-7-yl)-1-oxo-2-aza-4,4-diethyl-4-phenyl)-butan und 1-(2-Methoxyphenyl)-1-oxo-2-aza-4-(S)-(3-hydroxypropyl)-4-phenylbutan.

9. Verfahren nach Anspruch 1 oder 2, bei welchem der Kalium-Kanalblocker ist: 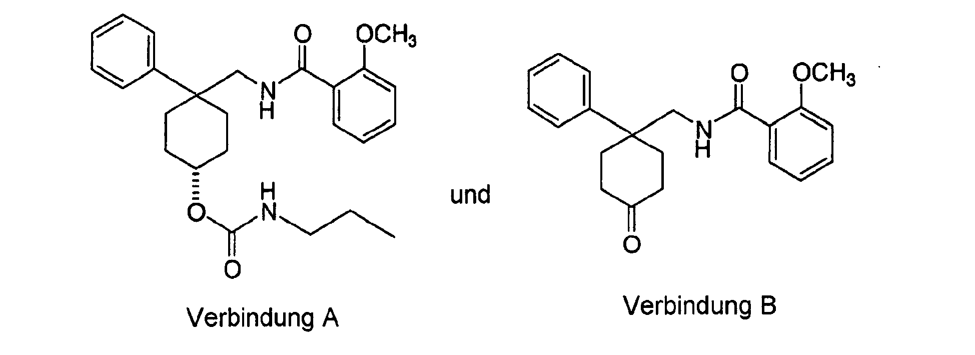 oder ein pharmazeutisch duldbares Salz, Ester, Diastereomer oder Enantiomer davon.

## Revendications

1. Procédé d'identification d'agents bloquants du canal maxi-K, qui sont utiles pour réduire la pression intraoculaire (1), en utilisant des cellules HEK-293, comprenant les étapes de:
(a) construction de lignées cellulaires HEK-293 stables exprimant à la fois les sous-unités alpha et bêta 1 du canal maxi-K;
(b) l'incubation des cellules HEK-293 avec un agent bloquant du potassium qui élimine de manière sélective la conductance du potassium endogène des cellules HEK-293 sans affecter l'activité du canal maxi-K; (2)
(c) la charge des cellules HEK-293 incubées avec un composé de test et l'incubation; et
(d) la mesure de l'activité des agents bloquants du canal maxi-K du composé de test en utilisant un instrument de lecture de tension/sonde ionique, un instrument de transfert d'énergie de résonance par fluorescence (3) et des colorants de détection du potentiel membranaire pour surveiller le potentiel membranaire.

2. Procédé selon la revendication 1, qui comprend les étapes de:
(a) mise en plaque des cellules HEK-293, 2E+06 cellules/mL, sur des plaques de poly-D-lysine de 96 puits à une densité d'environ 100 000 cellules/puits;
(b) l'incubation des plaques durant 16 à 24 heures;
(c) l'aspiration du milieu des cellules;
(d) le lavage des cellules 1 fois avec une solution saline tamponnée au phosphate de Dulbecco;
(e) l'addition de 100 µl de 9 µM de colorant coumarine-0,02 % de pluronique-solution saline tamponnée au phosphate de Dulbecco (4) par puits;
(f) l'incubation à l'obscurité durant environ 30 minutes;
(g) le lavage des cellules 2 fois avec une solution saline tamponnée au phosphate de Dulbecco;
(h) l'addition de 100 µl de colorant oxanol à 4,5 µM en (mM): NaCl 140, KCl 0,1, CaCl₂ 2, MgCl₂ 1, Hepes-NaOH 20, pH à environ 7,4, glucose 10 par puits;
(i) l'addition d'un inhibiteur de la conductance du potassium endogène des cellules HEK-293;
(j) l'addition d'un composé de test;
(k) l'incubation à la température ambiante durant environ 30 minutes;
(l) la charge des plaques sur un lecteur de tension/sonde ionique et l'enregistrement de l'émission de fluorescence des deux colorants durant environ 10 secondes;
(m) l'addition de 100 µl d'une solution à teneur élevée en potassium en (mM): KCl 140, CaCl₂ 2, MgCl₂ 1, Hepes-KOH 20, pH à environ 7,4, glucose 10; et l'enregistrement de l'émission de fluorescence des deux colorants durant environ 10 secondes et la détermination du potentiel membranaire à partir de ces données en utilisant un instrument de lecture de tension/sonde ionique.

3. Procédé selon la revendication 1 ou 2, dans lequel l'agent bloquant du canal potassique est un composé de formule structurelle I: 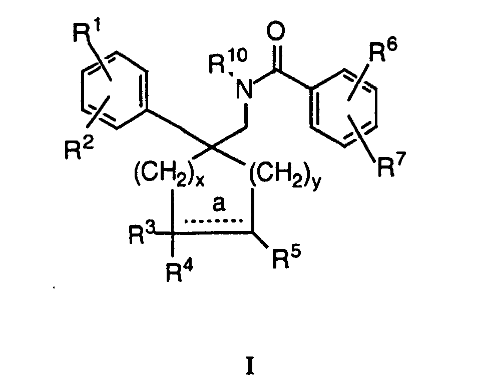 ou un sel pharmaceutiquement acceptable, une forme cristalline, ou un hydrate, dans lequel
a est une simple liaison ou une double liaison lorsque R⁴ est absent, et représenté par ------ dans la structure ci-dessus, à condition que a soit une simple liaison lorsque x + y = 0;
n vaut: 0, 1, 2 ou 3;
r vaut: 0 ou 1;
s vaut: 0 ou 1;
x et y valent indépendamment 0, 1, ou 2;
R¹, R², R⁶ et R⁷ sont indépendamment:
(1) un groupe halogéno, dans lequel le groupe halogéno est le groupe fluoro, chloro, bromo, ou iodo,
(2) hydroxy,
(3) alkyle en C₁ à C₆,
(4) HO(alkyloxy en C₁ à C₆),
(5) perfluoroalkyle en C₁ à C₄,
(6) alcényle en C₂ à C₆,
(7) alcynyle en C₂ à C₆,
(8) O[(C=O)Oᵣ]ₛ(alkyle en C₁ à C₆),
(9) (alkyle en C₁ à C₆)-S(O)ₙ-,
(10) -(O)ᵣ(alkyle en C₀ à C₆)-aryle, où le groupe aryle est un groupe phényle ou naphtyle non substitué ou substitué avec jusqu'à trois substituants choisis parmi le groupe alkyle en C₁ à C₃, trifluorométhyle, et halogéno;
(11) -(O)ᵣ-hétéroaryle, dans lequel le groupe hétéroaryle est un groupe pyridinyle ou pyrryle,
(12) cyano,
(13) nitro,
(14) CO₂H,
(15) CO(alkyle en C₁ à C₆),
(16) CO₂(alkyle en C₁ à C₆),
(17) CONR⁸R⁹,
(18) NR⁸R⁹,
(20) alcényloxy en C₂ à C₆,
(21) (CO)-aryle, dans lequel le groupe aryle est un groupe phényle, naphtyle, benzothiényle, ou un radical benzophénone et est non substitué ou substitué par jusqu'à deux substituants choisis parmi le groupe halogéno, trifluorométhyle, et alkyle en C1 à C3,
(22) un atome d'hydrogène,
(23) un groupe OCF₃,
(24) -(CH₂)-O-N=C(CH₃)(aryle), dans lequel le groupe aryle est un groupe phényle ou naphtyle et est non substitué ou substitué par jusqu'à trois substituants halogènes,
(25) -S(O)ₙNR⁸R⁹, ou
(26) R¹ et R² ou R⁶ et R⁷ peuvent être pris conjointement lorsque placés sur des carbones adjacents pour former un groupe condensé benzo, dihydrofuranyle, furanyle, pyrrolidyle, dihydropyrrolidyle ou 1,3-dioxolane;
R³ et R⁴ sont indépendamment:
(1) un groupe halogéno, dans lequel le groupe halogéno est un groupe fluoro, chloro, bromo, ou iodo,
(2) hydroxy, lorsque a est une simple liaison,
(3) HO(alkyloxy en C₁ à C₆),
(4) perfluoroalkyle en C₁ à C₄,
(5) O(CO)CCl₃,
(6) (alkyle en C₁ à C₆)-S(O)ₙ-,
(7) phényl-(CH₂)ᵣ-S(O)ₙ-,
(8) cyano,
(9) nitro,
(10) CO₂H,
(11) CO(alkyle en C₁ à C₆),
(12) CO₂(alkyle en C₁ à C₆),
(13) CONR⁸R⁹,
(14) NR⁸R⁹,
(15) O(CO)NR⁸R⁹,
(16) azido,
(17) NR⁸(CO)NR⁸R⁹,
(18) hydrogéno,
(19) alkyle en C₁ à C₁₀, dans lequel le groupe alkyle inclut un groupe cyclique ainsi que des groupes acycliques et est non substitué ou substitué par un ou deux des substituants choisis parmi le groupe constitué de:
(a) un groupe halogéno, dans lequel le groupe halogéno est un groupe fluoro, chloro, bromo, ou iodo,
(b) hydroxy,
(c) oxo,
(d) O[(C=O)Oᵣ]ₛ(alkyle en C₁ à C₆),
(e) (alkyle en C₁ à C₆)-S(O)ₙ-,
(f) aryl-(alkyloxy en C₁ à C₆),
(g) cyano,
(h) nitro,
(i) vinyle,
(j) NR⁸R⁹,
(k) O(CO)NR⁸R⁹,
(l) CHO,
(m) CO₂H,
(n) CO(alkyle en C₁ à C₆),
(o) CO₂(alkyle en C₁ à C₆),
(p) CONR⁸R⁹,
(q) aryle, dans lequel le groupe aryle est défini par un groupe phényle ou naphtyle, non substitué ou substitué par un, deux ou trois des substituants choisis parmi le groupe constitué de:
(a') halogéno, tel que défini ci-dessus,
(b') hydroxy,
(c') alkyle en C₁ à C₆,
(d') perfluoroalkyle en C₁ à C₄,
(e') alcényle en C₂ à C₆,
(f) alcynyle en C₂ à C₆,
(g') alkyloxy en C₁ à C₆,
(h') (alkyle en C₁ à C₆)-S(O)Sₙ-,
(i') phényle,
(j') phénoxy,
(k') cyano,
(l') nitro,
(m') CO₂H,
(n') CO(alkyle en C₁ à C₆),
(o') CO₂(alkyle en C₁ à C₆),
(p') CONR⁸R⁹, et
(q') NR⁸R⁹,
(r) hétéroaryle, dans lequel le groupe hétéroaryle est défini comme un groupe hétérocyclique aromatique à cinq ou six chaînons non substitué, monosubstitué, ou disubstitué contenant de 1 à 3 hétéroatome(s) choisi(s) parmi le groupe constitué de O, N et S et dans lequel les substituants sont des membres choisis parmi le groupe constitué de:
(a') halogéno, tel que défini ci-dessus,
(b') hydroxy,
(c') alkyle en C₁ à C₆,
(d') perfluoroalkyle en C₁ à C₄,
(e') alcényle en C₂ à C₆,
(f) alcynyle en C₂ à C₆,
(g') alkyloxy en C₁ à C₆,
(h') (alkyle en C₁ à C₆)-S(O)ₙ-,
(i') phényle,
(j') phénoxy,
(k') cyano,
(l') nitro,
(m') CO₂H,
(n') CO(alkyle en C₁ à C₆),
(o') CO₂(alkyle en C₁ à C₆),
(p') CONR⁸R⁹,
(q') NR⁸R⁹, et
(r') un groupe condensé benzo ou pyridyle,
(s) hétérocyclyle, dans lequel hétérocyclyle est défini comme un substituant non aromatique cyclique à 3 à 7 atomes contenant de 1 à 3 hétéroatome(s) choisi(s) parmi le groupe constitué de O, N, et S, ledit hétérocycle étant non substitué ou substitué par un, deux ou trois substituant(s) choisi(s) parmi le groupe constitué de:
(a') un groupe halogéno, tel que défini ci-dessus,
(b') hydroxy,
(c') alkyle en C₁ à C₆,
(d') perfluoroalkyle en C₁ à C₄,
(e') alcényle en C₂ à C₆,
(f') alcynyle en C₂ à C₆,
(g') alkyloxy en C₁ à C₆,
(h') (alkyle en C₁ à C₆)-S(O)ₙ-,
(i') phényle,
(j') phénoxy,
(k') cyano,
(l') nitro,
(m') CO₂H,
(n') CO(alkyle en C₁ à C₆),
(o') CO₂(alkyle en C₁ à C₆),
(p') CONR⁸R⁹,
(q') NR⁸R⁹,
(r') NR⁸CO(alkyle en C₁ à C₆),
(s') oxo,
(t') benzo condensé, et
(u') groupe pyridyle condensé;
(t) benzyl-S(O)ₙ-,
(u) O[(C=O)Oᵣ]ₛ(alcényle en C₂ à C₆),
(v) O[(C=O)Oᵣ]ₛaryle,
(w) O[(C=O)Oᵣ]ₛhétéroaryle,
(x) O(CH₂)ₙhétéroaryle, ou
(y) O(CH₂)ₙaryle;
(20) alcényle en C₂ à C₁₀, dans lequel le groupe alcényle est non substitué ou substitué par un ou deux des substituants choisis parmi le groupe constitué de:
(a) un groupe halogéno, dans lequel halogéno est fluoro, chloro, bromo, ou iodo,
(b) hydroxy,
(c) oxo,
(e) (alkyle en C₁ à C₆)-S(O)ₙ-,
(f) phényl-(alkyloxy en C₁ à C₆),
(g) cyano,
(h) nitro,
(i) NR⁸R⁹,
(j) CHO,
(k) CO₂H,
(l) CO(alkyle en C₁ à C₆),
(m) CO₂(alkyle en C1 à C₆),
(n) CONR⁸R⁹,
(o) aryle, dans lequel aryle est tel que défini ci-dessus,
(p) hétéroaryle, dans lequel hétéroaryle est tel que défini ci-dessus,
(q) hétérocyclyle, dans lequel hétérocyclyle est tel que défini ci-dessus,
(r) O[(C=O)Oᵣ]ₛ(alkyle en C₁ à C₆), alkyle tel que défini ci-dessus,
(s) O[(C=O)Oᵣ]ₛ(alcényle en C₂ à C₆), tel que défini ci-dessus,
(t) O[(C=O)Oᵣ]ₛaryle, aryle tel que défini ci-dessus,
(u) O[(C=O)Oᵣ]ₛhétéroaryle, hétéroaryle tel que défini ci-dessus,
(v) O(CH₂)ₙhétéroaryle, hétéroaryle tel que défini ci-dessus, et
(w) O(CH₂)ₙaryle, aryle tel que défini ci-dessus;
(21) alcynyle en C₂ à C₁₀, dans lequel alcynyle est non substitué ou substitué avec un ou deux des substituants choisis parmi le groupe constitué de:
(a) halogéno, dans lequel halogéno est un groupe fluoro, chloro, bromo, ou iodo,
(b) hydroxy,
(c) oxo,
(d) alkyloxy en C₁ à C₆,
(e) (C₁ à C₆)-S(O)ₙ-,
(f) phényl-(alkyloxy en C₁ à C₆),
(g) cyano,
(h) nitro,
(i) vinyle,
(j) NR⁸R⁹,
(k) NR⁸CO(alkyle en C₁ à C₆),
(l) CHO,
(m) CO₂H,
(n) CO(alkyle en C₁ à C₆),
(o) CO₂C(alkyle en C₁ à C₆),
(p) CONR⁸R⁹,
(q) aryle, dans lequel le groupe aryle est tel que défini ci-dessus,
(r) hétéroaryle, dans lequel le groupe hétéroaryle est tel que défini ci-dessus,
(s) hétérocyclyle, dans lequel hétérocyclyle est tel que défini ci-dessus,
(t) O[(C=O)Oᵣ]ₛ(alkyle en C₁ à C₆), alkyle tel que défini ci-dessus,
(u) O[(C=O)Oᵣ]ₛ(alcényle en C₂ à C₆), tel que défini ci-dessus,
(v) O[(C=O)Oᵣ]ₛaryle, aryle tel que défini ci-dessus,
(w) O[(C=O)Oᵣ]ₛhétéroaryle, hétéroaryle tel que défini ci-dessus
(x) O(CH₂)ₙhétéroaryle, hétéroaryle tel que défini ci-dessus, et
(y) O(CH₂)ₙaryle, aryle tel que défini ci-dessus,
(22) O[(C=O)Oᵣ]ₛ(alkyle en C₁ à C₆), alkyle tel que défini ci-dessus,
(23) O[(C=O)Oᵣ]ₛ(alcényle en C₂ à C₆), tel que défini ci-dessus,
(24) O[(C=O)Oᵣ]ₛaryle, aryle tel que défini ci-dessus,
(25) O[(C=O)Oᵣ]ₛhétéroaryle, hétéroaryle tel que défini ci-dessus
(26) O(CH₂)ₙhétéroaryle, hétéroaryle tel que défini ci-dessus,
(27) aryle, dans lequel aryle est tel que défini ci-dessus,
(28) O[(C=O)Oᵣ]ₛhétéroaryle, hétéroaryle tel que défini ci-dessus,
(29) O(CO)NH(CH₂-CO-NR⁸R⁹), ou
(30) O(CH₂)ₙaryle, aryle tel que défini ci-dessus;
R³ peut également être n'importe lequel des suivants lorsque a est une simple liaison et R⁴ est absent:
(31) oxo,
(32) =CH-(alkyle en C₁ à C₆), dans lequel alkyle est tel que défini ci-dessus,
(33) =CH-(alcényle en C₂ à C₆), dans lequel alcényle est tel que défini ci-dessus,
(34) =CH-aryle, dans lequel aryle est tel que défini ci-dessus,
(35) =CH₂, ou
R³ et R⁴ peuvent être pris conjointement pour former un groupe hétérocyclyle spiro-condensé, dans lequel le groupe hétérocyclyle est tel que défini ci-dessus, ou
R³ et R⁵ peuvent être pris conjointement pour former un oxirane condensé lorsque a est une simple liaison, à condition que R⁴ soit absent lorsque a est une liaison double;
R⁵ est:
(1) un atome d'hydrogène,
(2) un atome d'halogène,
(3) un groupe alcényle en C₂ à C₆,
(4) hydroxy,
(5) O[(C=O)Oᵣ]ₛ(alkyle en C₁ à C₆),
(6) O(CO)NR⁸R⁹,
(7) oxo, lorsque a est une simple liaison, ou
R⁵ et R³ peuvent être pris conjointement pour former un oxirane condensé lorsque a est une simple liaison;
R⁸ et R⁹ sont indépendamment choisis parmi le groupe constitué de:
(1) un atome d'hydrogène,
(2) [(C=O)Oᵣ]ₛaryle, dans lequel le groupe aryle est tel que défini ci-dessus,
(3) ((C=O)O_{r]s}(alcényle en C₂ à C₈), dans lequel le groupe alcényle est tel que défini ci-dessus,
(4) [(C=O)Oᵣ]ₛ(alkyle en C₁ à C₈), dans lequel le groupe alkyle est tel que défini ci-dessus,
(5) (C=O)ᵣS(O)ₙ(alkyle en C₁ à C₈), dans lequel le groupe alkyle est tel que défini ci-dessus,
(6) (C=O)ᵣS(O)ₙaryle, dans lequel le groupe aryle est tel que défini ci-dessus, et
(7) hétérocyclyle, dans lequel le groupe hétérocyclyle est tel que défini ci-dessus;
R¹⁰ est:
(1) un atome d'hydrogène,
(2) un groupe [(C=O)Oᵣ]ₛaryle, dans lequel le groupe aryle est tel que défini ci-dessus, ou
(3) [(C=O)Oᵣ]ₛ(alkyle en C₁ à C₆), dans lequel le groupe alkyle est tel que défini ci-dessus.

4. Procédé selon la revendication 3, dans lequel le composé de formule I est défini en outre comme dans le composé de formule II dans lequel x vaut 2 et y vaut 1.

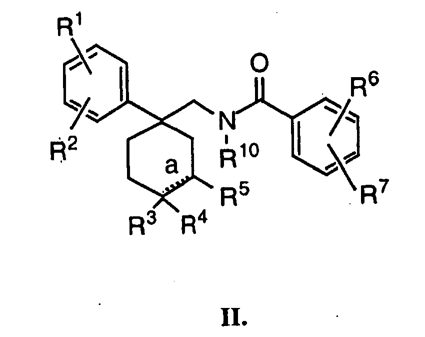

5. Procédé conformément à la revendication 4, dans lequel a est en outre défini comme une simple liaison;
R¹, R² R⁶ et R⁷ sont indépendamment:
(1) un groupe halogéno, dans lequel halogéno est un groupe fluoro, chloro, bromo; ou iodo,
(2) hydroxy,
(3) alkyle en C₁ à C₆,
(4) HO(alkyloxy en C₁ à C₆,
(5) alkyloxy en C₁ à C₆, dans lequel le groupe alkyle est un groupe cyclique ou à chaîne linéaire,
(6) acétoxy,
(7) nitro,
(8) NR⁸R⁹,
(9) -(O)ᵣ(aryle en C₀ à C₃), dans lequel le groupe aryle est un groupe phényle ou naphtyle non substitué ou substitué par jusqu'à trois substituants choisis parmi un groupe alkyle en C₁ à C₃, trifluorométhyle, et halogéno,
(10) hydrogène,
(11) (O)ᵣCF₃,
(12) (alkyle en C₁ à C₆)-S(O)ₙ-, dans lequel n vaut 0, 1, 2 ou 3,
(13) (CO₂)-(alkyle en C₁ à C₆),
(14) -(O)ᵣ-hétéroaryle, dans lequel le groupe hétéroaryle est un groupe pyridinyle ou pyrryle,
(15) (CO)-aryle, dans lequel le groupe aryle est un groupe phényle, naphtyle, benzothiényle, ou un radical benzophénone et est non substitué ou substitué par jusqu'à deux substituants choisis parmi un atome d'halogène, un groupe trifluorométhyle, et alkyle en C1 à C3,
(16) -(CH₂)-O-N=C(CH₃)(aryle), dans lequel le groupe aryle est un groupe phényle, non substitué ou substitué avec jusqu'à trois substituants halogènes,
(17) -S(O)ₙ-NR⁸R⁹, ou
(18) R¹ et R² ou R⁶ et R⁷ peuvent être pris conjointement pour former un groupe condensé benzo, dihydrofuranyle, furanyle, pyrrolidyle, dihydropyrrolidyle ou 1,3-dioxolane;
R³ et R⁴ sont indépendamment:
(1) un groupe halogéno, dans lequel le groupe halogéno est un groupe fluoro, chloro, bromo, ou iodo,
(2) hydroxy,
(3) HO(alkyloxy en C₁ à C₆),
(4) (C=O)O(alkyle en C₁ à C₆),
(5) O(CO)CCl₃,
(6) (alkyle en C₁ à C₆)-S(O)ₙ-, dans lequel n vaut 0, 1, 2 ou 3,
(7) CH₂CO₂-(alkyle en C₁ à C₆),
(8) cyano,
(9) benzyloxy,
(10) CH₂OAc,
(11) OAc,
(12) alcényle en C₂ à C₆,
(13) alkyle en C₁ à C₆, dans lequel le groupe alkyle peut être non substitué ou substitué avec du bromure
(14) NR⁸R⁹,
(15) O(CO)NR⁸R⁹,
(16) azido,
(17) NR⁸(CO)NR⁸R⁹,
(18) hydrogéno,
(19) CH₂OH,
(20) CH₂O(C=O)phényle, dans lequel le groupe phényle est non substitué ou monosubstitué par un groupe méthoxy,
(21) O(alcényle en C₂ à C₆),
(22) O(C=O)-phényle, dans lequel le groupe phényle est non substitué ou monosubstitué par un bromure,
(23) O(C=O)O-phényle, dans lequel le groupe phényle est non substitué ou monosubstitué par un groupe nitro,
(24) CH₂(CO)NR⁸R⁹,
(25) O(C=O)O-(alcényle en C₂ à C₆),
(26) O(C=O)-(alkyle en C₁ à C₃), dans lequel le groupe alkyle peut être non substitué ou substitué par un bromure ou -CO₂CH₃,
(27) O(alkyle en C₁ à C₆), dans lequel le groupe alkyle peut être non substitué ou substitué par un groupe phényle,
(28) O(C=O)O-(alkyle en C₁ à C₆),
(29) CH₂O(CO)NR⁸R⁹, ou
(30) CH₂(C=O)O-(alkyle en C₁ à C₆),
R³ peut également être n'importe lequel des suivants lorsque R⁴ est absent:
(31) oxo,
(32) =CH₂,
(33) =CH-CO₂-(alkyle en C₁ à C₆),
(34) =CH-(CO)-NR⁸R⁹, ou
(35) =CH-CO₂H, ou
R³ et R⁴ peuvent être pris conjointement pour former un groupe hétérocyclyle spiro-condensé, dans lequel le groupe hétérocyclyle est défini comme:
(36) un oxirane,
(37) 1,3-dioxolane,
(38) 2,2-diméthyl-1,3-dioxolane, ou
(39) du sulfite de glycol, ou
R³ et R⁵ peuvent être pris conjointement pour former un oxirane condensé;
R⁵ est:
(1) un atome d'hydrogène,
(2) un groupe halogéno,
(3) un groupe alcényle en C₂ à C₆,
(4) un groupe hydroxy,
(5) O(C=O)(alkyle en C₁ à C₃),
(6) O(CO)NR⁸R⁹,
(7) oxo, lorsque a est une simple liaison, ou
R⁵ et R³ peuvent être pris conjointement pour former un oxirane condensé lorsque a est une simple liaison;
R⁸ et R⁹ sont indépendamment choisis parmi le groupe constitué de:
(1) un atome d'hydrogène,
(2) (C=O)O(alkyle en C₁à C₆), dans lequel le groupe alkyle est éventuellement substitué par un groupe phényle ou méthoxy,
(3) (C=O)phényle, dans lequel le groupe phényle est éventuellement substitué par un bromure ou un groupe méthoxy,
(4) alkyle en C₁ à C₆, dans lequel le groupe alkyle est éventuellement substitué par un groupe phényle, méthoxy, hydroxy, OCH₂OCH₃, benzylSO₃, phénylSO₃, ou carboxyméthyle,
(5) alcényle en C₂ à C₆,
(6) (C=O)O-phényle, dans lequel le groupe phényle est éventuellement substitué par un groupe nitro,
(7) (C=O)O(alcényle en C₂ à C₆),
(8) (C=O)(alkyle en C₁ à C₃), dans lequel le groupe alkyle est éventuellement substitué par un groupe phényle,
(9) (C=O)(alcényle en C₂ à C₄),
(10) phényle,
(11) SO₂-phényle,
(12) SO₂-benzyle,
(13) CH₂(CO)CH₃,
(14) CH₂(CO)NH-benzyle,
(15) CH₂(CO)NH-allyle,
(16) CH₂(CO)N(CH₃)₂,
(17) CH₂(CO)NH(CH₃),
(18) 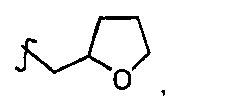
(19) 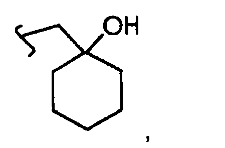
(20) 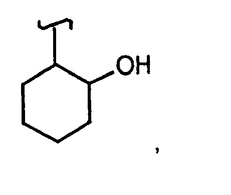
(21) 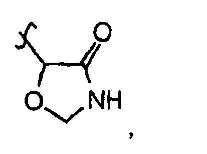
(22) 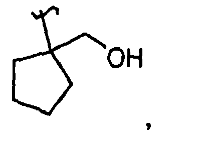
(23) 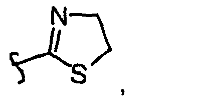
(24) CH₂CH₂NHCO₂(alkyle en C₁ à C₃),
(25) CH₂CH₂O(CO)NHCH₃,
(26) CH₂CH₂O(CO)NH-allyle,
(27) CH₂CH₂NH(SO₂)CH₃,
(28) CH₂CH₂NH₂,
(29) CH₂CH₂NH(CO)CH₂CH₃, et
(30) benzyle;
R¹⁰ est:
(1) un atome d'hydrogène,
(2) un groupe (C=O)phényle, dans lequel le groupe phényle est non substitué ou substitué par F, C₁, Br, ou I, ou
(3) un groupe alkyle en C₁ à C₃,

6. Procédé conformément à la revendication 1 ou 2, dans lequel l'agent bloquant du canal potassique est un composé de formule structurelle III: 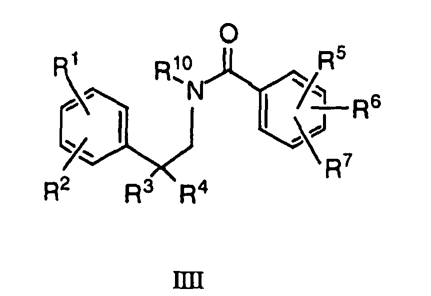 or un sel pharmaceutiquement acceptable, une forme cristalline, ou un hydrate, dans lequel:
n vaut: 0, 1, 2 ou 3;
r vaut: 0 ou 1;
s vaut: 0 ou 1;
R¹, R², R⁵, R⁶ et R⁷ sont indépendamment:
(1) un groupe halogéno, dans lequel le groupe halogéno est un groupe fluoro, chloro, bromo, ou iodo,
(2) hydroxy,
(3) alkyle en C₁ à C₆,
(4) HO(alkyloxy en C₁ à C₆),
(5) perfluoroalkyle en C₁ à C₄,
(6) alcényle en C₂ à C₆,
(7) alcynyle en C₂ à C₆,
(8) [O(C=O)Oᵣ]ₛ(alkyle en C1 à C6), dans lequel le groupe alkyle peut être cyclique ou à chaîne linéaire,
(9) (alkyle en C₁ à C₆)-S(O)ₙ-,
(10) phényle,
(11) phénoxy,
(12) cyano,
(13) nitro,
(14) CO₂H,
(15) CO(alkyle en C₁ à C₆),
(16) CO₂(alkyle en C₁ à C₆),
(17) CONR⁸R⁹,
(18) NR⁸R⁹,
(20) alcényloxy en C₂ à C₆,
(21) benzyloxy,
(22) hydrogéno,
(23) OCF₃, ou
(24) R¹ et R² ou R⁶ et R⁷ peuvent être pris conjointement lorsque placés sur des carbones adjacents pour former un groupe condensé benzo, dihydrofuranyle, furanyle, pyrrolidyle, dihydropyrrolidyle ou 1,3-dioxolane;
R³ et R⁴ sont indépendamment:
(1) un halogéno, dans lequel le groupe halogéno est un groupe fluoro, chloro, bromo, ou iodo,
(2) hydroxy,
(3) HO(alkyloxy en C₁ à C₆),
(4) perfluoroalkyle en C₁ à C₄,
(5) O(CO)CCl₃,
(6) (alkyle en C₁ à à C₆)-S(O)ₙ-,
(7) phényl-(CH₂)ᵣ-S(O)ₙ-,
(8) cyano,
(9) nitro,
(10) CO₂H,
(11) CO(alkyle en C₁ à C₆),
(12) CO₂(alkyle en C₁ à C₆),
(13) CONR⁸R⁹,
(14) NR⁸R⁹,
(15) O(CO)NR⁸R⁹,
(16) azido,
(17) NR⁸(CO)NR⁸R⁹,
(18) hydrogéno,
(19) alkyle en C₁ à C₁₀, dans lequel le groupe alkyle inclut un groupe cyclique ainsi que des groupes acycliques et est non substitué ou substitué avec un ou deux des substituants choisis parmi le groupe constitué de:
(a) un groupe halogéno, dans lequel le groupe halogéno est un groupe fluoro, chloro, bromo, ou iodo,
(b) hydroxy,
(c) oxo,
(d) O[(C=O)Oᵣ]ₛ(alkyle en C₁ à C₆),
(e) (alkyle en C₁ à C₆)-S(O)ₙ-,
(f) aryl-(alkyloxy en C₁ à C₆),
(g) cyano,
(h) nitro;
(i) vinyle,
(j) NR⁸R⁹,
(k) O(CO)NR⁸R⁹,
(l) CHO,
(m) CO₂H,
(n) CO(alkyle en C₁ à C₆),
(o) CO₂(alkyle en C₁ à C₆), dans lequel le groupe alkyle peut être substitué par un groupe phényle,
(p) CONR⁸R⁹,
(q) aryle, dans lequel le groupe aryle est défini par un groupe phényle ou naphtyle, non substitué ou substitué par un, deux ou trois des substituants choisis parmi le groupe constitué de:
(a') un groupe halogéno, tel que défini ci-dessus,
(b') hydroxy,
(c') alkyle en C₁ à C₆,
(d') perfluoroalkyle en C₁ à C₄,
(e') alcényle en C₂ à C₆,
(f) alcynyle en C₂ à C₆,
(g') alkyloxy en C₁ à C₆,
(h') (alkyle en C₁ à C₆)-S(O)ₙ-,
(i') phényle,
(j') phénoxy,
(k') cyano,
(l') nitro,
(m') CO₂H,
(n') CO(alkyle en C₁ à C₆),
(o') CO₂(alkyle en C₁ à C₆),
(p') CONR⁸R⁹,
(q') NR⁸R⁹, et
(r) hétéroaryle, dans lequel le groupe hétéroaryle est tel que défini comme un hétérocycle aromatique à cinq ou six chaînons non substitué, monosubstitué, ou disubstitué contenant de 1 à 3 hétéroatome(s) choisi(s) parmi le groupe constitué de O, N et S et dans lequel les substituants sont des membres choisis parmi le groupe constitué de:
(a') halogéno, tel que défini ci-dessus,
(b') hydroxy,
(c') alkyle en C₁ à C₆,
(d') perfluoroalkyle en C1 à C4,
(e') alcényle en C₂ à C₆,
(f') alcynyle en C₂ à C₆,
(g') alkyloxy en C₁ à C₆,
(h') (alkyle en C₁ à C₆)-S(O)ₙ-,
(i') phényle,
(j') phénoxy,
(k') cyano,
(l') nitro,
(m') CO₂H,
(n') CO(alkyle en C₁ à C₆),
(o') CO₂(alkyle en C₁ à C₆),
(p') CONR⁸R⁹,
(q') NR⁸R⁹, et
(r') un groupe condensé benzo ou pyridyle,
(s) hétérocyclyle, dans lequel le groupe hétérocyclyle est défini comme un substituant non aromatique cyclique de 3 à 7 atomes contenant de 1 à 3 hétéroatome(s) choisi(s) parmi le groupe constitué de O, N, et S, ledit hétérocycle étant non substitué ou substitué par un, deux ou trois substituant(s) choisi(s) parmi le groupe constitué de:
(a') un groupe halogéno, tel que défini ci-dessus,
(b') hydroxy,
(c') alkyle en C₁ à C₆,
(d') perfluoroalkyle en C₁ à C₄,
(e') alcényle en C₂ à C₆,
(f') alcynyle en C₂ à C₆,
(g') alkyloxy en C₁ à C₆,
(h') (alkyle en C₁ à C₆)-S(O)ₙ-,
(i') phényle,
(j') phénoxy,
(k') cyano,
(l') nitro,
(m') CO₂H,
(n') CO(alkyle en C₁ à C₆),
(o') CO₂(alkyle en C₁ à C₆),
(p') CONR⁸R⁹,
(q') NR⁸R⁹,
(r') NR⁸CO(alkyle en C₁ à C₆),
(s) oxo,
(t') benzo condensé, et
(u') un groupe pyridyle condensé;
(t) benzyl-S(O)ₙ-,
(u) O[(C=O)Oᵣ]ₛ(alcényle en C₂ à C₆),
(v) O[(C=O)Oᵣ]ₛaryle,
(w) O[(C=O)Oᵣ]ₛhétéroaryle,
(x) O(CH₂)ₙhétéroaryle, ou
(y) O(CH₂)ₙaryle;
(20) alcényle en C₂ à C₁₀, dans lequel le groupe alcényle est non substitué ou substitué par un ou deux des substituants choisis parmi le groupe constitué de:
(a) un groupe halogéno, dans lequel le groupe halogéno est un groupe fluoro, chloro, bromo, ou iodo,
(b) hydroxy,
(c) oxo,
(e) (alkyle en C₁ à C₆)-S(O)ₙ-,
(f) phényl-(alkyloxy en C₁ à C₆),
(g) cyano,
(h) nitro,
(i) NR⁸R⁹,
(j) CHO,
(k) CO₂H,
(l) CO(alkyle en C₁ à C₆),
(m) CO₂(alkyle en C₁ à C₆),
(n) CONR⁸R⁹,
(o) aryle, dans lequel le groupe aryle est tel que défini ci-dessus,
(p) hétéroaryle, dans lequel le groupe hétéroaryle est tel que défini ci-dessus,
(q) hétérocyclyle, dans lequel le groupe hétérocyclyle est tel que défini ci-dessus,
(r) O[(C=O)Oᵣ]ₛ(alkyle en C₁ à C₆), alkyle est tel que défini ci-dessus,
(s) O[(C=O)Oᵣ]ₛ(alcényle en C₂ à C₆), tel que défini ci-dessus,
(t) O[(C=O)Oᵣ]ₛaryle, aryle tel que défini ci-dessus,
(u) O[(C=O)Oᵣ]ₛhétéroaryle, hétéroaryle tel que défini ci-dessus,
(v) O(CH₂)ₙhétéroaryle, hétéroaryle tel que défini ci-dessus, et
(w) O(CH₂)ₙaryle, aryle tel que défini ci-dessus;
(21) alcynyle en C₂ à C₁₀, dans lequel le groupe alcynyle est non substitué ou substitué par un ou deux des substituants choisis parmi le groupe constitué de:
(a) un groupe halogéno, dans lequel le groupe halogéno est un groupe fluoro, chloro, bromo, ou iodo,
(b) hydroxy,
(c) oxo,
(d) alkyloxy en C₁ à C₆,
(e) (C₁ à C₆)-S(O)ₙ-,
(f) phényl-(alkyloxy en C₁ à C₆),
(g) cyano,
(h) nitro,
(i) vinyle,
(j) NR⁸R⁹,
(k) NR⁸CO(alkyle en C₁ à C₆),
(l) CHO,
(m) CO₂H,
(n) CO(alkyle en C₁ à C₆),
(o) CO₂C(alkyle en C₁ à C₆),
(p) CONR⁸R⁹,
(q) aryle, dans lequel le groupe aryle est tel que défini ci-dessus,
(r) hétéroaryle, dans lequel le groupe hétéroaryle est tel que défini ci-dessus,
(s) hétérocyclyle, dans lequel le groupe hétérocyclyle est tel que défini ci-dessus,
(t) O[(C=O)Oᵣ]ₛ(alkyle en C₁ à C₆), alkyle tel que défini ci-dessus,
(u) O[(C=O)Oᵣ]ₛ(alcényle en C₂ à C₆), tel que défini ci-dessus,
(v) O[(C=O)Oᵣ]ₛaryle, aryle tel que défini ci-dessus,
(w) O[(C=O)Oᵣ]ₛhétéroaryle, hétéroaryle tel que défini ci-dessus
(x) O(CH₂)ₙhétéroaryle, hétéroaryle tel que défini ci-dessus, et
(y) O(CH₂)ₙaryle, aryle tel que défini ci-dessus,
(22) O[(C=O)Oᵣ]ₛ(alkyle en C₁ à C₆), alkyle tel que défini ci-dessus,
(23) O[(C=O)Oᵣ]ₛ(alcényle en C₂ à C₆), tel que défini ci-dessus,
(24) O[(C=O)Oᵣ]ₛaryle, aryle tel que défini ci-dessus,
(25) O[(C=O)Oᵣ]ₛhétéroaryle, hétéroaryle tel que défini ci-dessus
(26) O(CH₂)ₙhétéroaryle, hétéroaryle tel que défini ci-dessus,
(27) aryle, dans lequel le groupe aryle est tel que défini ci-dessus ou
(28) O(CH₂)ₙaryle, aryle tel que défini ci-dessus;
R³ peut également être n'importe lequel des suivants lorsque R⁴ est absent:
(29) oxo,
(30) =CH-(alkyle en C₁ à C₆), dans lequel le groupe alkyle est tel que défini ci-dessus,
(31) =CH-(alcényle en C₂ à C₆), dans lequel le groupe alcényle est tel que défini ci-dessus,
(32) =CH-aryle, dans lequel le groupe aryle est tel que défini ci-dessus, ou
(33) =CH₂;
R⁸ et R⁹ sont indépendamment choisis parmi le groupe constitué de:
(1) un atome d'hydrogène,
(2) [(C=O)Oᵣ]ₛaryle, dans lequel le groupe aryle est tel que défini ci-dessus,
(3) [(C=O)Oᵣ]ₛ(alcényle en C₂ à C₈), dans lequel le groupe alcényle est tel que défini ci-dessus,
(4) [(C=O)Oᵣ]ₛ(alkyle en C₁ à C₈), dans lequel le groupe alkyle est tel que défini ci-dessus,
(5) (C=O)ᵣS(O)ₙ(alkyle en C₁ à C₈), dans lequel le groupe alkyle est tel que défini ci-dessus,
(6) (C=O)ᵣS(O)ₙ-aryle, dans lequel le groupe aryle est tel que défini ci-dessus, et
(7) hétérocyclyle, dans lequel le groupe hétérocyclyle est tel que défini ci-dessus;
R10 est:
(1) un atome d'hydrogène,
(2) un groupe [(C=O)Oᵣ]ₛaryle, dans lequel le groupe aryle est tel que défini ci-dessus, ou
(3) [(C=O)Oᵣ]ₛ(alkyle en C₁ à C₆), dans lequel le groupe alkyle est tel que défini ci-dessus.

7. Procédé selon la revendication 6, dans lequel R¹, R², R⁵, R⁶ et R⁷ de formule III sont indépendamment:
(1) un groupe halogéno, dans lequel le groupe halogéno est un groupe fluoro, chloro, bromo, ou iodo,
(2) hydroxy,
(3) alkyle en C₁ à C₃,
(4) alcényle en C₂ à C₃,
(5) O(alkyle en C₁ à C₄), dans lequel le groupe alkyle peut être cyclique ou à chaîne linéaire,
(6) O(CO)CH₃,
(7) CO(alkyle en C₁ à C₃),
(8) CO₂(alkyle en C₁ à C₃),
(9) hydrogéno,
(10) R¹ et R² ou R⁶ et R⁷ peuvent être pris conjointement lorsque placés sur des carbones adjacents pour former un groupe condensé benzo, dihydrofuranyle, furanyle, pyrrolidyle, dihydropyrrolidyle ou 1,3-dioxolane:
R³ et R⁴ sont indépendamment:
(1) un atome d'hydrogène,
(2) un groupe alkyle en C₁ à C₆, dans lequel le groupe alkyle inclut un groupe cyclique ainsi que des groupes acycliques et est non substitué ou substitué par un ou deux des substituants choisis parmi le groupe constitué de:
(a) un groupe halogéno, dans lequel le groupe halogéno est un groupe fluoro, chloro, bromo, ou iodo,
(b) hydroxy,
(c) oxo,
(d) O[(C=O)Oᵣ]ₛ(alkyle en C₁ à C₆), dans lequel r et s valent indépendamment 0 ou 1,
(e) CO₂(alcényle en C₂ à C₃),
(f) O[(C=O)Oᵣ]ₛ(alcényle en C₁ à C₆), dans lequel r et s valent indépendamment 0 ou 1,
(g) NR⁸R⁹,
(h) O(CO)NR⁸R⁹,
(i) CHO,
(j) CO₂H,
(k) CO(alkyle en C₁ à C₆),
(l) CO₂(alkyle en C₁ à C₆), dans lequel le groupe alkyle peut être substitué par un groupe phényle,
(m) CONR⁸R⁹,
(n) aryle, dans lequel le groupe aryle est défini comme un groupe phényle, non substitué ou substitué par un substituant choisi parmi le groupe constitué de:
(a') un groupe halogéno, tel que défini ci-dessus,
(b') hydroxy,
(e') alkyle en C₁ à C₆, et
(d') alkyloxy en C₁ à C₆, et
(o) O[(C=O)Oᵣ]ₛ(alcényle en C₂ à C₆), dans lequel r et s valent indépendamment 0 ou 1,
(3) alcényle en C₂ à C₆,
(4) aryle, dans lequel le groupe aryle est tel que défini ci-dessus ou
(5) O(CH₂)ₙaryle, dans lequel le groupe aryle est tel que défini ci-dessus;
R³ peut également être n'importe lequel des suivants lorsque R⁴ est absent:
(6) =CH-(alkyle en C₁ à C₆), dans lequel le groupe alkyle est tel que défini ci-dessus,
(7) =CH-(alcényle en C₂ à C₆), dans lequel le groupe alcényle est tel que défini ci-dessus,
R⁸ et R⁹ sont indépendamment choisis parmi le groupe constitué de:
(1) un atome d'hydrogène,
(2) [(C=O)Oᵣ]ₛaryle, dans lequel le groupe aryle est tel que défini ci-dessus et r et s valent indépendamment 0 ou 1,
(3) alcényle en C₂ à C₈, dans lequel le groupe alcényle est tel que défini ci-dessus, et
(4) alkyle en C₁ à C₆, dans lequel le groupe alkyle est tel que défini ci-dessus, et
R¹⁰ est:
(1) un atome d'hydrogène, ou
(2) (C=O)(alkyle en C₁ à C₃), dans lequel le groupe alkyle est tel que défini ci-dessus.

8. Procédé selon la revendication 1 ou 2 dans lequel l'agent bloquant du canal potassique est:
le *trans* 1-(N-éthylcarbamoyloxy)-4-phényl-4-(3-(2-méthoxyphényl)-3-oxo-2-azaprop-1-yl)cyclohexane, le *trans* 1-(N-allylcarbamoyloxy)-4-phényl-4-(3-(2-hydroxy-5-fluorophényl)-3-oxo-2-azaprop-1-yl)cyclohexane, le *trans* 1-(N-*n*-propylcarbamoyloxy)-4-phényl-4-(3-(2-méthoxyphényl)-3-oxo-2-azaprop-1-yl)cyclohexane, le *trans* 1-(N-méthylcarbamoyloxy)-4-phényl-4-(3-(2-méthoxyphényl)-3-oxo-2-azaprop-1-yl)cyclohexane, le *trans* 1-(N-allylcarbamoyloxy)-4-phényl-4-(3-(2-méthoxyphényl)-3-oxo-2-azapropyl)cyclohexane, le 1-(2-méthoxyphényl)-1-oxo-2-aza-(S)-4-*i*-butyl-4-phénylbutane, le 1-(2-méthoxyphényl)-1-oxo-2-aza-4-(S)-((3-allyloxycarbonyloxy)propyl))-4-phénylbutane, le 1-(2-méthoxyphényl)-1-oxo-2-aza-4,4-diéthyl-4-phénylbutane, le 1-(2,3-dihydrobenzofuran-7-yl)-1-oxo-2-aza-4,4-diéthyl-4-(phényl)-butane, et le 1-(2-méthoxyphényl)-1-oxo-2-aza-4-(S)-(3-hydroxypropyl)-4-phénylbutane.

9. Procédé selon la revendication 1 ou 2, dans lequel l'agent bloquant du canal potassique est: 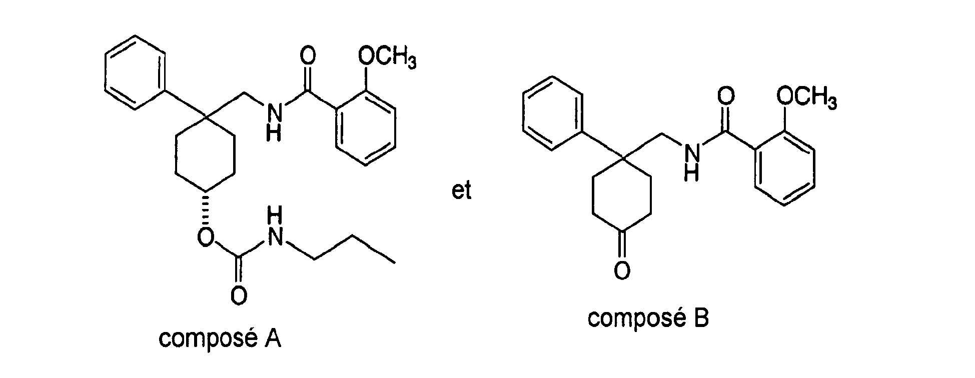 ou un sel pharmaceutiquement acceptable, un ester, un diastéréomère ou un énantiomère de celui-ci.
